# EUROPÄISCHE PATENTSCHRIFT

(11) **EP 1 485 412 B1**
(45) Veröffentlichungstag und Bekanntmachung des Hinweises auf die Patenterteilung: **14.01.2009**
(21) Anmeldenummer: 03712005.2
(22) Anmeldetag: 13.03.2003
(51) Int. Cl.: C07K 14/705, C07K 14/435, C07K 16/18, A61K 38/17, C12N 15/12

(54) **REPRESSOR DER SKELETTMUSKELDIFFERENZIERUNG, DAFÜR KODIERENDE NUCLEINSÄURE UND DESSEN VERWENDUNG IN DIAGNOSTIK UND THERAPIE**
REPRESSOR OF SKELETAL MUSCLE DIFFERENTIATION, NUCLEIC ACIDS CODING THEREFOR AND THE USE THEREOF IN DIAGNOSIS AND THERAPY
REPRESSEUR DE LA DIFFERENCIATION DES MUSCLES DU SQUELETTE, ACIDE NUCLEIQUE CODANT POUR LEDIT REPRESSEUR ET UTILISATION DUDIT REPRESSEUR A DES FINS DE DIAGNOSTIC ET DE THERAPIE

(30) Priorität: 20.03.2002 DE 10212397
(43) Veröffentlichungstag der Anmeldung: 15.12.2004
(73) Patentinhaber: Universitätsklinikum Freiburg, 79106 Freiburg (DE)
(72) Erfinder: SCHÜLE, Roland, 79367 Weisweil (DE); HUBLITZ, Philip, 00015 Monterotondo (IT)
(74) Vertreter: Keller, Günter
(86) Internationale Anmeldenummer: PCT/EP2003/002638
(87) Internationale Veröffentlichungsnummer: WO 2003/078463

(56) Entgegenhaltungen:
- EP-A- 1 074 617
- WO-A-01/57190
- DATABASE EMBL/GENBANK/DDBJ [Online] 9. März 2001 (2001-03-09) STRAUSBERG, R.: "Homo sapiens, DKFZP564C184 protein, clone MGC:1451 IMAGE: 3546209, mRNA complete cds." retrieved from EBI Database accession no. BC003555 XP002247292
- DATABASE SWISS-PROT [Online] 30. Mai 2000 (2000-05-30) "Hypothetical UPF0120 protein DKFZp564C186" retrieved from EBI Database accession no. Q9Y3T9 XP002247293
- DATABASE EMBL/GENBANK/DDBJ [Online] 4. Januar 2002 (2002-01-04) STRAUSBERG, R.: "Mus musculus cDNA sequence AF155546, mRNA (cDNA clone MGC:28032 IMAGE:3663482), complete cds." retrieved from EBI Database accession no. BC020013 XP002247294
- DATABASE SWISS-PROT [Online] 30. Mai 2000 (2000-05-30) "Hypothetical UPF0120 protein DKFZp564C186 homolog" retrieved from EBI Database accession no. Q9WV70 XP002247295

### SEQUENZPROTOKOLL

<110> Universitätsklinikum Freiburg
<120> Repressor der Skelettmuskeldifferenzierung, dafür kodierende Nucleinsäure und dessen Verwendung in Diagnostik und Therapie
<130> hsgrim1
<140>
   <141>
<160> 8
<170> PatentIn Ver. 2.1
<210> 1
   <211> 2250
   <212> DNA
   <213> Homo sapiens
<400> 1
   atggcagctg cggggagccg caagaggcgc ctggcggagc tgacggtgga cgagttccta 60
   gcttcgggct ttgactccga gtccgaatcc gagtccgaaa attctccaca agcggagaca 120
   cgggaagcac gcgaggctgc ccggagtccg gataagccgg gcgggagccc ctcggccagc 180
   cggcgtaaag gccgtgcctc tgagcacaaa gaccagctct ctcggctgaa ggacagagac 240
   cccgagttct acaagttcct gcaggagaat gaccagagcc tgctaaactt cagcgactcg 300
   gacagctctg aggaggaaga ggggccgttc cactccctgc cagatgtgct ggaggaagcc 360
   agtgaggagg aggatggagc ggaggaagga gaagatgggg acagagtccc cagagggctg 420
   aaggggaaga agaattctgt tcctgtgacc gtcgccatgg ttgagagatg gaagcaggca 480
   gcaaagcaac gcctcactcc aaagctgttc catgaagtgg tacaggcgtt ccgagcagct 540
   gtggccacca cccgagggga ccaggaaagt gctgaggcca acaaattcca ggtcacggac 600
   agtgctgcat tcaatgctct ggttaccttc tgcatcagag acctcattgg ctgtctccag 660
   aagctgctgt ttggaaaggt ggcaaaggat agcagcagga tgctgcagcc gtccagcagc 720
   ccgctctggg ggaagcttcg tgtggacatc aaggcttacc tgggctcggc catacagctg 780
   gtgtcctgtc tgtcggagac gacggtgttg gcggccgtgc tgcggcacat cagcgtgctg 840
   gtgccctgct tcctgacctt ccccaagcag tgccgcatgc tgctcaagag aatggtggtc 900
   gtatggagca ctggggagga gtctctgcgg gtgctggctt tcctggtcct cagcagagtc 960
   tgccggcaca agaaggacac tttccttggc cccgtcctca agcaaatgta catcacgtat 1020
   gtgaggaact gcaagttcac ctcgcctggt gccctcccct tcatcagttt catgcagtgg 1080
   accttgacgg agctgctggc cctggagccg ggtgtggcct accagcacgc cttcctctac 1140
   atccgccagc tcgccataca cctgcgcaac gccatgacca cccgcaagaa ggaaacatac 1200
   cagtctgtgt acaactggca gtatgtgcac tgcctcttcc tgtggtgccg ggtcctgagc 1260
   actgcgggcc ccagcgaagc cctccagccc ttggtctacc cccttgccca agtcatcatt 1320
   ggctgtatca agctcatccc cactgcccgc ttctacccgc tgcgaatgca ctgcatccgt 1380
   gccctgacgc tgctctcggg gagctcgggg gccttcatcc cggtgctgcc tttcatcctg 1440
   gagatgttcc agcaggtcga cttcaacagg aagccagggc gcatgagctc caagcccatc 1500
   aacttctccg tgatcctgaa gctgtccaat gtcaacctgc aggagaaggc gtaccgggac 1560
   ggcctggtgg agcagctgta cgacctcacc ctggagtacc tgcacagcca ggcacactgc 1620
   atcggcttcc cggagctggt gctgcctgtg gtcctgcagc tgaagtcgtt cctccgggag 1680
   tgcaaggtgg ccaactactg ccggcaggtg cagcagctgc ttgggaaggt tcaggagaac 1740
   tcggcataca tctgcagccg ccgccagagg gtttccttcg gcgtctctga gcagcaggca 1800
   gtggaagcct gggagaagct gacccgggaa gaggggacac ccttgacctt gtactacagc 1860
   cactggcgca agctgcgtga ccgggagatc cagctggaga tcagtggcaa agagcggctg 1920
   gaagacctga acttccctga gatcaaacga aggaagatgg ctgacaggaa ggatgaggac 1980
   aggaagcaat ttaaagacct ctttgacctg aacagctctg aagaggacga caccgaggga 2040
   ttctcggaga gagggatact gaggcccctg agcactcggc atggggtgga agacgatgaa 2100
   gaggacgagg aggagggcga ggaggacagc agcaactcgg aggatggaga cccagacgca 2160
   gaggcggggc tggcccctgg ggagctgcag cagctggccc aggggccgga ggacgagctg 2220
   gaggatctgc agctctcaga ggacgactga 2250
<210> 2
   <211> 2253
   <212> DNA
   <213> Mus musculus
<400> 2
   atggctgcgt ctcgcgctcc ccgcaggcgc ctggaggacc tcagtgtgga cgagttcctg 60
   gcttccggct tcgagtccgg atccgagtcg gagctggagg gcgccgcgga ggcggcggcg 120
   gaggagcgca gggcgcgagg agccgcgtgg aaccgggagc ggcggggcgc gcgcacctcc 180
   ccgggccccg caggacgccc gcgtaagggc cgcgcctctg agcacaaaga ccagctctct 240
   cggctgaagg acagagaccc cgagttctac aagttcctgc aggagaatga ccggagccta 300
   ctggacttca gtgactcgga cagctctgcg gaagaagaag agccattcca ctccctgcca 360
   gacacgctgg aggaagcgag cgaaacagag gaagacggag gagaggacag tgacgcgttg 420
   cccagagggc tgaggagcaa gaagaatgag cctgtacccg tgaccctcgc catggtggaa 480
   aggtggaggc agggctccag gcaccacctt agtcccaggc tgttccatga agttgtacag 540
   gcgttccgag cagctgtagc caccacccaa ggagagcagg aagctgctga gacttgcagg 600
   ttccaggttg cagatagtgc tgtgttcaat gctctggtta ctttctgcat tcgagacctc 660
   tgtggttgcc ttcagaagct gctgtttgga aagacaccaa aggatagcaa taggctgctg 720
   ccatccagta gcccactgtg ggggaagctc cgtgtggatg tcaagacata cctaagtgcg 780
   gtkgtgcagc tggtagcctg tctagcggaa gccacagtgt ctgcagctgt cctgcagcat 840
   atcagcagct tggttcctta cttcctgact ttcccgaagc agtgccgaat gctgctcaag 900
   aggatggtgg ttctgtggag cacgggtgaa gagtctctgc gggtcctggc cttcctggta 960
   ctcatcagag tctgtcggca caagaaggaa gccttccttg gtcccattct gaagcaaatg 1020
   tacatcatgt atgtgagaaa ctgcaagttc acctccccca gtaccctccc cctcataagc 1080
   ttcatgcagc ggacactgac tgaaatgctt gccttggacc ccagcgtctc ctatcagcac 1140
   gccttcctct acatccgcca gcttgccgtc cacctgcgga atgctatgac cacaggcaag 1200
   aaggagacac accagtctgt gtacaactgg cagtatgtgc actgcctcta cctgtggtgt 1260
   cgwgtcctga gtacccttgg ttccagtgag atcctgcagc cgctactcta ccctctctca 1320
   cagatcatca ttggctgtat caagttgttg cccactgctc gattttatcc attgcgcatg 1380
   cattgtgtac gtgccctgac actgctgtcc cagaccatcg gcaccttcat acctgtcctg 1440
   cccttcattc tygagatttt ccagcaggtg gacttcaata ggcggccagg tcgcatgagc 1500
   tccaagccca tcaacttctc tgtgatcttg aagctgtcca gcaccaacct gcaggagaag 1560
   gcgtaccggg acgggctgct ggaacagctg tgtgacctta ctctggaata cctgcacagc 1620
   caggcccaca gcatcgcttt cccagagttg gtgttgccta ctgttctaca gctgaaatct 1680
   tttctccggg agtgcaaagt ggctaactac tgccggcagg tgcgccactm gctggagaaa 1740
   gtgcaagaga atgcacaaca tatccaaagt cttcgacaga gcgcgacctt cagcgtgtct 1800
   gaccggacgg cagtggatgc gtgggagaag caggttygtg aagaggggac cccactcacc 1860
   agatactacg gccactggaa gaagctgagg gaccgtgaaa tccagctgga aatcagtggc 1920
   aaagagcggc tagaagacct gaacttccca gagatcaaaa ggcggaaggt ggaagacagg 1980
   aaggatgaag acaggaaaga attaaaggac ctgtttgagt tggacagttc tgagggcgag 2040
   gacagcaccg acttctttga gagaggagta cctaggctcc cagaagctca ccaaggactg 2100
   aaagaagatc aggaagaaga agataaagaa gaaggtgaca gcgattcaga ggatggagac 2160
   acagacacgg gagtggatct gagcgaactg tggcagctgg ctcagggacc acaagatgag 2220
   ctggaggatc ttcagctctc agaagaggac tga 2253
<210> 3
   <211> 749
   <212> PRT
   <213> Homo sapiens
<400> 3
   Met Ala Ala Ala Gly Ser Arg Lys Arg Arg Leu Ala Glu Leu Thr Val
    1               5                  10                  15
   Asp Glu Phe Leu Ala Ser Gly Phe Asp Ser Glu Ser Glu Ser Glu Ser
               20                  25                  30
   Glu Asn Ser Pro Gln Ala Glu Thr Arg Glu Ala Arg Glu Ala Ala Arg
           35                  40                  45
   Ser Pro Asp Lys Pro Gly Gly Ser Pro Ser Ala Ser Arg Arg Lys Gly
       50                  55                  60
   Arg Ala Ser Glu His Lys Asp Gln Leu Ser Arg Leu Lys Asp Arg Asp
   65                  70                  75                  80
   Pro Glu Phe Tyr Lys Phe Leu Gln Glu Asn Asp Gln Ser Leu Leu Asn
                   85                  90                  95
   Phe Ser Asp Ser Asp Ser Ser Glu Glu Glu Glu Gly Pro Phe His Ser
               100                 105                 110
   Leu Pro Asp Val Leu Glu Glu Ala Ser Glu Glu Glu Asp Gly Ala Glu
           115                 120                 125
   Glu Gly Glu Asp Gly Asp Arg Val Pro Arg Gly Leu Lys Gly Lys Lys
       130                 135                 140
   Asn Ser Val Pro Val Thr Val Ala Met Val Glu Arg Trp Lys Gln Ala
   145                 150                 155                 160
   Ala Lys Gln Arg Leu Thr Pro Lys Leu Phe His Glu Val Val Gln Ala
                   165                 170                 175
   Phe Arg Ala Ala Val Ala Thr Thr Arg Gly Asp Gln Glu Ser Ala Glu
               180                 185                 190
   Ala Asn Lys Phe Gln Val Thr Asp Ser Ala Ala Phe Asn Ala Leu Val
           195                 200                 205
   Thr Phe Cys Ile Arg Asp Leu Ile Gly Cys Leu Gln Lys Leu Leu Phe
       210                 215                 220
   Gly Lys Val Ala Lys Asp Ser Ser Arg Met Leu Gln Pro Ser Ser Ser
   225                 230                 235                 240
   Pro Leu Trp Gly Lys Leu Arg Val Asp Ile Lys Ala Tyr Leu Gly Ser
                   245                 250                 255
   Ala Ile Gln Leu Val Ser Cys Leu Ser Glu Thr Thr Val Leu Ala Ala
               260                 265                 270
   Val Leu Arg His Ile Ser Val Leu Val Pro Cys Phe Leu Thr Phe Pro
           275                 280                 285
   Lys Gln Cys Arg Met Leu Leu Lys Arg Met Val Val Val Trp Ser Thr
       290                 295                 300
   Gly Glu Glu Ser Leu Arg Val Leu Ala Phe Leu Val Leu Ser Arg Val
   305                 310                 315                 320
   Cys Arg His Lys Lys Asp Thr Phe Leu Gly Pro Val Leu Lys Gln Met
                   325                 330                 335
   Tyr Ile Thr Tyr Val Arg Asn Cys Lys Phe Thr Ser Pro Gly Ala Leu
               340                 345                 350
   Pro Phe Ile Ser Phe Met Gln Trp Thr Leu Thr Glu Leu Leu Ala Leu
           355                 360                 365
   Glu Pro Gly Val Ala Tyr Gln His Ala Phe Leu Tyr Ile Arg Gln Leu
       370                 375                 380
   Ala Ile His Leu Arg Asn Ala Met Thr Thr Arg Lys Lys Glu Thr Tyr
   385                 390                 395                 400
   Gln Ser Val Tyr Asn Trp Gln Tyr Val His Cys Leu Phe Leu Trp Cys
                   405                 410                 415
   Arg Val Leu Ser Thr Ala Gly Pro Ser Glu Ala Leu Gln Pro Leu Val
   420 425 430
   Tyr Pro Leu Ala Gln Val Ile Ile Gly Cys Ile Lys Leu Ile Pro Thr
   435 440 445
   Ala Arg Phe Tyr Pro Leu Arg Met His Cys Ile Arg Ala Leu Thr Leu
   450 455 460
   Leu Ser Gly Ser Ser Gly Ala Phe Ile Pro Val Leu Pro Phe Ile Leu
   465 470 475 480
   Glu Met Phe Gln Gln Val Asp Phe Asn Arg Lys Pro Gly Arg Met Ser
   485 490 495
   Ser Lys Pro Ile Asn Phe Ser Val Ile Leu Lys Leu Ser Asn Val Asn
   500 505 510
   Leu Gln Glu Lys Ala Tyr Arg Asp Gly Leu Val Glu Gln Leu Tyr Asp
   515 520 525
   Leu Thr Leu Glu Tyr Leu His Ser Gln Ala His Cys Ile Gly Phe Pro
   530 535 540
   Glu Leu Val Leu Pro Val Val Leu Gln Leu Lys Ser Phe Leu Arg Glu
   545 550 555 560
   Cys Lys Val Ala Asn Tyr Cys Arg Gln Val Gln Gln Leu Leu Gly Lys
   565 570 575
   Val Gln Glu Asn Ser Ala Tyr Ile Cys Ser Arg Arg Gln Arg Val Ser
   580 585 590
   Phe Gly Val Ser Glu Gln Gln Ala Val Glu Ala Trp Glu Lys Leu Thr
   595 600 605
   Arg Glu Glu Gly Thr Pro Leu Thr Leu Tyr Tyr Ser His Trp Arg Lys
   610 615 620
   Leu Arg Asp Arg Glu Ile Gln Leu Glu Ile Ser Gly Lys Glu Arg Leu
   625 630 635 640
   Glu Asp Leu Asn Phe Pro Glu Ile Lys Arg Arg Lys Met Ala Asp Arg
   645 650 655
   Lys Asp Glu Asp Arg Lys Gln Phe Lys Asp Leu Phe Asp Leu Asn Ser
   660 665 670
   Ser Glu Glu Asp Asp Thr Glu Gly Phe Ser Glu Arg Gly Ile Leu Arg
   675 680 685
   Pro Leu Ser Thr Arg His Gly Val Glu Asp Asp Glu Glu Asp Glu Glu
   690 695 700
   Glu Gly Glu Glu Asp Ser Ser Asn Ser Glu Asp Gly Asp Pro Asp Ala
   705 710 715 720
   Glu Ala Gly Leu Ala Pro Gly Glu Leu Gln Gln Leu Ala Gln Gly Pro
   725 730 735
   Glu Asp Glu Leu Glu Asp Leu Gln Leu Ser Glu Asp Asp
   740 745
<210> 4
   <211> 750
   <212> PRT
   <213> Mus musculus
<400> 4
   Met Ala Ala Ser Arg Ala Pro Arg Arg Arg Leu Glu Asp Leu Ser Val
    1               5                  10                  15
   Asp Glu Phe Leu Ala Ser Gly Phe Glu Ser Gly Ser Glu Ser Glu Leu
               20                  25                  30
   Glu Gly Ala Ala Glu Ala Ala Ala Glu Glu Arg Arg Ala Arg Gly Ala
           35                  40                  45
   Ala Trp Asn Arg Glu Arg Arg Gly Ala Arg Thr Ser Pro Gly Pro Ala
       50                  55                  60
   Gly Arg Pro Arg Lys Gly Arg Ala Ser Glu His Lys Asp Gln Leu Ser
   65                  70                  75                  80
   Arg Leu Lys Asp Arg Asp Pro Glu Phe Tyr Lys Phe Leu Gln Glu Asn
                   85                  90                  95
   Asp Arg Ser Leu Leu Asp Phe Ser Asp Ser Asp Ser Ser Ala Glu Glu
               100                 105                 110
   Glu Glu Pro Phe His Ser Leu Pro Asp Thr Leu Glu Glu Ala Ser Glu
           115                 120                 125
   Thr Glu Glu Asp Gly Gly Glu Asp Ser Asp Ala Leu Pro Arg Gly Leu
   130 135 140
   Arg Ser Lys Lys Asn Glu Pro Val Pro Val Thr Leu Ala Met Val Glu
   145 150 155 160
   Arg Trp Arg Gln Gly Ser Arg His His Leu Ser Pro Arg Leu Phe His
   165 170 175
   Glu Val Val Gln Ala Phe Arg Ala Ala Val Ala Thr Thr Gln Gly Glu
   180 185 190
   Gln Glu Ala Ala Glu Thr Cys Arg Phe Gln Val Ala Asp Ser Ala Val
   195 200 205
   Phe Asn Ala Leu Val Thr Phe Cys Ile Arg Asp Leu Cys Gly Cys Leu
   210 215 220
   Gln Lys Leu Leu Phe Gly Lys Thr Pro Lys Asp Ser Asn Arg Leu Leu
   225 230 235 240
   Pro Ser Ser Ser Pro Leu Trp Gly Lys Leu Arg Val Asp Val Lys Thr
   245 250 255
   Tyr Leu Ser Ala Val Val Gln Leu Val Ala Cys Leu Ala Glu Ala Thr
   260 265 270
   Val Ser Ala Ala Val Leu Gln His Ile Ser Ser Leu Val Pro Tyr Phe
   275 280 285
   Leu Thr Phe Pro Lys Gln Cys Arg Met Leu Leu Lys Arg Met Val Val
   290 295 300
   Leu Trp Ser Thr Gly Glu Glu Ser Leu Arg Val Leu Ala Phe Leu Val
   305 310 315 320
   Leu Ile Arg Val Cys Arg His Lys Lys Glu Ala Phe Leu Gly Pro Ile
   325 330 335
   Leu Lys Gln Met Tyr Ile Met Tyr Val Arg Asn Cys Lys Phe Thr Ser
   340 345 350
   Pro Ser Thr Leu Pro Leu Ile Ser Phe Met Gln Arg Thr Leu Thr Glu
   355 360 365
   Met Leu Ala Leu Asp Pro Ser Val Ser Tyr Gln His Ala Phe Leu Tyr
   370 375 380
   Ile Arg Gln Leu Ala Val His Leu Arg Asn Ala Met Thr Thr Gly Lys
   385 390 395 400
   Lys Glu Thr His Gln Ser Val Tyr Asn Trp Gln Tyr Val His Cys Leu
   405 410 415
   Tyr Leu Trp Cys Arg Val Leu Ser Thr Leu Gly Ser Ser Glu Ile Leu
   420 425 430
   Gln Pro Leu Leu Tyr Pro Leu Ser Gln Ile Ile Ile Gly Cys Ile Lys
   435 440 445
   Leu Leu Pro Thr Ala Arg Phe Tyr Pro Leu Arg Met His Cys Val Arg
   450 455 460
   Ala Leu Thr Leu Leu Ser Gln Thr Ile Gly Thr Phe Ile Pro Val Leu
   465 470 475 480
   Pro Phe Ile Leu Glu Ile Phe Gln Gln Val Asp Phe Asn Arg Arg Pro
   485 490 495
   Gly Arg Met Ser Ser Lys Pro Ile Asn Phe Ser Val Ile Leu Lys Leu
   500 505 510
   Ser Ser Thr Asn Leu Gln Glu Lys Ala Tyr Arg Asp Gly Leu Leu Glu
   515 520 525
   Gln Leu Cys Asp Leu Thr Leu Glu Tyr Leu His Ser Gln Ala His Ser
   530 535 540
   Ile Ala Phe Pro Glu Leu Val Leu Pro Thr Val Leu Gln Leu Lys Ser
   545 550 555 560
   Phe Leu Arg Glu Cys Lys Val Ala Asn Tyr Cys Arg Gln Val Arg His
   565 570 575
   Xaa Leu Glu Lys Val Gln Glu Asn Ala Gln His Ile Gln Ser Leu Arg
   580 585 590
   Gln Ser Ala Thr Phe Ser Val Ser Asp Arg Thr Ala Val Asp Ala Trp
   595 600 605
   Glu Lys Gln Val Xaa Glu Glu Gly Thr Pro Leu Thr Arg Tyr Tyr Gly
   610 615 620
   His Trp Lys Lys Leu Arg Asp Arg Glu Ile Gln Leu Glu Ile Ser Gly
   625 630 635 640
   Lys Glu Arg Leu Glu Asp Leu Asn Phe Pro Glu Ile Lys Arg Arg Lys
                   645                     650                     655
   Val Glu Asp Arg Lys Asp Glu Asp Arg Lys Glu Leu Lys Asp Leu Phe
               660                     665                     670
   Glu Leu Asp Ser Ser Glu Gly Glu Asp Ser Thr Asp Phe Phe Glu Arg
           675                     680                     685
   Gly Val Pro Arg Leu Pro Glu Ala His Gln Gly Leu Lys Glu Asp Gln
       690                     695                     700
   Glu Glu Glu Asp Lys Glu Glu Gly Asp Ser Asp Ser Glu Asp Gly Asp
   705                     710                     715                     720
   Thr Asp Thr Gly Val Asp Leu Ser Glu Leu Trp Gln Leu Ala Gln Gly
                   725                     730                     735
   Pro Gln Asp Glu Leu Glu Asp Leu Gln Leu Ser Glu Glu Asp
               740                     745                     750
<210> 5
   <211> 15105
   <212> DNA
   <213> Homo sapiens
<400> 5
   ggcggcggaa gtgcgcagcc gcgcggcatt ctggggccgg aagtggggtg cacgcttcgg 60
   gttggtgtca tggcagctgc ggggagccgc aagaggtaag ccgcgggtcc gagggccgat 120
   ttggcctccc ggtgggtgtc tgtatccaag ggggcttttc ttgctcctct caacggggct 180
   tgggccaact tggccttccg gccactttga cttcttcctt aaccgcaggc gcctggcgga 240
   gctgacggtg gacgagttcc tagcttcggg ctttgactcc gagtccgaat ccgagtccga 300
   aaattctcca caagcggaga cacgggaagc acgcgaggct gcccggagtc cggataagcc 360
   gggcgggagc ccctcggcca ggttagtggg gacatgcgtg agcagaacct cttgctcgtc 420
   ctgtcccacc ccggttgggt tgctgacctc gcctggggtc tcagggccaa ggcctaagga 480
   tcgccgacct ctgcccccca gtcctttcag cttaacttac tctttcatcc gatgtttact 540
   gagctcctgt tccgtgccaa actctgtcct aggcagggag cgtatgttgg tgagttgtgc 600
   aaaaatctct tcccgatcta ggagaactct cacgttcttg ggtgaaatca ccgcctgcag 660
   ttcggtagaa gagagataag tgctggggag ggaaaggaag gacagagggg ctgaagagag 720
   agttgcaggg gagccgagtt gctctgttag aaggagcgga ggctgtgctc ggtggctcac 780
   gcctgtaatg ccagcacttt gggaggccga ggcgggcaga ttacctgagg tcaggaattc 840
   gagaccagcc tgaccaacac ggaaaaatca tgtctctact aaaaatataa aaattagccg 900
   ggcgtggtgg gcacctgtaa tcccaactac tcgggaggct gaggcaggag aatcgcttga 960
   acttgggagg cggaggttgc agtgagccga gatggcacca ttgcattcca gcctgggtga 1020
   gagagtgaga ctccatctca aaaagaaaaa gaaagaaatc aaaagataca aaaattagcc 1080
   tggcgtggtg acacacacct gtcgtcccag ctactcggga ggctgaggca ggagaatcgc 1140
   ttgaacccgg gaggcggagg ttgcggtgag tggagattgc gccactgcac tccagcctgg 1200
   gcgacagagt gagattccgc ctcaggagaa aaaaaaaaaa aaaaaaaagc ggaggtggtt 1260
   gtttgagaag atgacgtctg aataaagacc tcgaacaagg aggccacaca gacagtaggg 1320
   gaaacgtgtc ttaggatttc aggcagcaga aaacagcaaa agccttgcag tgggagcatt 1380
   tggggagagt acagaggtaa acgtggctac ggcaggagtg agggggtgaa taggggtaga 1440
   tgagcccaga agggtgatga agagccaggt gccgtagacc gtcataagat tgcctcgtaa 1500
   aacagggagt tacttctggg tttgagcggt ggagtgcctt gtaggtgaca agggtggaaa 1560
   cagagagacc tttaggttaa ctgagaattg aactatgtgg tggtttgaga ggaggcacga 1620
   acggttttga aggtgttgct gatgagatct gatgacaagt tgcgtgctgg gtattagaga 1680
   cagagatttg cctttagtct gagcctctgg aagggtgaag ccaccatcag gcaaaagtag 1740
   cgcagggcac aggcacagca agtccaggga agaccgcggc tgggggcctg tgtagcgcag 1800
   ggcgcaggca cagcaagtcc agggaagacc gcggctgggg ggcctgtgta gtgtgtttga 1860
   gggtatgttg tagaggttgt tgggtaggag acgtgagtct gccaaaaacg tacctcctgg 1920
   caatcctgtc ttgctagttg gtaacccttg tgaagggagt agactgaccc tgtaggccac 1980
   tccccgcccc ctctacttcc acttcccact ggggtcgctg acttctgcct cctcagccgg 2040
   cgtaaaggcc gtgcctctga gcacaaagac cagctctctc ggctgaagga cagagacccc 2100
   gagttctaca agttcctgca ggagaatgac cagagcctgc taaacttcag cgactcggac 2160
   agctctgagg aggaagaggg gccgttccac tccctgccag atgtgctgga ggtgagggcg 2220
   tgggccaaac cagaaggggg gcacttgtct ctacactctc cttcagctca gcctttctgt 2280
   gcaggaagcc agtgaggagg aggatggagc ggaggaagga gaagatgggg acagagtccc 2340
   cagagggctg aaggggaaga agaattctgt tcctgtgacc gtcgccatgg ttgagagatg 2400
   gaagcaggca gcaaaggtga gcagcagcca ggggcgggca gctgggtgcc caggcagaaa 2460
   tctggccttg cctcacctga ggtgagcagc agcgaggggc gggcagctgg gtgcccaggc 2520
   agagatctgg ccttgcctca cctgaggcgc ctgaggctgt gctggtggga ggggctgttc 2580
   tccacgcagg ggacgcttgg agccttcttc agcgcaagtg acaacacgtt gctcctcctg 2640
   cttgcttggt tgccccagga ctgtgtgttc acttttgggt agattccagg ggctctgttg 2700
   gcgccccact ccccaaagtc agtcccgctg tgggtgggac ggactgtgcc tttgttggtt 2760
   ggtggagctg gggtccttct gagcctctca cagtgttttt ttccagggac aaggatacgg 2820
   agagctccag ataccacctg gaggtggcca tagtccagga tctggaactc ccagtccttt 2880
   tcctggggct cctgagccag actccctcct cttcccagag aattctagac tttgtttcca 2940
   tttttgttat cagtatgggg ctctgtgcct ccccccaacc tctgccctat gtctgagggt 3000
   gagggtgagg gtgcctttct ctggggctgc cgtttccact ctgccaagtg cagtctcagc 3060
   tcccctgacg cccctggtac tcttgctcct tcagcaacgc ctcactccaa agctgttcca 3120
   tgaagtggta caggcgttcc gagcagctgt ggccaccacc cgaggggacc aggaaagtgc 3180
   tgaggccaac aaattccagg tcacggacag tgctggtgag cttgggggga gcctggcatc 3240
   caggctgtct gttgcgttct ctgtcccgtg agtacatcca ggccttttcg ttgcagcatt 3300
   caatgctctg gttaccttct gcatcagaga cctcattggc tgtctccaga agctgctgtt 3360
   tggaaaggtg gcaaaggata gcagcaggta agaggggagg gggtgaaggg gggtagggtg 3420
   gaaggtgggt cgggaccaca cacaggagaa gccagaggcc ttgtggctag gacagagaca 3480
   tggcaacaga gccagcgtct tcttggggac cctgagaagg cagcggggca cgagggaccg 3540
   ttgggagagg ctgggcactt ggggccagtt ggagcggccc caccttcttt ccttcctgaa 3600
   aagggaagtt gtctgctcag agtctcagaa ggtgtggcgt gtgcagctgc ctttgctctc 3660
   agattttccc aggttagggt tcttgctcta tttccctttg taagaacttg taagtcctgt 3720
   ccatactcct tgatctctgc actatgactg gcctgtttcc tggagctgtg tgtgtctgta 3780
   gacaggggcc tcatcaatgc tgagaacctg agggcctcaa cttccccttt agagaaagat 3840
   tgggcagaga aggccgtgga aggttttcag agaagagaga cgcacggcca agcatttggt 3900
   agagcatgga gcacgttggg gttccggggc tgtgcagcca tgtgaccttg aggcagtgga 3960
   ggcctagagt tggaactgcc ccaggacacc ttcaaaggaa gaaaggagac ccacaggaga 4020
   ggtctcgccg acagcggctc aggagacagg ggcactgttg ctggtggtag tggtcggggc 4080
   tggacggata gaacagagcc atgtccacat ggaggcagtg ctgtccgaag gggacaagga 4140
   agctggcaga tgggggtgga gggacaggca gcgattggga ggcagcacgc actgcagcct 4200
   gagccaccag gtcagggacc cctgtcgagg cagcagctgt accggaggga gtcaggccgt 4260
   ggggagcaga gctgcctcct gggtctgggt gggcagctgc aggtgcccag cagccagtgt 4320
   cagcacaggt gtgaggaggg tcgaggacgg ggggcctttc tggggccctg ccctttgtcc 4380
   ttcccatgct tcagcagatt ttagtgagcg cctactgcgt gggctctggt aaatgttttg 4440
   ttttgttttg ttttgtttgt ttggtttttg gttttttttt ttccttacga gataggatct 4500
   tgctctgtcg cccaggctgg aatgcagtga tcatagctca ctgcgacacg taccttctgg 4560
   attcaagtga ttctctcacc ttagcctccc aagtaactgg gactccgcca ccaggcctgg 4620
   ataatatttt gtatattttt gtagagacgg ggtctcactg tgttgttcag gctggtctca 4680
   aactcctggg cttggagttt gatcctcctg ccttggcctc ccaaagtgca gtgagtactg 4740
   gcatgagtct ccacacctgc cctctgattt tcttttcttt gtgcagcaaa tgtgggccaa 4800
   aggaaaccag tctgcggcag ttggtggtgc ctgggcgacc ccagaggtgc tgagtaccag 4860
   ctgctgggct tagggacctc gtgtggtctc acggtgggga tgtgatcaag gggacctaga 4920
   aaaggtttat gtctgagagg gagttggagg ctgggacttc cggggactct tagggcggtg 4980
   gccaaccctg ggcagggcag acaggagttc aaggccactg aaggaggagc taatgcactt 5040
   gagagggtcc tcctaagccc ctgtgtctgt ccagctgtaa ggggccctga gctttttgga 5100
   gtggagagac ggggggtctc tgcagagtcg tagaggctat gctggccagg gcacgcgcca 5160
   acatgctgag cagcctcgcg tccgagccgt ggggcctccc agccaggggt gggtggtctc 5220
   tctgcaggat gctgcagccg tccagcagcc cgctctgggg gaagcttcgt gtggacatca 5280
   aggcttacct gggctcggcc atacaggtgc tattctggtg gggagggcac gggggcctgg 5340
   ctgtatctgg tggtcggtcc ttttttgtat cccagaatac atgggtttgg ggcttcactg 5400
   tcccctcctg cccccagctg gtgtcctgtc tgtcggagac gacggtgttg gcggccgtgc 5460
   tgcggcacat cagcgtgctg gtgccctgct tcctgacctt ccccaagcag tgccgcatgc 5520
   tgctcaaggt tcgtggccca gtcccctccc tgtgtctgtc atggggtcgg ggggccacac 5580
   ggctaccccc accacatccc actcctggcc agggcacagg tggtgcccac actccactgg 5640
   ctccgcttgg ctagagaggc cacagaagca cctggccccc acccccacct ggggtttctg 5700
   tctcaggcag tccctgcctg cccgggcagc gcggctcagt ccgtgagcaa agccacggtg 5760
   aggtatcttc ttcccggtgt gatctcacat acgtcggcgt gtctgacgtg gtaaactttt 5820
   gtggtcctct tctcacaagg agcaacacac ctgttctgtt cacttctgta actgagcacc 5880
   taactcacgg ctcccccgag gtgctttggg aagagctggt ggccatggga gcctttgcct 5940
   ggctggggag ggctgtgctg gccctggggc atccctgctg aggaggctgg ggggccacca 6000
   gtgacgtctg accttctgca gagaatggtg atcgtatgga gcactgggga agagtctctg 6060
   cgggtgctgg ctttcctggt cctcagcaga gtctgccggc acaagaagga cactttcctt 6120
   ggccccgtcc tcaaggtagt ggtgggccct gcgtctgtgt ccctcagcat ctgcattgga 6180
   aatctcggcc taagggcagg gcgggctgcc tttgtggttg gtgcccctca ctggaccctc 6240
   actgcagctc tgagcagacc tgggcccttg gatcacgaat gtctcacaga gcacctgggg 6300
   gtggtgggca gggaagagag ccctcggcct tctcagggcc ccacctgacc ctgcttcaca 6360
   cagcttcccc aggggagggc ctctctggct ggaggaggac actgggtgtt gggacctgag 6420
   gccatggcca gggtacagtc ctactgcccg tccccaagcc atgggtggcc tgcatgtggg 6480
   gaccctcagc ccccagaggg gcccagtgtg cagcaggagc ttctgcccca gcttctccca 6540
   ggcctgaggc tggtgggcac ttgggggtgg gggctgtgca aggggctacg gctcttcctc 6600
   gaggcccagc tctgagggaa aggcccaggt gttcacaggg gccctggagt gggcggtgga 6660
   ggtgcatggc cctgatccca ggtggctctg acccgggtct ctccgcagca aatgtacatc 6720
   acgtatgtga ggaactgcaa gttcacctcg cctggtgccc tccccttcat cagtttcatg 6780
   cagtggacct tgacggagct gctggccctg gagccgggtg tggcctacca gcacgccttc 6840
   ctctacatcc gccagctcgc catacacctg cgcaacgcca tgaccactcg caagaaggtg 6900
   tgtggtgggg cccttccagg ctcatgctgg gcatggggtg gggcagccca ggtgcccgac 6960
   ccaaggcagg gcctggggcc tccccgaagc ccctgtctgg agacagccca gcaccctggt 7020
   gcagtcggtc cttgcaggtg ggggaagggt ggatgggttg agaccccgtg tgcaagatga 7080
   ggaaatgatt cctgtgccgg cccaggagga acgtgcatca gcctgacttg tcagcctggc 7140
   cagtagctga cgtggttctc tctgaccagg aaacatacca gtctgtgtac aactggcagt 7200
   atgtgcactg cctcttcctg tggtgccggg tcctgagcac tgcgggcccc agcgaagccc 7260
   tccagccctt ggtctacccc cttgcccaag tcatcattgg ctgtatcaag tgagttgtgg 7320
   ggtgggcagg gttgtgcggg agggtcagga gaagcaaatt tgctgggact gtgtgggcgg 7380
   tgcctctggc gttcagggcc ttggggcctg agtctgtgtc ctggccgtcc ctgaggaagg 7440
   gctggggtcc ctgtacctgc tacggggaga tgctctggat tctggagagc tagggctggt 7500
   gggcacctgt gacatgagct cctccaacag cggtttagcc gcctcgggtg ccacccagcg 7560
   tgtgttctgg gggcttgtgt gcagtttgca gtgagtttgg ttcattacgt ggggttcttg 7620
   ggtggagcac atctgatgca gtgaactgca ttttgggtgt gagcgcttag gagggtccag 7680
   gcacggtagg ggctgcagca ggaaaaggtg ggagcagtac tgtggcctct ttggccagga 7740
   gggggcatct gtctggctga gccttagaaa ctcaaggctg ggaagggagg tgggaagtcc 7800
   aagggaagaa gtaggaaagg cgggaacagg gaggagagca ggcaggaaca gatgacccct 7860
   gccaccgtgt tgaaataaag ctgaaagctg ggtaaggtac ctgcagcccc atagctgggc 7920
   aaggggtgca ggtccctgcg gttcagacgt gccttgtcct gctttagggg tctgatgtcg 7980
   gtgagtgggg gaggaggtcc aagacagcag ggggaggggc aggggctgcc agagccgggg 8040
   cctctgctca ctcggccttc ccacccccag gctcatcccc actgcccgct tctacccgct 8100
   gcgaatgcac tgcatccgtg ccctgacgct gctctcgggg agctcggggg ccttcatccc 8160
   ggtgctgcct ttcatcctgg aggtgagtga ggctgtggtg ggcgtgtggc acctctgcct 8220
   gctcctgtag ggagcatctg ctgctccggg cgtctgtgct gagttgtccg gcgacttccc 8280
   ggagccctgg ccgcctcctt gtcacgggtg tcacgaggac agtgctcctc cgtgtgctgg 8340
   gggcagtgtt ggggaacgtg ggaccactgg ggatgaaggc ggctgctgct agggctgtgc 8400
   ttgaggatgc cgggacctga ccctgtaggt gcttgcccaa gactggagta ggcaggaggc 8460
   gagatgggcc aaaacccaag tctgattgct gaactgtaca ctgaacagtg ccctgccctg 8520
   acagttgtgt gcatgtgtat gcatttgtat ggaggatgtg tgtacatctg ggtgggtgcg 8580
   tttgtctgtg catgcatgcg tgtgtgtgca ccatctgggt gcatgggtct gtaaatctgt 8640
   gtgtgtgtgc atatcagttc atgtgtgcat ctgtatgtgt gtatgcacgt gtatccatga 8700
   atgcctgtgt gcctgcaggt gtgtgcatct gtgcgtgtgt acacctgtgt gtatgcatgt 8760
   gtgtaccttt gcgtgtacct ttgcgtgtgt gcacctgtgc atgtgtcttt gtataccagt 8820
   gtgtacctgt gtgtacctgt atgcatgcac atgcgtgtgt acctgtgtgc acctgtctgc 8880
   atgtgtgtac ctgtgcgtgt gtgcacctgt gtgcatgcat ttgcgtctgc atgtgtctac 8940
   ctgtgcatgc atgaaccttt gcatgtgtgc atctgtgtgc atgcatgtga tgtgtgtctg 9000
   cgtgcatcta tgtacctgtg tgcacctatg tgcatacaca cgtgtctgtg tgcacttgtg 9060
   tgcatgcatt tgcatctgca tgtgtgtact gtgcgtgtgt acctgtgcgt gtacctgtac 9120
   acttgggtgc atgcatgcac gtctgcttgt gtgtgcctgt gcgtgtgtgc acctgtgtgc 9180
   atgtacgtgc gtctacgtgt gtgcctgtgc atgtgtgcgc ctgtgtgtac ctgtgtgtat 9240
   gcatctttgc acgtgcacat gccactcagg tagggaaaga ttggagtccg aagtttcaac 9300
   tttcagtggg tgggttaggc cccaccccgc tgtaaattta ggaattcacg atctccaccc 9360
   tgtttatcta atgagttctc agcctcatga aggcccagag tcgtgtcaca gctgtccttg 9420
   gggctgggtc ccaggttgct gggtccagaa ggtatggaag ccccaggcac gttctgattc 9480
   cccttccact gaggcaggga tgctgaacat ctttaggaag ccatgttcac tcccatggca 9540
   gccagcagtg gtctctgatt gcccagccct tggcctggcc cctgtgtctg tgggcctcca 9600
   gctctgctgc ccagctccag gcatgctttg tgtctgtttc ccttgtccaa tctccttggc 9660
   tacgtgcttt cttactctct tgcagtgtct gtttcttcac ttgtgcactg ccctggttca 9720
   ctgcagccgc accctgtagg cccctctcac gcagggatgc aggcctctcc tctccggaaa 9780
   aagcaaaccc taaaagctaa aacaaagccc tcagctgtag gccgtgcctg cccttccccg 9840
   gtgcctggac aggaagccag tcgcctgccc atacttttgg cccaggctag agaagggcag 9900
   tgtcctccca gaggttcatc agtaccaggg cgttttccca tctggacctg agctcagctg 9960
   tctggcagcc acccctgctg agtggggtgt cttgctgggg cctccaccct tgggcccccc 10020
   ataatctgct tctgtcctct ggtgccccag catgtaccct ggatctctct ggttcacagc 10080
   ctgagggctc ctagtggttg gggaggggtc acaagactga gaggccaggc tgactctttc 10140
   tctgctcctc ctggcatgtc ctacggaggt gcatggcctg tggcttctgt ggagggtgtg 10200
   ggaggggccc cccaggcctc ccgtgacctc catctgtccc gtcctgtgtc tggcactctt 10260
   tgctgttgct gctgcgtctt ctggttgctc gggacggagc cccatgtggc attgctgtgc 10320
   tgagggccag gatgggcctc agtgccatgt tgtcaggaat gggggctgtc ctggtactct 10380
   gtgtggcagg gacctctagg tctccagacg tgggtcctta gtgcttccca ggattttggg 10440
   agagggcccg tgttcctgat ccttccctgc tgatcagagc cccactcggg gacacgccag 10500
   gctgtgtggg gccatggggc tgggaccgtg cctagctgct tatctcttgt ttcgggttgg 10560
   gtctcctcgt gctgaagcct gaggaccagg gtgaccaggg tgcagccagg tgcagggcca 10620
   aagggaccag gggaccaggg gtgcagccag ggtgaagcca gggtgaccag gcatggggcc 10680
   gagagagcct gacactggcc cttggggcag atgttccagc aggtcgactt caacaggaag 10740
   ccagggcgca tgagctccaa gcccatcaac ttctccgtga tcctgaagct gtccaatgtc 10800
   aacctgcagg agaaggcgta ccgggtgagg ctggctcctg gggagggcct gggcagttcc 10860
   cagggtgggg ttgggggtgc tgaggtggat gggagggggc tggcatcctc caagttcaag 10920
   catggacctt catggtctcc cagggctggg gcatggagcc ccttccttgc agtccgtgcc 10980
   ctggagatgg cgctgccctg acagcctgag gggaaggggc ctgagtgcag ccccagcctc 11040
   tccctgatgc actcggccct ctctcctgct cttcaggacg gcctggtgga gcagctgtac 11100
   gacctcaccc tggagtacct gcacagccag gcacactgca tcggcttccc ggagctggtg 11160
   ctgcctgtgg tcctgcaggt gtgtgtcctg cccacaccgg ctcgtggccg actcagactg 11220
   tcttataacg ggcttggctg ccccaggctg gtaggaggtg cccttgtccc gggtacctgg 11280
   gactggggtg gaacctgact gctggcaacg cgccccgtga ggcccccttg gaggggctga 11340
   tgaggggttc taagccattc aggaggtttg ggggcaggcc tgggcagtgg gctgaggacc 11400
   ctgggagcac aacagcctcc tccccaggtg ggaaccacaa ggctgatctt tgcttcgggg 11460
   ttgggactga gcatgccgac cctggctcag gctggtacgc tgatcgcaca cttccccagg 11520
   ccatcccggg tgtggggagt gggtggagtg gcttctcagg tggcaggaag gcctggcctg 11580
   ccccgccaaa taccccacat cagcctcata ggaaggccca gcctgccccg ctaaataccc 11640
   cacgtcagcc tcatcttagg caggagtggg gtggaggagg gggttctcct tatcctcaga 11700
   aggtccttct gggcccccac gggaggtctg tttgctctca gccgtgatgt ttccagcctc 11760
   agggagcgct gtgctagtgg aaggggtggg ggcctgctgt ctggccctgg tgctggaatc 11820
   agatgtgccg actataggtc tgtgcagtgt ggggaggagg gatctgctca catgagccac 11880
   aattggtcag aggcttatcc agatagaggt gtgtgcatgt gtgtgtactc acacacggcc 11940
   acacatgtca catgcacaga gccgggaccc cctttctggg gcactcacag catgggccac 12000
   agcctctgtt cctgcccatg tcctacttgg gtggtgatac ctggcattgg ggcatctctg 12060
   cttctggact caagggccca gggtcgggtt ctggggtagg ggtcagaaaa tgtttttggt 12120
   gaggggccaa atggtaaacg tggctttcta cggatgttca gctgcacagg caactgcaga 12180
   accatgcaat gcacacgtgt ggctgcacgt cagcgagact gtattttatt agtagtagta 12240
   gtattgtttg agatggagtc tcgctgtgtt gcccaggctg gagtgcaatg gtgcgatctc 12300
   ggctcactgc aatctccatc tcctgggctc aagcgattct ccagcctcag cctcccaagg 12360
   agctgggttt acaggcgccc accaccacac ccagctaatt ttggtatttt agtagagacg 12420
   gggttttgcc acgttggcca ggctggtctt gaactcctga cctcaggtga tccacccgcc 12480
   tcagcctccc aaagtgctgg gattacaggc gagagccacc acgcctggca taaaacttta 12540
   tttccagagg tgagtagtgg gcagggttgg cccagagggt gggctgattc tgcctctgcc 12600
   atcccagctg caacagctat gcacttgagc cctgagatgg gatccatgtc ccctcctggg 12660
   gtatccccgt ggccaccacg cgtggttttg cacaggacct gggccagctg tgcacatgga 12720
   gcggtcctgg gcttcagtgg ctgacccctc ccttccgcag ctgaagtcgt tcctccggga 12780
   gtgcaaggtg gccaactact gccggcaggt gcagcagctg cttgggaagg ttcaggagaa 12840
   ctcggcatac atctgcagcc gccgccagag ggtttccttc ggcgtctctg agcagcaggc 12900
   agtggttagt gggccctggg ggtagtgcca cctgagggca cctgccaggg tatagcccca 12960
   gctacatgtg ggggtttgcc cagggtgagg catgaccctg aactccccca accccccagg 13020
   aagcctggga gaagctgacc cgggaagagg ggacacccct gaccttgtac tacagccact 13080
   ggcgcaagct gcgtgaccgg gagatccagc tggagatcag tggcaaagag cgggtgcggc 13140
   tcggcgaggg gacctggggg tgtgttgtga cttcctgggt ttcagatcta gcgcactatg 13200
   acttgagacc agggcgaggg tttggaaaca gtgccaggcg gccagggccg tgcccggatg 13260
   attcgacttg gagagggggt aggtgttgga gaactggcca gaaccaggcg tttccaggga 13320
   ggggaagccc caggctgcac taggttgggg aggccatgcc ccctcaggcc tgatgggctg 13380
   gaggctccgg gcaggtggag tggctggact gacctcgtca cccaggccag tatgtgggca 13440
   ccaggggccc gtgaggagaa gcaggaaggg ctctgccttt gaccttggac atgggatgga 13500
   caacttggag gatggctttg tgatttggga acagagggga ctagaaattg gccacatggg 13560
   gccctggtgg tgggtctggc gatgcctggc cctgctgtgg ccgccagccc ctgccctctc 13620
   tcacctgagc ccctggttct ttggccttcc agctggaaga cctgaacttc cctgagatca 13680
   aacgaaggaa gatggctgac aggaaggatg aggacaggaa gcaatttaaa gacctctttg 13740
   acctgaacag ctctgaagag gacgacaccg agggattctc ggagagaggt ggggcctgcg 13800
   tggtgctccc aggggaaggg tgggcctgga gggctctgct ggacttccca gagccacgag 13860
   ggccacctgt acccatcctg cagggggctc accagtctct ggcccagctg gggccaacct 13920
   cagtgttgcc aggcttctgg tgccagcgcc ttccctcctt gaagtgaagg cctactggga 13980
   ttggtaactc tgtccccagg cctgtgacct cccagttcct ccccagggct cctctccacc 14040
   tgctggaagt cagcggaggg aagggtgttg ggagcctggc caccctcctg cccccactgt 14100
   gactttgctg gtggaccctg tgggtgggag tcatatggac tctgcttctt gttcctcagg 14160
   gatactgagg cccctgagca ctcggcatgg ggtggaagac gatgaagagg acgaggagga 14220
   gggcgaggag gacagcagca actcggaggg tgaatggtct tggggtgaga gggtgtggcc 14280
   ctgtgagccc atctggcggg agggcagagc cacgtgggcg gggggcgtgg ggctctgggc 14340
   caggcttttc cctccctggg aaggccaggc caaatgctct gttctctggc agccagcaac 14400
   agggataaat taattagtgc cgtgattaat tagtgatgag taacctctaa ggctggcttc 14460
   ttcctgataa agcaaaattt atgtagcctc catctctccc cgcagatgga gacccagacg 14520
   cagaggcggg gctggcccct ggggagctgc agcagctggc ccaggggccg gaggacgagc 14580
   tggaggatct gcagctctca gaggacgact gaggcagccc atctgggggg cctgtagggg 14640
   ctgccgggct ggtggccagt gtttccacct ccctggcagt caggcctaga ggctggcgtc 14700
   tgtgcagttg ggggaggcag tagacacggg acaggcttta ttatttattt ttcagcatga 14760
   aagaccaaac gtatcgagag ctgggctggg ctgggctggt gtggctgctg aagccccaca 14820
   actgtgggct gctgaagtca actccgcggg ggaaacttac ccttgacgtc agcagaccga 14880
   gaccagttcc cagttccagg ggaaggcttc aaggcccctg gcccttccac ccacctttgc 14940
   cctcagtatg cagacctcgt ccatttgcac caggttctgc cttcactcca ccaagtcttt 15000
   gaaatttgtt tcctttcctt tgaagtcaca ttttctttta aaattttttg ttttgcatcc 15060
   gaaaccgaaa gaaataaagc ggggggaggc agggccattg tgttg                15105
<210> 6
   <211> 23806
   <212> DNA
   <213> Mus musculus
<400> 6
   aacatgcagg atgccgccac ccacttgttg gtcctcgggt tgtacttctc tatggagttg 60
   aggctggagc tgccgtcatt gccccccacg gcgtagagcc agccgtccat ggccaccagg 120
   tcatgtgtgc tcctggggag ttggataggc aagagtaagc ccaaggggca agccaagaga 180
   caggccaaat gagtggggca cagagggagt aaccataggg acagggcaac gaaaggagcc 240
   ccctatgccc ggggggaacc caaggacctg ccgtactgtt gaggtgctgt gtgcacaacc 300
   cctggggcta cactcctgga ataaaccaga tgtagtggca gaaccactca ccacccgccc 360
   ctggccttat acacctgcgg atattcatgg gtgccacact ctcccaggca ccagccttgg 420
   tgctatatct ctccactgag ttgaggcagc ttgtgccatc attgccccct gcgacataca 480
   gggcaccctc cagcactgct actcctgctg agctgcgccg gctcagcatg gaagctacag 540
   gtgtccatga attcacctgt agagacaata ggacaggctg gaggcagacc caccaagggc 600
   cccccaaccc tgccaccaca tagactctac tcttggttgg gtatctgagg gtcaggggtg 660
   ggatatagaa agcagacccc ttgggtggca cagatggcca gaatctggga ttatgcccca 720
   tcccccccag cactttagag gaggacattg tgatggggtg cgctatgcac ctggggctca 780
   tacttctcca cggtggccag gtgtgacgag ctgtcataac cacccacggc gtacaggttc 840
   ccatctgtgg gaaggcaata tacccacgaa gctcctgcag ggtgtaggag ttttgggggt 900
   gggtgggaag gggagaggca ggaaagcggg taccacggga agcctgtcac ccaccaagtg 960
   tggccacacg cacatatcgc ctccgggtgc tcatggcagc aatggatgtc catgttcccg 1020
   tcagtgggtc atatcgttca gcactgtgga caccagaatg atgccgacgt tcagttacaa 1080
   cagttaagtc atccttgccc aggctctgtc ccctcactcc ccccaaagct acggctgaaa 1140
   tcccgggcat cacagactct atgaatagag agcaaggtgt gctgggcgga tactggctgc 1200
   tggctgcttg gactggaggg agggcccctc caggaggaga atggtgccaa ggccctgcgg 1260
   atgctgtgcc ctgaggtaag gaagccttga ggacccagta gcctggatta ggccctcatt 1320
   ctcttagcca gatgtgactt catagttaag ccagccttag ctgctgctgc tgagccgacc 1380
   ctgcaaagcc actggggccc taacccaaac tactacctgt tgaggcagga agccccatcg 1440
   tagccaccag ctgcatacag gagcccatgc agagcagcta cacctaggca gctccgcctt 1500
   gtgcccatgg agacctcggg ctgccatgtg tttgtcacag ggtcatagga ctccaccgta 1560
   gccaggtctg aggttccatc gtagctagga gaagagtccg ctactgacca aggatggccc 1620
   ggcaggcttg tgacaaacct ttcagcctac atggccagct tctatgctta cccgcccaca 1680
   gcatacagtc ggttcccgac ggcagcaact cctacacgag cccgacgtgt agacatggag 1740
   gccaccacat gccaacggtc agtccgcgtg tcatacgctt cacagtctcc gtggatcgcg 1800
   aacaggctcc caccacctag agatgggtaa ctataaataa aggggctcaa ggctgggcag 1860
   aggactctag tgtgacccaa gtacaaagac atgaaggctc caggggtggg aagagataga 1920
   gactgggaca cactagtggg ccccatccta tagggtctct gagacacaca ggtctaagta 1980
   taatgggttg ggtttatagt ctgagttggg gtggatggcc tagggcaaaa tgggaaggag 2040
   aaggggttta gcataccgac agcaaagagc acagggccag cgccctcaca tcgacggggt 2100
   ctcgtgcggc tggttcccag aacacctctc tgctcaggca gcaggtggaa cttaagggcc 2160
   tcgatgagca gatccttgca gtcagggtga tgcctcacaa ggctttcggc atccacgtgg 2220
   cccaggagga agtctcggct cagcagaggc agccgtacac acttcatcaa ctagggcagg 2280
   aagcgccatg taaggcaaaa ccctgggcgt acttggccca gggctaagac ccactcaggt 2340
   gcctatagca cctgtgagat tgggtcttga gacaggtggc tcaaccactc caaaggagtc 2400
   cctggatgga agatgctaag gacttgaaga agtaagctta taccgcagcc acctgcattc 2460
   ccttctgaca gcacgggagc tccaggacct ggcctcaccc ttggtacatg ctgcctccga 2520
   gtgtctacgt catgtttgac ccagctcagg actgcacggt acacatcttc ctctgagggc 2580
   acgttcaggc tatcactaga gaccaattcc agcacctggg ggcgagaggt cctcagacct 2640
   gacatctggg taggggaggg cccagagaac cctgaacgtg tctgagagta tcagacagag 2700
   ggcatgtgcg tgtccatgca caaacacgca cacttaacgt tcttagagtg aagaaccaag 2760
   cagagtcaag attcacagat tttgttccca gtagaggggt gaggggtgtc caaggtccta 2820
   agtagtctcg ggtacataga atcagagact gggaaatcat ggccctagaa tgcggtaaag 2880
   acaggcatct aagacaaatg attgggtgac atgagaagat ggaaacaggg ttgggaggag 2940
   atggccaggg aatgaatcat agggtgaaac tcgcaccaga aacttgaaat gaggattgca 3000
   gaacactggc tgcaagggtc tgggcttggg caatacatgg tgggggctgg gcccaaactg 3060
   gcatgttacc tgcttcagtg gttacctgct tcagtggcaa cagcatgaac tcctcagtct 3120
   tggccacatc cacgaagtgc tgcagcacgt acctgtgtgc tgccttgagc aggtcactgc 3180
   atgagtgtgt gtctgcaaat cctcgaatgc ccaggcagtt ggaagggtcc agctggctca 3240
   ggaggaactt gcagcaggca tctcgaacac cattcaactg tagaaggctg gcagctggga 3300
   gcagagtctg gggcatgaag atgggtgagg caggagggtt ccagaagagc acagagtgac 3360
   tggatattgg ggagaggcat gggggggggg cagggcacac agatctgggc tggagatctc 3420
   aggggacgtg ggtatgggaa tggtaggcct cacctgaacg ttgccctcgc ccaccacgat 3480
   ctcagctgtg tatgcaaact gcaccagctg gtctaaggct tgcgggtcaa tgtcatgcag 3540
   tgtcacatgt gtctggcggc tctcactcat ctcatctgtg aaaacagtgg attaggcggc 3600
   ctacccgggg agatctgcgg agattccccc tagggctgcc cagactgttg tatgtggtac 3660
   ttgcttgtga acatggcgtg gaagtagggg ctacaggagg ccaacaccac cttatgagcc 3720
   cggatctcct tggcagccac atgcaggacg atgtcgcaca ggaggccacg ctgccgcatg 3780
   cggctcatag ccacaaaagc atcatggtag tgccgctttg agttgtgtgc cacactgtga 3840
   ccctctctgc tcaacagctg catcgcacct tccatcgggg ctgcaggccg ggcctgccgg 3900
   ggccgagctc gctctgcctc ggggctgcag gccgcaggac taggtcaggg tttgtaccct 3960
   gtagagggcc actcacccgc ctttgagaac tcccagcaca gccaaggcag ctgcggtcta 4020
   tgcccaggag ttcgttcagt catcaggcac caaagcccat ggaggaccag cctgtcactc 4080
   actgccactt tccagggctc tagatctctc tccggtccag gaacctcgct ggagccggag 4140
   cagattcttt cataaatgta agcctgctct acctgtcccc cttcctctgc tgactacctc 4200
   tcctggggct ccgagcccct catctagtgc ccaggccact catggggagg aagttggcaa 4260
   acgttgtcct ctgaatcccc agatcccagt cannnnnnnn nnnnnnnnnn nncccgccct 4320
   cccgcccgcc ctcccactca cgccggcggc tggggcggcg gtggcgcctc aggcccgggc 4380
   cccgggctgc tgtgctcggg actctgcgtc ctgcccgccg gccgctcgcc acggggctgc 4440
   ataactccag ccgccaaagc cgcgaccctg atggacggat tgggacgcgg gtgcccgccc 4500
   ggccgctcgc tccactcggg cgctcgcccg tcgccgcaga ccctgtttct cggcgccgcc 4560
   cgccctgccg cacgcaaggt tgcaggagcc gctgccaccc gcgtccccgc tctcctccca 4620
   ggagcgcctt ccttcgtgtg gcgcctaggt tagcttcaca tcccagcgcc ccggttctca 4680
   cctgctagag cttgctgtga ctgacaaagc tccatagcac gggggtagag cgggacactt 4740
   gcaagtgtgt gtcccccccc ttaccctcag gcctcctgtg cacaagcgcg tgcaaaggtt 4800
   cagcgcccct atacatgcat atttgagcat gaggcggacg ggggctgcta gcgtctgaga 4860
   ggtgcaatta tctattgaca tggacgcagc attcttgtca cacccaggct cgccaaggaa 4920
   ttgggtgtcc tttgacttaa aaaaaaaaat atatatatat gtttatatat atatattata 4980
   tatatatata cccgtacaca taatatatat atatatatat atacatacac acatatatat 5040
   atatatatat tttttttatg tgtacgggtg ttttgcctac ttgtgtacta tataaattac 5100
   gtgtgtgcag tgcctacgga aaccagaaga gagcatcaga cctcctggga caacattggt 5160
   gatccacttc gtagaccctg ggaattggac cccagtcctc tggaagagca gcttagtgct 5220
   tttaagtccc ggccatctct atgcagccct tgcttgctcg gtcggtcggt ctgctgcctg 5280
   cctacaattt atcatctatc aatccatcca tctatccctt tatctgcttt tttcctggaa 5340
   tttgctctgt atggactgat ctcagagaca gcacagagcg agctcgcttg tctctgagcg 5400
   cggagataaa aggcgtgcgg tcgggacagc cctttacttt ttaaaaaaaa aaaagatatt 5460
   tttccccctc caccctccgg gcgtggtagg caggctgttc agatttccag ccttgtttcc 5520
   cgggtgcgag cggtcgttcc aggcggggct ttgagctgtg agagcctgag ccgagctcgc 5580
   cgccctcccg cccggggacc gcggccgcgg cggctcgaga acccgaaagc cagcagcagg 5640
   ggtggcatgg gcggggctcg cgggaggaag tcggagggcg caaggcattc gtgggcccgg 5700
   aagtgcggcg cacgcggctg ggcgcgccat ggctgcgtct cgcgctcccc gcaggtgagt 5760
   ggcctgggcg ggcgcgcgga cccgggcggg cgcctggtcg cggggtccgt tccgatcctg 5820
   acgcggtgtc actggcaggc gcctggagga cctcagtgtg gacgagttcc tggcttccgg 5880
   cttcgagtcc ggatccgagt cggagctgga gggcgccgcg gaggcggcgg cggaggagcg 5940
   cagggcgcga ggagccgcgt ggaaccggga gcggcggggc gcgcgcacct ccccgggccc 6000
   cgcaggacgg tacgcggggc tccagaaagt ctttttgcaa aacgggcagc tgtcgtgggc 6060
   tggagcagcg ggttgagctt aaggaacaca cgacgggggc ctcggggtcc gagcgatggg 6120
   aagggttgca aggttggaaa tggaggagag cttggagggg gaagcgcacg cgtggttggg 6180
   agggggaagc gcacgcgtgg ttgggagggg gaagcgcann nnnnnnnnnn nnnnnnnnnn 6240
   nnnnnnnnnn nnnnnnnnnn nnnnnnnnnn nnnnnnnnnn nnnnnnnnnn nnnnnnnnnn 6300
   nnnnnnnnnn nnnnnnnnnn nnnnnnnnnn nnnnnnnnnn nnnnnnnnnn nnnnnnnnnn 6360
   nnnnnnnnnn nnnnnnnnnn nnnnnnnnnn nnnnnnnnnn nnnnnnnnnn nnnnnnnnnn 6420
   nnnnnnnnnn nnnnnnnnnn nnnnnnnnnn nnnnnnnnnn nnnnnnnnnn nnnnnnnnnn 6480
   nnnnnnnnnn nnnnnnnnnn nnnnnnnnnn nnnnnnnnnn nnnnnnnnnn nnnnnnnnnn 6540
   nnnnnnnnnn nnnnnnnnnn nnnnnnnnnn nnnnnnnnnn nnnnnnnnnn nnnnnnnnnn 6600
   nnnnnnnnnn nnnnnnnnnn nnnnnnnnnn nnnnnnnnnn nnnnnnnnnn nnnnnnnnnn 6660
   nnnnnnnnnn nnnnnnnnnn nnnnnnnnnn nnnnnnnnnn nnnnnnnnnn nnnnnnnnnn 6720
   nnnnnnnnnn nnnnnnnnnn nnnnnnnnnn nnnnnnnnnn nnnnnnnnnn nnnnnnnnnn 6780
   nnnnnnnnnn nnnnnnnnnn nnnnnnnnnn nnnnnnnnnn nnnnnnnnnn nnnnnnnnnn 6840
   nnnnnnnnnn nnnnnnnnnn nnnnnnnnnn nnnnnnnnnn nnnnnnnnnn nnnnnnnnnn 6900
   nnnnnnnnnn nnnnnnnnnn nnnnnnnnnn nnnnnnnnnn nnnnnnnnnn nnnnnnnnnn 6960
   nnnnnnnnnn nnnnnnnnnn nnnnnnnnnn nnnnnnnnnn nnnnnnnnnn nnnnnnnnnn 7020
   nnnnnnnnnn nnnnnnnnnn nnnnnnnnnn nnnnnnnnnn nnnnnagggg gaagcgcacg 7080
   cgtggttggg agggggaagc gcacgcgtgg cttggagggg gaagtgcacg cgtggttggg 7140
   agggggaagc gcacgcgtgg ttgggagggg gaagtccacg cgtggttggg agggggaagc 7200
   gcacgcgtgg ttgggagggg gaagcgcacg cgtggcttgg agggggaagt ccacgcgtgg 7260
   ttgggagggg gaagcgcacg cgtggcttgg agggggaagt gcacgcgtgg tttggaggtg 7320
   ctgttggagg agagctgagg acggtctgtt ttacagcaat gaggcgagca tggtgggtgg 7380
   ggtcacagct gctctggggc cgggagggcg ctcagcgggt gctgagtttg agatggttgt 7440
   gttggaacaa tgtcagaggt gctgtgcgtg tgacgatgga ggagaggcaa gcccgatggt 7500
   catcgatccc acagtgaagg gaataaacta acatggcctc acggaggcga caaccagatt 7560
   ggctgtgtag ggtcgaaaaa ctccgtcccc cgacccgaca cccgttttct cagcccgcgt 7620
   aagggccgcg cctctgagca caaagaccag ctctctcggc tgaaggacag agaccccgag 7680
   ttctacaagt tcctgcagga gaatgaccgg agcctactgg acttcagtga ctcggacagc 7740
   tctgcggaag aagaagagcc attccactcc ctgccagaca cgctggaggt gaaacccggc 7800
   agacctgggc gtgggggcac ctgtggtttc tatacaccgt cccagccttg tccttctgta 7860
   tctggtccag gaagctagcg aaacagagga agacggagga gaggacagtg acgcgttgcc 7920
   cagagggctg aggagcaaga agaatgagcc tgtacccgtg accctcgcca tggtggaaag 7980
   gtggaggcag ggctccaggg taagaagcag tataagctgg gtggctgcat ctccacaggc 8040
   gggagactct ggcctcgcct cgtttgaggc gtctgagaca gcaggtgaaa agactgtgtt 8100
   tctgggaagc gcacaatgga ggtctttcca agtgccaggg tcctccagct acgtctcctt 8160
   atttgtagtg catagtcctg cctgcttcct cacactctct atcagggttc tgacccttgt 8220
   ggggtggttg tagagtttgt ttcatttctg ttaacaggat accacagacg gggcaatttg 8280
   ttttttgttg ttgacttact cacctgcata taggttggtg taccgtatgc aagttcatag 8340
   ttaaccgatg gttgtgagcc actgtgtggg tgctgggagt caaacctgga tcgtctggaa 8400
   gaacagtcag tactcttaac taccaagcca tctttccagc tccagactag ttaatttgga 8460
   aagaaattta actggctcac attgctggag gtaggcaggt caaaaagtac ggtgatgtct 8520
   ttgttaaagg ccttgtgttg tgatccttgt tcagttacat cttttgaatt gttagaaatc 8580
   aagcccagga cttgtacatt gtaggtaatc acactgtcct gagccctgag ccttttcgtt 8640
   tggtttagtt tggtttggtt tttgagacag ggtttctctg tgcagccctg gctgtcctgg 8700
   aactcactct gtagaccagg ctggcctcga actcagaaat ctgcctgcct ctgccttcca 8760
   agtgctggga ttaaaggcat gtgccaccac tgcccggatg tttcatgtga tcttcattac 8820
   tttccaaagg ctttatttcc aaataccacc aaataaactt ggatattaaa tttctactac 8880
   ataagccttt gggctatagt caggcttagc attatttctt cactggggag tgggaaatga 8940
   agtcagtgtc ctttgaataa tttcacactt aacttagagg ttttgggatt tttttttttt 9000
   tttttttaca gttggagatc actatgtaaa tcaccctggt ggctaacttt gaactctatg 9060
   tagagtagtt tggcctcaaa acttgctgta gtctccttgc ctttgctttc ccagtgctgg 9120
   cgtagccatc agctatcatg cttggctttc actttggttt ctttagggaa gggagtgaaa 9180
   agggagattc ccatacactt tctggagcaa gtttgtaatg cagtattctc tcccctttct 9240
   tctgggactc ttgacacagg ctctgcctgt ccaggtgtgc gcaataccta ttttgttgtt 9300
   ggctcatggt ccagtgatct tagccccaca gttgctctgg ccctgagatt gtgggttaga 9360
   ttagcacctt ttcaacttgg agcaggttcc tagagccagg tactgtgaac tactctttta 9420
   gtgatcttga tctcttgccc cttcagcacc accttagtcc caggctgttc catgaagttg 9480
   tacaggcgtt ccgagcagct gtagccacca cccaaggaga gcaggaagct gctgagactt 9540
   gcaggttcca ggttgcagat agtgctggtg agctggggac tggggacctg tgccaggctt 9600
   cagtgtgaca cctgtgtgcc ctatataggt gaatgacact ttggttcctt gcagtgttca 9660
   atgctctggt tactttctgc attcgagacc tctgtggttg ccttcagaag ctgctgtttg 9720
   gaaagacacc aaaggatagc aataggtaag gtatggtcag gggttttgtc attaaggtag 9780
   aggaggtcac aaagtcttaa ggaagcacag aaagcagcta ataaatacag atgagtcatg 9840
   gtggggctgg gagtagacat agccatgtgt cttgagatag cagatgcaga agagcaaccc 9900
   taaccctaac ctgaaggaga agatggtggc agttggttga tgcactgaca ggatgagtac 9960
   atctagaggc agcagagagt gaggagagca agaaaaccca acaggtggag agggcagaaa 10020
   ataagccagg tgatatgaca gaagcctaag tgaatggcta acacacccag aatctgggga 10080
   gcagttgtca tgaagggaca agtgagattt tagaaacagg gttgccctct gaattgcata 10140
   caatgcttga ggtgtcacac agatgagata gtgttggtct aggtatctgg aaggttagac 10200
   agtggggtgc cttaaatggg ctttggtttt tgttcttggt acagttcaat agatcttggt 10260
   aaacacctac tatgttgcca ggtgttcatg gttatacggc ttgtgctcgt gcaggcttgt 10320
   gtctgaattt cggaatgggg tgggtgtatt ccctgcaggc tgctgccatc cagtagccca 10380
   ctgtggggga agctccgtgt ggatgtcaag acatacctaa gtgcggtggt gcaggtacag 10440
   aatcccagga gacctggtca tgtctgatag tctggtgctc tcttacaccc cagattccag 10500
   tgactttctc ttcttcttta gctggtagcc tgtctagcgg aagccacggt gtctgcagct 10560
   gtcctgcagc atatcagcag cttggttcct tactacctga cattccctaa gcagtgccga 10620
   atgttgctca aggtacgtta gcttgtcctt gtcctactcc tggctagaat agaggcagca 10680
   tttgtgccta tacttgctct gctcctactg tgtcctctac tcagcaaggg tccccagtct 10740
   cagatggtcc cccgtccttt caggacagcg tggcttagta atgtgagact aggctccact 10800
   gaggcatgcg tgctagccat ggagggttct aggccaggaa aggcagcgat gtatcttttt 10860
   tcagaggatg gtggttctgt ggagcacggg tgaagaatct ctgcgggtcc tggccttcct 10920
   ggtactcatc agagtctgcc ggcacaagaa ggaagccttc cttggtccca ttctgaaggt 10980
   agcgtgagtc ttctatttgt tcattaaggt ctgtgttggg aattgtggtt gatggtcctt 11040
   acgaggactt gacaccaaac tctgatcata gttttcttac caagaaaatt gggtgatggt 11100
   gggaacagga acccatggtc ttgtgggtat tggtggtgat ggtgggaggg gctcttttcc 11160
   tctgggtacg agaacgttat acctagaaaa taggaacgtg tctgaggcca ccaccaagat 11220
   cagatcctgc tgtgtagctc caggcctgga gtggcatcac ctggactcct gctgtacaga 11280
   cctagctcga agtagagcgt ctctacttcc ttcctcggcc ttcggtccag tccctgccta 11340
   gtcctcatct tacccttctc ttgtacacct tcctctcttc tgcatatctt agattatttg 11400
   tggtgggtac aagtgtgtag gctgagaaac atcagagttc ttgagtgctg ggaaccattc 11460
   aggaattgag tctgcagctc tttcagacgg cagttctaat ggaaaaccct aggtgtgggc 11520
   aggtggcttg gaactgaaca gtcgttgctc aggtggctct taattttggt ttgtcagcaa 11580
   atgtacatca tgtatgtgag aaactgcaag ttcacctccc ccagtaccct ccccctcata 11640
   agcttcatgc aacggacact gactgaaatg cttgccttgg accccagcgt ctcctatcag 11700
   cacgccttcc tctacatccg acagcttgcc gtccacctgc ggaatgctat gaccgcaggc 11760
   aagaaggtag gaggaggtca gacccttcga agccagtagc cccacccggg ccttagggtg 11820
   gctagaagcc actctttaat gggggaggct gctcagcgct gagggaggct ttgggtggtt 11880
   ctggcaggga agaacgggcc taggatgtgg actcactctc cccaggagac acaccagtct 11940
   gtgtacaact ggcagtatgt gcactgcctc tacctgtggt gtcgagtcct gagtaccctt 12000
   ggttccagtg agatcctgca gccgctactc taccctctct cacagatcat cattggctgt 12060
   atcaagtgag ttcagggaca gggctatgtt agattagcct aggggttggg ggcagcaagt 12120
   tcctcttggt atagggcaga agttcctgat ttgctagctt ctgtgaatta cattatacaa 12180
   agaattttct cgggtggcca caccttaaat cttagctttt gtggctatag tctctgagga 12240
   agggcctgag aagatgaact ggatgtggat ttcaggcagc tgggcttcgc agtcagcctc 12300
   agcttaggta aataggttag gtgtccacag tgtagcacct atatggaccc ggctcgggcc 12360
   cttgtgctat tggagggcaa tcagatgtag ttctaagtgc ttttgatagt ctgcgaatgt 12420
   gctgtgccta cctctttggc taggaggggc ctctgtgtcc tctcgagctt tctcctgaca 12480
   ctggagactg agctaggaag gggaagaggc aggtggtaca agaaaacaag gcagcaggtg 12540
   gtgaggaaag gaggcaggag tgaatgaact aagctaaagt tggatgagcg cctttcagtc 12600
   tcgcagctga gcagtggggc accaaggcca agcattttgg ttgtccctgt tggttttgct 12660
   gactgtttaa attactattc cccatgggtg ctatgagtac ccaccctgta ggagtggcca 12720
   gggtctcctg gagttggagc ttgtgaacgg ggaggagtca gaggcagcgg gcagttaact 12780
   gatgtttggg cctggctacc tgactgggct ccgctcctct ttctccaggg ttgttgccca 12840
   ctgctcgatt ttatccattg cgcatgcatt gtgtacgtgc cctgacactg ctgtcccaga 12900
   ccatcggcac cttcatacct gtcctgccct tcattctcga ggtgactgtg ctgggcacaa 12960
   ctcgtgttgg acttagaaat gtctttagga aaccacacct ccgtctctgc gtctgccagt 13020
   gctaacttct aacaatgacc ccgtgacccc cctctctttg cagtccttac agatctgcat 13080
   tgagtctctc caagtctcac agttactatg acacctttct cgttttcctt attgtacctc 13140
   taccttgagt cgctgtccct gccctcgggc ttcctatcag gtgcagattc cttgagaaag 13200
   tgaagcacaa gggtgaatcc aagcccctgc taacaccgac accgcccttt ctttagtcta 13260
   tggcgaggat aaaggtggat attgataacc agctctatat attagttagc atcctttgct 13320
   cagagaaagg actttcccta cctccaattt attaatcact gtagctggtt caccttctag 13380
   caacatgtta cgtaagttct cttatcaggg cttccctata gaacctcccc tctctatgct 13440
   tctgtgtgta ccttgtgcct ccttggttta cctgttctgt gtggggcatc ctcggaccaa 13500
   gaggttaggc taggctccga tttccctctt aggaaggcca aagtgtatat agagcctgca 13560
   aggtttgttg ggtgtggaaa acacagtctc ccttctgtct ttctttattg gtggttttgt 13620
   ccatagtgcc tgggatgcgt gaggcccaga tccgtatcta aaggcggtta agtgtatgca 13680
   ggcgtcctta tggttatctg tcaaggggct cagggctctg gaaggcctgg tcttggggga 13740
   tctctttggc tcgggcttac actctgtggg tagattttcc agcaggtgga cttcaatagg 13800
   cggccaggtc gcatgagctc caagcccatc aacttctctg tgatcttgaa gctgtccagc 13860
   accaacctgc aggagaaggc gtaccgggtg aggctgaact caaggccttc tgggaaaggc 13920
   ctggggctat gttttagttg gtttacaagg acttaataag gccgttgata tacaagggag 13980
   ggattcccat tgtggaccat ttgtggtttc ttttgagaca agatctctct attatattgc 14040
   tcttgctgtc ctggaacttg ctatattgat caggctagcc ttgaactcac agagttccac 14100
   ttttcaaatg ctaagattga ggtttttgta gtcatggagc aatgactcct taatctcagt 14160
   tcccgtgaag ctgggtgtgg cttggggggg tctgtgcgta ctgttttata gccctggtct 14220
   tggagattga gcagagttgg ggtgaaatgt aagaaaatgg gctactgggc ttcatcatct 14280
   gatgaggttg ggcctgcccc ccaggacggg ctgctggaac agctgtgtga ccttactctg 14340
   gaatacctgc acagccaggc ccacagcatc gctttcccag agttggtgtt gcctactgtt 14400
   ctacaggtat gtactcatca tctgacttcc tttccctggc cagaccttgg cgtcctcctt 14460
   gtggctatct tggacaatct tttagtaggt ttgtcgtccc cttatgttct agtcctggac 14520
   cagtagcaac accgtgttta tccagttacc aatgggctgc cagatccatt ggcgccagtg 14580
   cagggagaga gcgatcattt gccttgatag cttaaggaac tttctctagg gactgtggaa 14640
   ttaacttgta aaggattatc ggcctctgct gaagttgggc tggggctggg taacttaatc 14700
   aggatctcag gaatttgcag acccttcccc agtctgactg gagcctacca tcaactatgg 14760
   aagtctaaag cctgggttgg tctggtgccc gttttgtttt tccgtgtggt catggtctct 14820
   ctgtgcccct tctgagaatc tgtttgctct cagctcagct attttccatc taggtccttc 14880
   tcataggagg gggctggtgc tgtctctttt ctggtactgg taggggatta gatccattgc 14940
   tcacagactt gtcgtggtgt tgctcatacc tgagctatgc ctgggtcagg ctcctatggg 15000
   cacaggtgta tgtgtgacgt tacatgtata gctgtgcaca cggctgtgtc gcggttggtt 15060
   gcataatgtg gcattggctt ccccctgtgt cctcttggct gatgatgtca ggtagtcagg 15120
   atacctttag ttttaggctc agcattttag gtgtatggat gtgtggatct agagtagaag 15180
   ttagcagatg tgtttcagtt aaaataaaag tagcaaaaaa aaaaaaagaa acttttaagg 15240
   tttatgtctt gtcacagctc ttcatctgct tacctctcgg gctgcgggga caagcatggt 15300
   cttatgcctg tgagacctaa gagattgaca gcaggtcctt gtgattgtca catggctttt 15360
   cactgcttgg acccctcgag ggtgaggaat ggaactgtca ctggagctgt actgagtctt 15420
   tcctttctgc agctgaaatc ttttctccgg gagtgcaaag tggctaacta ctgccggcag 15480
   gtgcgccagc tgctggagaa agtgcaagag aatgcacgac acatccaaag tcttcgacag 15540
   agcgcgacct tcagcgtgtc tgaccggacg gcagtggtgg gttggacttg gcatctgcac 15600
   tggaatggtg ctgcctgggg tcacctgtta gggtgtggtg gatgtttaag ggttttccag 15660
   aatgatcatg gtcctgaatt tacttcaatc cttaggatgc gtgggagaag caggttcgtg 15720
   aagaggggac cccactcacc agatactacg gccactggaa gaagctgagg gaccgtgaga 15780
   tccagctgga aatcagtggc aaagagcggg tatggctggg gtctgtgaag atggcctagt 15840
   agaaagtggc caagcaggtt atttgtaaag gggttggggc tgagggggag aagttggaaa 15900
   aggaaacgaa atcaggaggg aagaaagaaa ggtgagtttg agtgaaccct gtaagtttct 15960
   gtttacctgc tgggcagctg gtccccggta ttgatataga tggttcccga ggcgcagctg 16020
   ggccagttct ctcggcctct ccctaccctc acttgttgtt ctgtttcttg gtccttgaag 16080
   ctagaagacc tgaacttccc agagatcaaa aggcggaagg tggaagacag gaaggatgaa 16140
   gacaggaaag aattaaagga cctgtttgag ttggacagtt ctgagggcga ggacagcacc 16200
   gacttctttg agagaggtga ggcctgagct ggagtggttg agagctgtgt gtgtctgggg 16260
   cattcatgtc tggctagtgg gcagacctgg tttgttgttt ttaattttta tgtctgtgtg 16320
   ttttggctac atgtctgtct gtatccagat cccctgagac tggagttaga gacacttgtg 16380
   agcctcagtg tgggcactgg gatcaaaccc atgtccttga accactgagc catctctcta 16440
   gcctccagat ctgttttttt tttttttttt taaagattta tttatttatt atatgtaagt 16500
   acactgtagc tgtcttcaga cactccagaa gagggcgtca gatcttgtta cagatggttg 16560
   tgacccacca tgtggttgct gggatttgaa ctcaggacct ttggaagagc agtcgggtgc 16620
   tcttacccac tgagccatct caccagccct agatctgttt cttataccac tcattggccc 16680
   cttatgtttg agctggggac tcagtattgg taattgtcac catgctgggg cctctatagg 16740
   tggcagagag ggctagtggc cctgttgcta ctgctgagac ctggggccct gttgcatgtc 16800
   cagagccagc cctcctgtgt agctactcat tgctttgtta tttcacccgg tgtgtgtgtg 16860
   tgtctgtgtg tgtgtctgtg tgtgtctgtg tgtgtgtgtg acgaccgttc ttcatttgca 16920
   ggagtaccta ggctcccgga agctcaccaa ggactgaaag aagatcagga agaagaagat 16980
   aaagaagaag gtgacagcga ttcagagggt aagtggtttc tatatgaaat agattggccc 17040
   tggagtctgt ctggaggcca gaccatggga agacagagct ctacgccagg cttctgcccc 17100
   tccctggggt gccaggccag ctgctctgta ctctttagca accagctata gcatgctgat 17160
   aaatttaatt aatgctgcaa ttaattagtg atgagtaact tctaaagctg gctttttcat 17220
   gatatagcag aatttattta tgtggcctct tccttctctg tagatggaga cacagacacg 17280
   ggagtggatc tgagcgaact gtggcagctg gctcagggac cacaagatga gctggaggat 17340
   cttcagctct cagaagagga ctgagggcct cctgctgcct cctggaggcc tcgacagcaa 17400
   ctgtgcggcg agaggacagc agagcgtaaa caggctttta ttgttacagc acagcagaac 17460
   aagtgtacta ggagccattt tgaccctgaa agggagggaa ctgtttctgc agagccttct 17520
   gctgccggaa gccgaaggct gaccctgatg ttgtcccagt tcagagtggg gagggcggct 17580
   ccatggcctc tcctctccac tttgtctttg gtttgtctgc agagcactgc agttacctct 17640
   agaagataaa cccattactt ttgtttgttt gtttgtttgt ttgtttgttt gtttgttttt 17700
   tcgagacagg gtttctctgt gtagccctgg ctgtcctgga actcactctg tagaccaggc 17760
   tggcctcgaa ctcagaaatc cgcctgcctc tgcctcccga gtgctgggat taaaggtgtg 17820
   cgccacctct gcccagctca ttgcttttct tcttaagcct tatgttttcc ttttaaaaat 17880
   actctcggca tttttgcatc taaaaagggg aaataccctg ggggcggagt gaggttagca 17940
   atttgactgg gaatcacgga gtatgacggt tgcctgccgg atcattggag aagctgggaa 18000
   gggtctgggg cacctgaaag cagagctatg gtccctgacc cattctcctg cttgggtgag 18060
   gcttgtcctg tgggcaggtg ggtcccttca tacggggagg tggtggttgc tggcgacaga 18120
   ggttgatgtc ctgggctgag ctcaggtgct tgcaggcttg gtggctgcag cggcagggcc 18180
   acagggaagc tggccatgta gaagacacgg cccaggcgct tggccaccta caaagagacg 18240
   aagctgggtg aatacctgct aggtaaaggc gcctagggaa acaactccag gtacagagaa 18300
   tcctaaaacc ctctctagag tggtgaaaac actggacaaa cttacttgtg ccctgatctt 18360
   gagggcaggc cctagcttca gtcccatggt gttcagaagg tgctcttctg tcagtagagg 18420
   caaagtctct ccgtctattc cttgttctct gaatacctgg ggacaaggtg ggcccataga 18480
   gtagagttgt agagcttgca tggaaaaata acaaaaggaa agttcttttt tgtgggagac 18540
   cgggacattg tccagaactg ggccagaacc ctagggacct gccactcccc tcaccctggc 18600
   atactcgcca cagccagaaa ggccccccac gaagttgcag acatcatcta cagtccactt 18660
   gttgacatcc tcaagggttg tggtctcctc atcagtgaag agccccccca tggtacctgg 18720
   agatagaaag gatgtcacta gggcttgggc tttcccattt ctttgcctcc ttccccactg 18780
   gctgaccttt ctctgaattg gtaaagtctt tggcttaatg acaaatgatt tttctcccca 18840
   cgggatcccc tatcagtggg tgactaaggc tgcccacctg tgtggaagta ggggctcact 18900
   gccccgcaag ggaatcccag gggcagtgga gggggcagtg tggaccctga tagaagcccc 18960
   cttccttctg ctctggtccc tcctgctggg acttggctgg gagtggaggt cccttccctg 19020
   gcagctgctg tctcggggtc ctctccatct gagtccttgg atggctcctc agagacttct 19080
   tgagcccaga ggccagcccc tgtcgtctcc ttgggttggc tgggctggac accaaggctc 19140
   cccttttcgg accggcttcg agcagactcc ttgggtggga tagggggtcc tgggcctggg 19200
   ggtccctggg gtggcagggc cagtagtggt gctgagctgt gtttcaaaac cagcatggag 19260
   ccacgtcgct gcagttcgtc gggaccctcg gggatatgca aggccacctc tggtgccggt 19320
   agcagtggcc ggtgaacact gcccagttct ttctgcctaa gcaactctga catttccagc 19380
   ctggaccaaa aggacagacg agtcagcctg ggcacgctta gaactgctcc cgctggcctt 19440
   gcagtcgctt accgagccag gctctgcttc cgtagaagtt cctgctgccg agctaacatc 19500
   tcagcctggg cggagggtag gaagctgaaa cctgggatag aagaaaaggc tgctgaagcc 19560
   ggatgctctt cactactttt atctatctgc ctaaagagag cctcatagga acacaaacgg 19620
   cttacatacc cgactttctc acctggggtc tgacacacag ctgtgggcat ccccagaaaa 19680
   ggaggcggga gatgggagct cattgtgatg tgggcggcat tctggggtga cagcaaaggg 19740
   ggtggctgtg acatttccct gtggacagca gaatagtgag cacatgccta ggtccgtctg 19800
   tgcacacccc ctctttcctg tcatcacctc tgggtgcacc cctgctaacc cttctagttg 19860
   ccgacccctc aagtggccgt tggtttcctg attatggctt gcttgtgtac acacacacac 19920
   acacacacac accccgcgcg caagtctcct tgtcagcaca gaggcaatgg ccccatgggc 19980
   cacactgcgt cagtaattac cggggtgggg gatacctctc atcctgagca gtgaggggag 20040
   gggtgcagga cagggcaatt agccccaatt tgtagatgtg gcagatgctg cagtctgcca 20100
   ggatcagggt gagataggat gtgtttggga aacccgggga ccctgcctaa tgctctcctt 20160
   ggcttaactc gtctgccgcg gagactactg tgagcatgat gtctctggag tttatagcag 20220
   ggtagggatg gagggggcgt atgtccttca ctctgaatcc tctgtggttg ctttctcctt 20280
   gccttcctca acatcccacc accaccacct gtgaggggag ggcctctgct ctcggggcag 20340
   ctctactagg ctggaatagt cagagttgct tgacaagaaa attgtatttg gcattcttgc 20400
   cagggagtgg gcatttctgc atattttgcc ccccaaattg agtctttgca gtagttttag 20460
   gagcttcctg cagtggacct tgaacgtggt gaatgcatta tgcatatatg tgatggaaga 20520
   ctcaagggaa agaaagaatt ggccttctca agtaacaagc atagcatgtg gggccttgct 20580
   gaagcacgtc ttaatacctt tcagagaaag atgtggcagc agctggaacc gccccttccc 20640
   ggcgctgaac taggccttgc ttccgtcgat ggtctgctgt agacgagggc aggtgaactt 20700
   ccaagccact gggacccctc agtgttgcag ttgctacttc ctggcgtacc ctaagcagat 20760
   ctgagaaggg aggtgatagc gggcccccct gataagctcc cgtaggattt cccctttcca 20820
   tcttggtgag cagggcattg gcccggtgaa ggtatgaaca gagtgggagg aggagactct 20880
   cacatgtcct gagcctgctt ttgtgtcaca ggctagagga tcagcaggga aggcactttg 20940
   ctttaacacc tacatgctgt agccaggcaa gtgaacttga aaaggaatgt gaataagtag 21000
   gtcatcgaaa ctggagaaga aaggctccaa catgtagagt attagtgcta atttggtttg 21060
   aatattcaca aggtcagagg tggaagggca tagggctgct ccaagtaggt gagtgggaca 21120
   agagtctatg gattaggagg ggacctcaaa cagaagcctg cccaggtaac tcctttctag 21180
   acaggagaca agaacaggaa ggacttgccg tccctataaa gcgattcata gcatacatgg 21240
   ttataatagc agataccact gactagcagc aacacacaat cacagacctg catggttgtg 21300
   cccccattgc atttaggaag agcggaggct ccgagcccct ttaatccacc tgctcaagct 21360
   ccacttctct accgctctgc ctgtttataa atcacgtagc tgaaaaaggg acgtgggcct 21420
   agctagggct tgaagccaaa tcccagaccc taataagccc aggccacccc agtcatgcgc 21480
   cacgcaatgg tgattgacag ccagcttgct tgcacagtct cctgcacagg ccagcagagc 21540
   tgggtaagca attaaaaaga aagattttta aattattaac atttagtgaa ttattcaggc 21600
   tctcggtgcc tgagctgctg ccctagggaa cagcgccttc tgcaagaatc aggaatcctt 21660
   ttcccttctc tgagcccccg tccacttctg ccagaaaaga caagatttgt ccggtgctgt 21720
   agcagcacag ggtgtctcac aagtcctcgg tagcaaggac ggtgcctcaa ggcttggctt 21780
   ccctcaagca acctggggtg catcctgact gtgacgcagg ttcctatttt tgccatgccc 21840
   tcctccctgt gtatcaacaa aacctgaaaa gtctgacaca gcatcaacac gaccacagat 21900
   tcacgtattg cactgaccac tctgggagaa actagcacag gccagaggcc acatctcttc 21960
   aacaaagaac aggtgaacaa actcttctgt cccaccctca cctgggctcc tcggggacgc 22020
   acagtagagg actgaggcca gtgaggctgc atcactaccc ggaagccctt ctatgtattc 22080
   aatcctggtc ttggtgttga cctccaagca tccagatctt tttcttgggg ttggcttagt 22140
   ggggtggaaa ggtaagagag atttggctca cttgaagtct gagcgccggc cgtgggggtg 22200
   gcacataccg tggctgggga tacccaagtg gtagagacgt tgagtttcct caaaggaact 22260
   ggcctcactc agggccgaca taagctgatg gtagtggtcc tcaggggcca ttgtagactc 22320
   tagcttggga agcagcgaag tcgctgttgg acagagcaca tggtgaggac cacggggaaa 22380
   gtcaaggctt cttatggttt ccacccaagg tctggggagc ccttcaggac ccagttgctc 22440
   ccccccttcc ctgtgacctg catccttttc cagagtcggg ctttcacctt gggggtcctt 22500
   gcttctggcc ttcttctctg aagagcagtg actggtgatg caggggcagc tcagcctctt 22560
   gcccaacagg tcgcctgtag aggatagagt gagccccacc cagtgtctca tggcttcgag 22620
   agaaagccat ctctgcctta gctcagtctg gagccccaat caccactatg ttctccctgg 22680
   cagttaaaga ccttgtccga tctggtgtcc caggctagag agcacgatga caggtagatg 22740
   gagagtgcag taaagacaaa gcgatcctga cgctggcctt gggcacgtaa gtaacagtga 22800
   gagctctgca tcagtgtttc cccagtcagt ctcctgtaag ctccttgcct ctcccctcgg 22860
   tgcatttgcc catctgcata tccaggtctg ggttcttaat gggactccta agcatttcac 22920
   tccatagtgc acagtctata tgtgctatgc ttttatgagg ccaggccaga cactgcttac 22980
   aatatcccca gagataatac atgccgaatc tcaggcatta attgacccaa ttccagactg 23040
   gcctgaacac tgctgcctaa cttcctcctg agctcctgcc ctgaggctag ggcctttgct 23100
   gaacagccca tgacatcgcc atcatggctt caagctaagg ttcctgtctc tgccctcctt 23160
   atcctcacca gctaggggag tgtggaggaa caattccgtg ggtgattcaa aatctccata 23220
   atgcccaata aggggctgct tctacttgtg tgcccaccct ccctcaaagc tggtggtgtc 23280
   agcgccagga ctgaattcca tttaaaaaaa attccttaat ttgtcatttt tatcagattg 23340
   gggaagtgtg gcagcaaaaa cgctgatcaa tctccacgtt atggctgaca gaatggtgct 23400
   aataacttat tgatctcaac tacctcactg ctgttagtct agcaggccat ctcagcctcc 23460
   gccccagaga aagagacagc gcctactgtc tggacaaggg ctccacccat aatatctcaa 23520
   ggcagatggg tccagcagac agataatggg tatctggctt cacttggtgc caccaatagt 23580
   ttatcagaga ccagtaggca gaagaggcag cagctggagt agacaggaag gagggtccct 23640
   caggtctgtg tggcctggtc tcactcactt tgtacctgtt attcttgtcc caacccacag 23700
   atccccttta aggactaggc aagggctctt ttcacagcag tctattctca ctttaaaagt 23760
   ggctaccccc aaaggagagg gggagggaga gagggagaaa aggagg 23806
<210> 7
   <211> 163
   <212> PRT
   <213> Homo sapiens
<400> 7
   Met His His His His His His Gly Met Ala Ser Glu Phe Gly Ser Ala
   1 5 10 15
   Gly Ser Arg Lys Arg Arg Leu Ala Glu Leu Thr Val Asp Glu Phe Leu
   20 25 30
   Ala Ser Gly Phe Asp Ser Glu Ser Glu Ser Glu Ser Glu Asn Ser Pro
   35 40 45
   Gln Ala Glu Thr Arg Glu Ala Arg Glu Ala Ala Arg Ser Pro Asp Lys
   50 55 60
   Pro Gly Gly Ser Pro Ser Ala Ser Arg Arg Lys Gly Arg Ala Ser Glu
   65 70 75 80
   His Lys Asp Gln Leu Ser Arg Leu Lys Asp Arg Asp Pro Glu Phe Tyr
   85 90 95
   Lys Phe Leu Gln Glu Asn Asp Gln Ser Leu Leu Asn Phe Ser Asp Ser
   100 105 110
   Asp Ser Ser Glu Glu Glu Glu Gly Pro Phe His Ser Leu Pro Asp Val
   115 120 125
   Leu Glu Glu Ala Ser Glu Glu Glu Asp Gly Ala Glu Glu Gly Glu Asp
   130 135 140
   Gly Asp Arg Val Pro Arg Gly Leu Lys Gly Lys Lys Asn Ser Val Pro
   145 150 155 160
   Ser Thr Ile
<210> 8
   <211> 176
   <212> PRT
   <213> Homo sapiens
<400> 8
   Met Arg Gly Ser His His His His His His Gly Met Ala Ser Met Thr
    1               5                  10                  15
   Gly Gly Gln Gln Met Gly Arg Asp Leu Tyr Asp Asp Asp Asp Lys Asp
               20                  25                  30
   Arg Trp Gly Ser Glu Glu Gly Thr Pro Leu Thr Leu Tyr Tyr Ser His
           35                  40                  45
   Trp Arg Lys Leu Arg Asp Arg Glu Ile Gln Leu Glu Ile Ser Gly Lys
       50                  55                  60
   Glu Arg Leu Glu Asp Leu Asn Phe Pro Glu Ile Lys Arg Arg Lys Met
   65                  70                  75                  80
   Ala Asp Arg Lys Asp Glu Asp Arg Lys Gln Phe Lys Asp Leu Phe Asp
                   85                  90                  95
   Leu Asn Ser Ser Glu Glu Asp Asp Thr Glu Gly Phe Ser Glu Arg Gly
               100                 105                 110
   Ile Leu Arg Pro Leu Ser Thr Arg His Gly Val Glu Asp Asp Glu Glu
           115                 120                 125
   Asp Glu Glu Glu Gly Glu Glu Asp Ser Ser Asn Ser Glu Asp Gly Asp
       130                 135                 140
   Pro Asp Ala Glu Ala Gly Leu Ala Pro Gly Glu Leu Gln Gln Leu Ala
   145                 150                 155                 160
   Gln Gly Pro Glu Asp Glu Leu Glu Asp Leu Gln Leu Ser Glu Asp Asp
                   165                 170                 175

## Beschreibung

Gegenstand der vorliegenden Erfindung ist die Verwendung eines Antikörpers, der spezifisch an ein Epitop eines Polypeptids mit der Aminosäuresequenz SEQ ID NO:3 bindet, zum Nachweis eines Korepressors in Skelettmuskelzellen und Präadipocyten.

Die für den Korepressor gewählte Bezeichnung GRIM1 stellt die Abkürzung der Englisch-sprachigen Bezeichnung "Global Repressor Involved in Myogenic Differentiation" dar. Dieser Name wurde gewählt, da der klonierte Faktor eine wichtige Rolle insbesondere während der Skelettmuskeldifferenzierung spielt.

Innerhalb der letzten Jahre wurde eine Vielzahl an Molekülen aufgefunden, die für die unmittelbare transkriptionelle Regulation verantwortlich zeichnen. Neben den DNA-bindenden Faktoren (im engeren Sinne als Transkriptionsfaktoren bezeichnet) gibt es regulatorische Moleküle, die als Koaktivatoren Genexpression erleichtern oder die als Ko-Repressoren aktiv eine transkriptionelle Ruhigstellung bewirken. Kofaktoren sind promiskuitiv, können in verschiedenen Kombinationen von verschiedenen DNA-bindenden Partnern rekrutiert werden. Der Wechsel zwischen assoziierten Koaktivator- und Ko-Repressorkomplexen ist ein wichtiger regulativer Schritt innerhalb zellulärer Differenzierungsprozesse.

Am Beispiel der Skelettmuskeldifferenzierung hat man im Laufe der Jahre eine Kaskade von Transkriptionsfaktoren ermittelt, die für die Myogenese notwendig sind. Neben anderen Transkriptionsfaktoren bewirken die bHLH-Proteine MyoD, myf5, MRF4 und Myogenin, daß in einer proliferierenden Myoblasten-Vorläuferzelle das genetische Programm ausgeführt wird, das eine terminal differenzierte funktionale Skelettmuskelzelle hervorbringt. Im Laufe dieser Phylogenese durchläuft die Zelle einen Zellzyklus-Arrest, sie fusioniert mit anderen determinierten Muskelzellen zu multinukleären Myoblasten und exprimiert skelettmuskelspezifische Struktur- und Stoffwechselenzyme.

Die assoziierten Kofaktoren, die innerhalb dieses Prozesses eingreifen, wurden erst in den letzten Jahren erforscht, Positiv regulierte Expression myogen spezifischer Gene erfolgt zum Beispiel durch eine funktionale Assoziation von MyoD mit dem Ko-Aktivatorprotein p300. Acetyliertes MyoD agiert transkriptionell weitaus aktiver als nicht-modifiziertes Protein.

Auf der anderen Seite gibt es bestimmte Ko-Repressoren, die eine gezielte Repression der Expression muskelspezifischer Gene in proliferierenden Zellen aktiv verhindern. So wurde zum Beispiel eine funktionelle Assoziation zwischen MyoD und dem Ko-Repressor N-CoR beschrieben und brachte so die bisher nur nukleären Hormonrezeptoren zugeordneten Kofaktoren in ein neues Betätigungsfeld. Desweiteren wurde beschrieben, daß Mitglieder der Histon-Deacetylase (HDAC) Familie innerhalb der Skelettmuskel-Differenzierung (C2C12-Zellkulturmodell) beteiligt sind und eine subzelluläre Relokalisation innerhalb der ersten Differenzierungsschritte zeigen.

Die Verbindung von HDACs mit aktiver Repression gilt als einer der Grundpfeiler der negativen transkriptionellen Regulation, da sich die von den HDACs katalysierte Reaktion direkt auf das Chromatin überträgt und die beobachteten Effekte mechanistisch erklärt.

WO 01/57190 A2 beschreibt eine Vielzahl von Nukleinsäuren und Polypeptiden, die von diesen Nukleinsäuren codiert werden. Diese Nukleinsäuren wurden in einem Screeningverfahren identifiziert.

Die in dieser Anmeldung gezeigten Daten beschreiben GRIM1 als einen neuen "bona fide" transkriptionellen Repressor mit einem neuen Repressionsmechanismus. GRIM1 ist nicht mit einer HDAC-Aktivität assoziiert und die GRIM1-vermittelte Repression lässt sich nicht durch spezifische HDAC-Inhibitoren beeinflussen. GRIM1 kann über saure Domänen im N- und C-Terminus die Acetylierung von Histon-N-Termini (eine der Voraussetzungen für gerichtete transkriptionelle Aktivierung) direkt inhibieren. Bisher noch nicht eingehender charakterisierte Repressionsdomänen innerhalb von GRIM1 haben ebenfalls ein hohes Potential, Transkriptionsaktivität zu reprimieren. GRIM1 hat in transienten Transfektionen das Potential, sowohl komplexe als auch synthetische Minimalpromotoren dosis-abhängig zu reprimieren. Basierend auf diesen Daten wurde der neu klonierte Faktor als GRIM1 (Global Repressor Involved in Myogenic differentiation) bezeichnet.

GRIM1 zeigt eine Relokalisation während der Skelettmuskeldifferenzierung, allerdings zu einem deutlich späteren Zeitpunkt als es bisher für die HDACs 4, 5 und 7 im Zusammenhang mit MEF2 spezifischer Transkription beschrieben wurde. Die vorliegenden Daten legen nahe, daß das Repressionspotential von GRIM1 erst zu späteren Zeitpunkten der Skelettmüskeldifferenzierung abgeschaltet werden muss, damit die Myotuben terminal differenzieren können. Desweitern zeigt GRIM1 während der Präadipocytendifferenzierung ebenfalls eine subzelluläre Lokalisationsänderung.

Eine Funktion von GRIM1 ist, daß es als Repressor die Differenzierung von Skelettmuskelzellen mitreguliert. Die Daten zeigen, daß GRIM1 eine deutlich andere Rolle als bisher beschriebene beteiligte Faktoren zeigt. Die Aufklärung der *in vivo* Rolle von GRIM1 erfolgt über GRIM1 knock-out Tiere und transgene Mäuse. In den transgenen Tieren werden GRIM1-Mutanten unter Skelettmuskel-spezifischen Promotoren exprimieren, die nicht mehr in den Nukleus gelangen oder die nicht mehr aus dem Nukleus heraustransportiert werden können. Der Phänotyp der Mäuse, der durch die erzeugte atypische Lokalisation von GRIM1 hervorgerufen wird, wird weiter zum Verständnis der GRIM1 Funktion innerhalb von Differenzierungsprozessen beitragen.

Die Anwendung der vorliegenden Erfindung liegt vor allem im Bereich von degenerativen Muskel-Erkrankungen oder in der Kontrolle des Muskelschwundes beim alternden Mann. Die therapeutische Modifikation der GRIM1 Funktion kann dabei einem vorzeitlichen Muskelabbau entgegenwirken öder aktiv in muskelaufbauende Prozesse eingreifen.

In der vorliegenden Anmeldung beschrieben sind daher Polypeptide, die eine Sequenz von wenigstens 20 aufeinanderfolgenden Aminosäuren aus der Sequenz des hsGRIM1 (hs = homo sapiens) mit der Sequenz ID Nr. 3 aufweisen.

Bevorzugt weisen die beschriebenen Polypeptide eine Sequenz von wenigstens 40 aufeinanderfolgenden Aminosäuren auf.

Bevorzugter weisen die Polypeptide eine Sequenz von wenigstens 80 aufeinanderfolgenden Aminosäuren auf, noch bevorzugter eine Sequenz von wenigstens 120 aufeinander folgenden Aminosäuren und am bevorzugtesten weisen die beschriebenen Polypeptide eine Sequenz von wenigstens 200 aufeinanderfolgenden Aminosäuren aus Seq.ID Nr. 3 auf.

Des Weiteren werden in der vorliegenden Anmeldung Polypeptide beschrieben, die eine Sequenz von wenigstens 20 aufeinanderfolgenden Aminosäuren aus der mmGRIM-Sequenz (mm = mus musculus) mit der Seq.ID Nr. 4 aufweisen.

Bevorzugter weisen dieser Polypeptide eine Sequenz von wenigstens 40 aufeinanderfolgenden Aminosäuren, noch bevorzugter eine Sequenz von wenigstens 120 aufeinanderfolgenden Aminosäuren und ganz besonders bevorzugt eine Sequenz von wenigstens 200 aufeinanderfolgenden Aminosäuren der Seq.ID Nr. 4 auf.

Ebenfalls werden in der vorliegenden Anmeldung Antikörper beschrieben, die spezifisch an ein Epitop eines erfindungsgemäßen Polypeptids binden. Die beschriebenen Antikörper können nach üblichen Standardmethoden hergestellt werden. Entweder können die für GRIM1 kodierenden Polypeptide zur Immunisierung von geeigneten Labortieren, wie beispielsweise Kaninchen oder Ziegen dienen und es können auf diese Art und Weise geeignete polyklonale Antikörper hergestellt werden. Die Antikörper können gegen Epitope, wie Konformationsepitope, aber auch gegen beschriebene Peptide gerichtet sein.

Alternativ hierzu können geeignete monoklonale Antikörper nach Methoden hergestellt werden, die seit der Publikation von Köhler und Milstein im Jahr 1975 wohlbekannt sind und zum Standardrepertoire eines durchschnittlichen Molekularbiologen gehören.

Derartige Antikörper können Einsatz in der Therapie finden. Hierzu ist es aber in der Regel erforderlich, daß humanisierte Antikörper hergestellt werden, weil Antikörper mit von Tieren stammenden Bestandteilen unerwünschte Nebenwirkungen hervorrufen können. Wenn die bindenden Regionen eines geeigneten Antikörpers sequenziert wurden, können diese Sequenzen in ein menschliches Antikörpergrundgerüst eingebaut werden und ein derartiger Antikörper kann therapeutisch verwendet werden.

Ein alternatives Anwendungsgebiet von derartigen Antikörpern stellt die Diagnostik dar. Hierfür können durchaus einfache polyklonale Antikörper ausreichen, mit denen qualitativ undloder quantitativ die Anwesenheit von GRIM1 Polypeptiden in den interessierenden Zellen bzw. Zellkompartimenten nachgewiesen werden kann. Mit derartigen Antikörpern kann der Durchschnittsfachmann die geeigneten diagnostischen Methoden durchführen.

Arzneimittel, die ein hierin beschriebenes Polypeptid enthalten, können zur Behandlung von Störungen der Skelettmuskulaturdifferenzierung eingesetzt werden, sowie zur Behandlung von Störungen der Fettzellendifferenzierung.

Die Arzneimittel können ein Polypeptid mit der vollständigen GRIM1-Sequenz oder einen geeigneten Teil der Sequenz beinhalten. Die Arzneimittel werden in geeigneter Form den zu behandelnden Patienten verabreicht. Geeignet sind hierbei orale oder bevorzugt parenterale pharmazeutische Formulierungen

Der Wirkmechanismus der Arzneimittel greift auf eine Modulation der GRIM1-Funktion zurück. Während der Skelettmuskeldifferenzierung wird GRIM1 aus dem Nucleus in das Zytoplasma transportiert. Die Arzneimittel können in diesen Differenzierungsprozeß dadurch eingreifen, daß entweder der Import gezielt blockiert oder aber der Export blockiert wird. Dies kann dadurch erreicht werden, daß die geeigneten Abschnitte der Polypeptide in entsprechende Formulierungen eingearbeitet werden, damit der interessierende Zielort erreicht werden kann.

Im Rahmen der vorliegenden Erfindung wurde auch herausgefunden, daß GRIM1 an weiteren Differenzierungsprozessen beteiligt ist. GRIM1 zeigt eine subnucleäre Relokalisation während der Adipozytendifferenzierung. Eine Beteiligung von GRIM an verschiedenen anderen Regulationsprozessen ergibt sich aus den Erkenntnissen der vorliegenden Erfindung, insbesondere aus den experimentellen Daten, die zeigen, daß GRIM1 eine Repression verschiedenster Komplexe und synthetischer Promotoren zeigt und eine ubiquitäre Expression von GRIM1 vorliegt.

Ein weiterer Gegenstand der vorliegenden Erfindung ist ein Verfahren zur Identifizierung von Stoffen, die die biologische Funktion eines hierin beschriebenen Polypeptids beeinflussen. Hierbei wird der zu identifizierende Stoff mit dem Polypeptid in einem Testsystem in Kontakt gebracht. Über dieses Testsystem können solche Stoffe identifiziert werden, die mit dem GRIM1 Polypeptid in Wechselwirkung treten und die biologische Funktion von GRIM1 hemmen oder verstärken.

Bei den erfindungsgemäß verwendeten Systemen handelt es sich bevorzugt um zelluläre Differenzierungssysteme, die sich insbesondere myogener C2C12-Zellen oder schnell und induzierbar differenzierender 1OT_{½} MyoD-ER Zellen bedienen. Der Nachweis von GRIM1 bzw. der GRIM1-Lokalisation kann beispielsweise immunhistologisch erfolgen. Geeignete Methoden sind unter anderem die Verwendung von Fluoreszenz-gekoppelten oder Enzymgekoppelten Antikörpern in einem direkten Färbungsschritt.

Es können auch geeignete Zellinien bereitgestellt werden, die ein entsprechendes genetisches Konstrukt mit einem GRIM1-Gen beinhalten. Die zu untersuchenden Stoffe können dann mit den Zellen in Kontakt gebracht werden und über entsprechende Vergleichsversuche kann die Wirkung der zu untersuchenden Substanz bestimmt werden.

In einer weiteren Ausführungsform können geeignete genetische Konstrukte in Labortiere eingeführt werden und die transgenen Tiere können für die Testverfahren verwendet werden.

In einer weiteren Ausführungsform der Erfindung können rekombinant hergestellte GRIM1-Fragmente in zellfreien Testsystemen mit zu testenden Substanzen inkubiert werden und durch Einsatz eines geeigneten Reportermoleküls kann die gewünschte Testaussageerhalten werden.

cDNA, die für hsGRIM1 (homo sapiens) kodiert, die eine Sequenz von wenigstens 1200 aufeinanderfolgenden Nucleotiden aus der Seq.ID Nr. 1 aufweist, ist ebenfalls hierin beschrieben.

Beschrieben ist weiterhin die cDNA, die für mmGRIM (mus musculus) kodiert, die eine Sequenz von wenigstens 1200 aufeinanderfolgenden Nucleotiden aus der Seq.ID Nr. 2 aufweist.

Die beschriebenen cDNAs können eingesetzt werden in Transfektionsvektoren, die eine dieser cDNAs aufweisen. Hierbei kann es sich bevorzugt um einen adenoviralen Vektor handeln.

Verwendet werden diese Transfektiönsvektoren zur Herstellung von Zellen, die mit einem solchen Vektor transfiziert wurden.

Bei den nachfolgend näher beschriebenen Beispielen wurden neben üblichen Standardmethoden bevorzugt folgende Methoden verwendet, die unterstrichenen Methodenbereiche sind im Folgenden nähergehend erläutert:

### Methode 1 (Transiente und stabile Transfektion von Zellkulturzellen):

Die Zellen wurden in den von ATCC empfohlenen Medien auf 15cm-Zelllculturschalen unter sterilen Bedingungen kultiviert. Zellen werden alle zwei bis drei Tage in einem Verhältnis von 1:5 bis 1:20 subkultiviert und auf maximal 60% Konfluenz gehalten. Altes Medium wird entfernt, die Zellen werden mit PBS-Puffer gewaschen und mit Typsin/EDTA-Lösung (0,25% Trypsin, 0,04% EDTA in PBS-Puffer) bis zur Ablösung von der Kulturschale behandelt. Die Zellen werden in frischem Medium vereinzelt und auf neuen Zellkulturschalen subkultiviert.
- PBS-Puffer:: 137 mM NaCl; 2,7 mM KCI; 8 mM Na₂HPO₄; 1,8 mM KH₂PO₄
- Verwendete Medien:: DMEM (Gibco BRL), 100 IU Penicillin G/ml, 100µg/ml Streptomycin, 2 mM Glutamin, 10% fötales Kälberserum. Als Differenzierungsmedium bei C2C12- und L6-Zellen wurde 10 % FCS durch 2 % Pferdeserum ersetzt. 1oT1/2 MyoD-ER Zellen wurden in hormonfreiem Serum kultiviert, als Differenzierungsmedium wurden 1 µm Estradiol, 10 µg/ml Insulin und 10 µg/ml Transferrin supplementiert.

Zur Bestimmung der transkriptionellen Eigenschaften von hsGRIM1-Proteinen in eukaryontischen Zellen wurden pCMX-hsGRIM1-Expressionsplasmide mit Luciferase-(LUC)-Reporterplasmiden kotransfiziert. In einem pCMX-Expressionsplasmid steht die Expression des jeweiligen hsGRIM1-Proteins unter der Kontrolle des Cytomegalievirus-Promotors. In den verwendeten LUC-Reporterplasmiden wird die Expression der Luciferase entweder durch den Thymidinkinase-(TK)-Promotor, einen E1b-Minimalpromotor (TATA-Box), oder durch die respektiv angegebenen komplexen Promotoren gesteuert.

Die Transfektion erfolgt mit der Calciumphosphat-Methode oder mit der DOTAP-Liposomen Methode (Roche Diagnostics, Produktinformation). In 12-well-Schalen werden wenn nicht anders angegeben 10⁵ Zellen/well in 1 ml Kulturmedium ausgesät. Bei der CaPO₄-Method wird die zu transfizierende Plasmid-DNA mit Calciumphosphat kopräzipitiert und das Präzipitat auf den zur Teilung befähigten Zellen verteilt. Zur Herstellung des Calciumphosphat-Präzipitats wird der unten angegebene Ansatz für Doppelwerte in 12-Loch-Schalen hergestellt:

| | |
|---|---|
| Reporterplasmid | 1 µg |
| hsGRIM1-Expressionsplasmid | 20-400 ng |
| Träger-DNA (pUC18) | ad 8 µg |
| CaCl₂-Lösung (2,5 M) | 20 µl |
| ddH₂O | ad 80 µl |

Unter Schütteln (Vortexen) werden zu diesem Ansatz langsam und gleichmäßig 80µl 2xBES-Puffer zugetropft. Das Präzipitat wird 10min bei RT inkubiert und gleichmäßig auf den Zellen verteilt (80µl pro well) und nach 8 Stunden durch Waschen mit PBS entfernt. Die Zellen werden in frischem Kulturmedium für weitere 18 bis 24h inkubiert.
- BES-Puffer (2x):: 50mM N,N-bis-[2hydroxyethyl]-2-aminoethansulfonsäure; 280 mM NaCl; 1,5 mM Na₂HPO₄2H₂O; pH 6,95.

Stabile Transfektion von Zellkulturzellen wird mit dem pcDNA6-His System der Firma Invitrogen durchgeführt. Zellen werden wie oben beschrieben transfiziert und stabile Integration der verwendeten Plasmide wird durch das Selektionsantibiotikum Blasticidin S detektiert. Einzelne Klone werden gepickt, expandiert und über Western Blotting und Immunodetektion auf stabile Expression hin getestet.

### Methode 2 (Protein-Protein-Interaktionen):

Hefe Zwei-Hybrid Interaktionsassays, mammalian THS, Pull-down-Analysen, Immunpräzipitation von zellulären Proteinen gefolgt von Immunodetektion oder in Kombination mit *in vitro* translatierten ³⁵S-Methionin markierten Interaktionspartnern, Phast-Gel System.

### Glutathion-S-Transferase-Pulldown

Ein Protein wird in *E*. *coli* als GST-Fusionsprotein exprimiert, an einem GST-bindenden Trägermaterial immobilisiert und mit dem in der Regel radioaktiv markierten, potentiellen Interaktionspartner inkubiert. In den nachfolgenden Waschschritten werden nicht oder nur schwach interagierende Proteine vom Trägermaterial bzw. vom Trägermaterial-gebundenen GST-Fusionsprotein gewaschen. Die Menge an assoziiertem Interaktionspartner kann durch eine Auftrennung der Proteine durch SDS-PAGE und eine anschließende Autoradiographie bestimmt werden. Zur Kontrolle der Spezifität der Wechselwirkung zwischen poteritiellei-n Interaktionspartner und GST-Fusionsprotein wird die Interaktion mit GST allein überprüft. Aliquots von GST-GRIM1-Fragmenten oder GST enthaltendem *E, coli*-Rohlysat werden mit jeweils 30µl Glutathion-(GSH-)Sepharose versetzt und 1h bei 4°C auf einem Bohemian Wheel inkubiert. Die GSH-Sepharose wird durch Zentrifugation (1' bei 2000rpm) pelletiert und zweimal in Pulldown-Puffer gewaschen. Das Pellet wird anschließend in 500µl Pulldown-Puffer resuspendiert und mit dem *in vitro* translatierten, ³⁵S-Methionin-markierten potentiellen Interaktionspartner 1h bei 4°C auf dem Bohemian Wheel inkubiert. Nach dreimaligem Waschen mit Pulldown-Puffer wird das GSH-Sepharose-Pellet in 30µl SDS-Probenpuffer aufgekocht, die Proteine durch SDS-PAGE aufgetrennt und das Gel nach dem Trocknen einer Autoradiographie unterzogen.
- Pulldown-Puffer:: 20 mM HEPES (pH 7,7); 150 mM KCl; 0,1 mM EDTA; 25 mM MgCl₂; 10 mM DTT; 0,15% (v/v) NP40.

### Immunpräzipitation

Interagieren zwei Proteine in Lösung, so läßt sich der stabile Komplex beider Proteine mit einem Antikörper, der nur gegen einen der beiden Interaktionspartner gerichtet ist, präzipitieren. Die Isolierung des Protein-Antikörper-Komplexes aus der Lösung erfolgt dabei mit Hilfe eines Antikörper-bindenden Trägermaterials (γ-bind G Sepharose, Pharmacia). Zur Untersuchung von Wechselwirkungen zwischen GRIM1 und putativen Interaktionspartnern werden entweder native Zellextrakte oder GRIM1 beinhaltende Zellextrakte mit *in vitro* translatierten, ³⁵S-Methionin-markierten Target-Proteinen in 500µl IP-Puffer gemischt. Je 5µg anti-GRIM1 oder nicht-spezifischer Antikörper (Kaninchen IgG₁) werden zusammen mit 30µl prääquilibrierter γ-bind G Sepharose dazugegeben und der ganze 60 min bei 4°C auf einem Bohemian Wheel inkubiert. Anschließend wird die G-Sepharose 1' bei 2000rpm pelletiert, dreimal mit jeweils 300µl eiskaltem IP-Puffer gewaschen und abschließend das Pellet in 30µl SDS-Probenpuffer aufgekocht. Die Proben wurden abschließend durch SDS-PAGE separiert und Interaktion entweder über Western-Blotting oder im Falle der in Gegenwart von ³⁵S-Methionin *in vitro* translatierten Proteine, über Autoradiographie detektiert.
- IP-Puffer:: 20 mM TrisHCl (pH 8.0), 300mM NaCl, 5mM EDTA, 0,3% (v/v) NP40, 0.5mM Pefablock
- Zell-Lyse-Puffer:: 50mM TrisHCI (pH 8.0), 170mM NaCl, 0.1% NP40, 50mM NaF, 2mM NaO₃V₄, 0.2mM DTT, 0.1 mM Pefablock, 1µg/ml Aprotinin, 10% Glycerol

### Methode 3 (Nachweis intrazellulärer Lokalisation):

Indirekte Immunfluoreszenz, Zellfraktionierung gefolgt von Immunodetektion.

### Indirekte Immunfluoreszenz (IIF)

Zellen werden in der gewünschten Zelldichte auf autoklavierte Objektträger ausgesät und mit 5% Paraformaldehyd für 10' auf dem Träger fixiert. Zellmembrane werden durch 10' Inkubation in 0.2% TritonX100 für Antikörper durchlässig gemacht. Vor der Inkubation mit den Primärantikörpern werden die Zellen 1h bei RT in 0.2% Gelatine (in PBS) geblockt. Anti-hsGRIM1 Antikörper werden in 0.2%iger Gelatinelösung 1:500 verdünnt und 1h bei RT inkubiert. Nach dreimaligem Waschen mit PBS für je 5' wird der jeweilige Fluorochromgekoppelte Sekundärantikörper 1:2000 in 0.2% Gelatinelösung zugegeben und 30' bei RT im Dunkeln inkubiert. Nach den Waschschritten wird der Nukleus über DAPI (1µg/ml PBS, Roche Diagnostics) gegengefärbt und die Zellen werden final 2 mal in 0.1% TritonX100 gewaschen, um Autofluoreszenzen zu minimieren. Die Objektträger werden in Fluoromount M (Southern Biotechnology Associates) konserviert und mit einem Fluoreszenzmikroskop analysiert.

### Zellfraktionierung

Über hypotone Lyse der Zellmembran können intakte Nuklei isoliert und separat lysiert werden. Zellen werden in eiskaltem PBS geerntet, pelletiert und in hypotonem Lysispuffer (LB) zum Quellen gebracht. Durch Zugabe von NP40 ad 0.1% wird die Zellmembran permeiert, durch sanftes Schütteln und Inkubation auf Eis für 15' werden die cytoplasmatischen Bestandteile freigesetzt. Intakte Nuklei werden durch Zentrifugation bei 14.000 rpm für 15' pelletiert, die CP-Fraktion wird quantitativ abgenommen und die Proteinkonzentration bestimmt. Kerne werden durch 15' Inkubation unter Vortexen in Kern-Lysis-Puffer (KLB) lysiert. Debris wird pelletiert und die lösliche Kernpräparation wird abgenommen. Die Qualität der Preparation wird durch Western Blotting und Immunodetektion mit kernspezifischen (z.B. TIF2) und cytoplasmatischen (z.B. RasGAP) Markern überprüft.
- LB:: 10mM HEPES (pH 7.9), 1.5mM MgCl₂, 10mM KCI, 0.5mM DTT, 0.5mM Pefablock
- KLB:: 20mM HEPES (pH 7.9), 420mM NaCl, 1.5mM MgCl₂, 0.2mM EDTA, 0.5mM DTT, 0.5mM Pefablock, 10% Glycerol

### Methode 4 (Nachweis enzymatischer Aktivitäten):

### HDAC-Assay, INHAT-Assay, Luciferase-Assays.

### HDAC Assay

Die durchgeführten Histon-Deacetylierungsreaktionen wurden im Rahmen eines von Upstate Biotechnology erhaltenen Kits (³H markierter Histon H3 N-Terminus als Substrat) durchgeführt. Als alternative Substrate wurden mit p300 ¹⁴C-Acetyl-markierte Histone oder *in vivo* markierte Histone (freundlicherweise zur Verfügung gestellt von Dr. Martin Göttlicher, Kernforschungszentrum Karlsruhe) verwendet.

### INHAT-Assays

INHAT assays wurden durchgeführt wie unter Eckner *et al.* und unter Seo *et al.* (Eckner et al., 1996, Genes and Development 10 (19), pp 2478-2490, Seo et al., 2001, Cell 104 (1), pp 119-130) beschrieben. Histone oder Histon-Mischungen werden mit den entsprechenden Peptiden in insgesamt 50µl HAT-Puffer präinkubiert, dann mit bakteriell exprimierter p300-HAT-Domäne und 0.5µCi ¹⁴C-Acetyl-Coenzym-A versetzt und 2h bei 30°C und 1000rpm inkubiert. Reaktionen werden durch Zugabe von SDS-Laemmlipuffer gestoppt, Proteine werden in denaturierender SDS-PAGE aufgetrennt und die Gele 1h in Amplify (NEN) inkubiert. Acetylierungen werden in Autoradiographien nachgewiesen.

### Luciferase-Assays

Transient transfizierte Zellen werden einmal mit PBS gewaschen und durch Zugabe von 50µl Reporter-Lysepuffer (Promega) lysiert und durch Einfrieren bei -80°C weiter aufgeschlossen. Zell-Lysate werden in Eppendorf-Reaktionsgefäße überführt und Zelltrümmer 2' bei 14000 rpm pelletiert. Jeweils 10 µl der Lysate werden zur Bestimmung der LuciferaseAktivität in 96-Loch-Mikrotiterplatten pipettiert. Der Luciferaseassay erfolgte in einem EG&G Berthold Microluminomat. Zu den 10 µl Zell-Lysat werden 40 µl Luciferase-Assaypuffer injiziert und die resultierende Chemolumineszenz in einem Zeitintegral von 10" bestimmt. Die gemessenen Werte der Luciferase-Aktivät werden auf die im Zell-Lysat vorhandene Proteinmenge abgeglichen. Dazu werden jeweils 3µl der Lysate in 96-Loch-Mikrotiterplatten mit 100µl 1:5 mit H₂O verdünnten Bradford-Assay-Reagenz (BioRad) versetzt und die Absorption der Lösung bei 595nm bestimmt. Relative Luciferaseaktivitäten werden aus dem Verhältnis der Luciferaseaktivität zur Proteinabsorption ermittelt.
- Luciferase-Assaypuffer:: 200mM Tricine; 1,07mM (MgCO₃)-4Mg(OH)₂; 0,1M MgSO₄ 10mM EDTA (pH 8,0); 33mM DTT; 0,5M ATP; 270mM Acetyl-Coenzym A; 470mM Glühwürmchen-Luciferin.

Die Erfindung wird durch die nachfolgende Beispiele näher erläutert:

### Beispiel 1

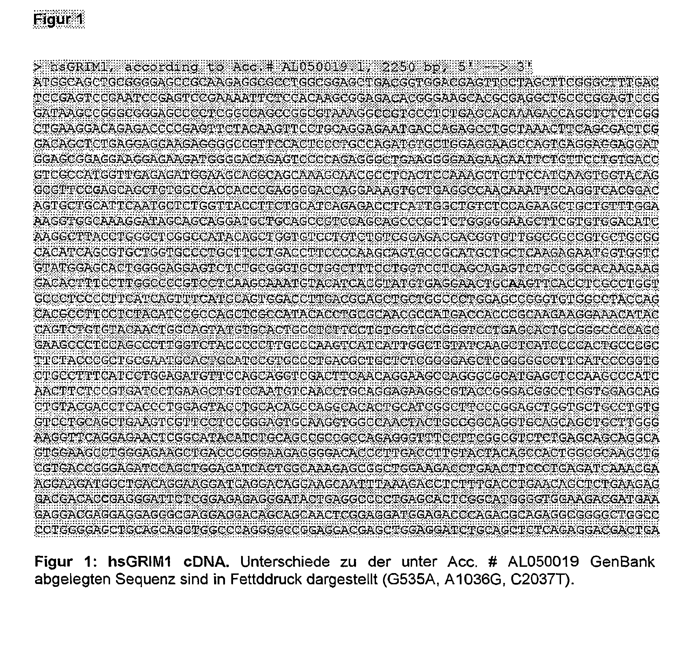

Das klonierte humane GRIM1 Protein umfasst 2250 Basenpaare und codiert für 749 Aminosäuren. Die klonierte und durch Sequenzierung gewonnene humane GRIM1 Sequenz ist zuerst 1999 unter der Accession Nummer AL050019 (Protein DKFZp564C186) im Rahmen eines "Random EST sequencing"-Projektes des DKFZs in Heidelberg als unbekannte cDNA niedergelegt worden. Eine weiter überarbeitete Sequenz wurde im März 2001 niedergelegt (Wiemann et al., 2001). Eine Funktionsbeschreibung für das Protein DKFZp564c186 ist bisher noch nicht erfolgt. Die erfindungsgemäße cDNA ist in der Figur 1 dargestellt. Die Sequenz hat die Seq. ID Nr. 1.

### Beispiel 2

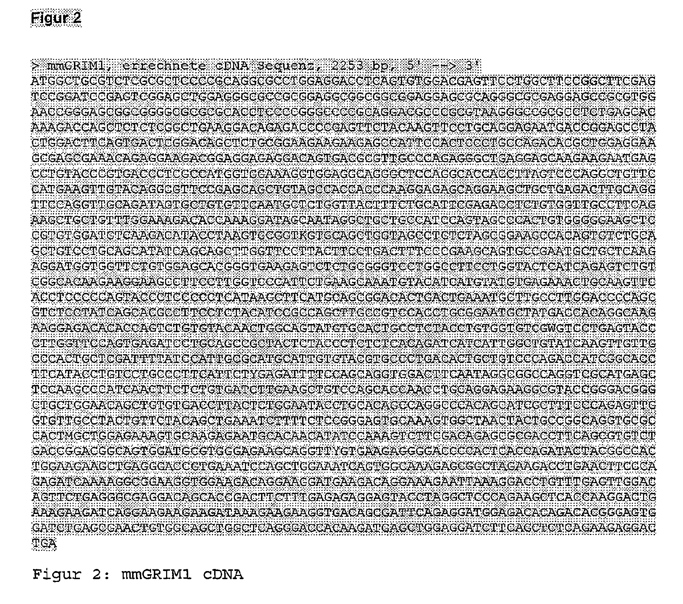

Aus Maus-EST Datenbanken konnte durch vergleichende Analyse mit der hsGRIM1 aa-Sequenz aus unabhängigen ESTs ein Gesamtlänge-Protein mit insgesamt 750 Aminosäuren berechnet werden, welches insgesamt 60% identische und zu über 85% homologe Reste zur bekannten hsGRIM1 Sequenz aufweist. Analyse von Maus cDNA- und Maus genomischen Banken (Celera) haben die berechnete und erwartete mmGRIM1 aa-Sequenz bestätigt. Die fiktive cDNA konnte über RT-PCR Analysen bestätigt werden. Die Gesamtlänge cDNA von mmGRIM1 ist noch nicht beschrieben und niedergelegt. Die Sequenz mit der Seq. ID Nr. 2 ist in Figur 2 dargestellt.

Die Seq.ID Nr. 2 wurde erhalten durch Screening von mmEST-dbs mit der hsGRIM1 aa-Sequenz. Die erhaltenen Daten wurden mit mm-cDNA-dbs und Maus genomischen-dbs verglichen und die virtuelle mmGRIM1 cDNA wurde erstellt. Die Sequenz wurde über RT-PCR und genomische Sequenzierung sowie über einen direkten Sequenzvergleich mit der Maus genomischen Datenbank von Celera Inc. bestätigt.

### Beispiel 3

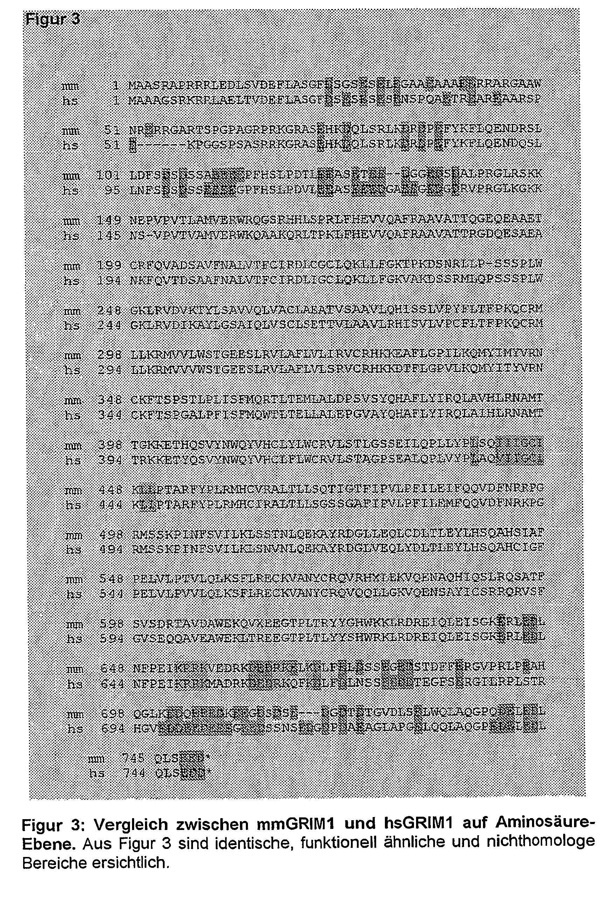

Eine Homologie-Darstellung zwischen hs und mmGRIM1 auf cDNA und auf Protein-Ebene ist in der Figur 3 gezeigt. In Figur 3 ist eine vergleichende Homologiedarstellung der mmGRIM1 und hsGRIM1 Aminosäuresequenzen inklusive konservierter Sequenzmotive dargestellt.

Die Aminosäuresequenz von mmGRIM1 (Maus) ist Seq. ID Nr. 4 und die Aminosäuresequenz von hsGRIM1 (Mensch) ist Sequenz ID Nr. 3.

Alle im weiteren detailliert beschriebenen funktionellen und potentiell interessanten Sequenzotive sind innerhalb des Maus und der Human-Proteins sequenzidentisch konserviert (nukleäres Lokalisationssignal NLS, nukleäres Exportsignal NES, sogenannte "CoRNR-box" mit der Ko-Repressoren mit Ihren assoziierten Transkriptionsfaktoren interagieren können). Weitere Motive sind HMG-box ähnliche Domänen innerhalb des C-und des N-Terminus von GRIM1 beider Species, die eventuell Kontakt mit DNA und/oder Histonen bewerkstelligen können. Desweiteren sind innerhalb der primären hsGRIM1 aa Sequenz insgesamt 5 sogenannte LXXLL oder verwandte Motive enthalten, eine alphahelicale Struktur, die beschrieben wurde, solitär für den Kontakt von Ko-Faktoren mit Ihren assoziierten Transkriptionsfaktoren verantwortlich zu sein. Innerhalb der mm GRIM1 Primärsequenz sind nur 2 dieser Motive gegenüber der humanen Sequenz konserviert. Funktionelle Daten zu diesen Motiven fehlen bisher. Berechnungen zufolge ist hsGRIM1 hauptsächlich alpha-helicaler Struktur (PHD-Predict Programm der Columbia University) und nicht-globulär (GLOBE des PredictProtein-Servers der Columbia University). GRIM1 ist in proliferierenden Zellen nukleär lokalisiert, innerhalb von zellulären Differenzierungssystemen (z.B. bei C2C12, L6, 10T1/2 und 3T3-L1 Zellen) wird GRIM1 aus dem Nukleus in das Cytoplasma re-lokalisiert.

### Beispiel 4

Im Rahmen der vorliegenden Erfindung wurde auf Basis von cDNA nur mit hsGRIM1 gearbeitet. Es wird aber davon ausgegangen, daß bei Einsatz von Maus-cDNA entsprechende Ergebnisse erhalten werden. Von dem humanen GRIM1 wurden zur eingehenden funktionalen Charakterisierung über Deletions- und sequenzspezifische Punktmutagenese verschiedene Konstrukte hergestellt, die in den nachfolgenden Tabellen zusammengestellt wurden.

**Tabelle 1: Verwendete hsGRIM1 Punktmutanten**

| **Name :** | **aa Positionen** | **Verwendete Konstrukte** |
|---|---|---|
| ΔNES I | L309K. L312A | -, FLAG, His, Xpress. GFP |
| ΔNESII | L309R, L312A, L315Q, | -, FLAG, His. Xpress, GFP |
| | L317A | |
| ΔNLS I | K649A. R650L, R651E, | -, FLAG, His, Xpress, GFP |
| | K652A | |
| ΔNLS II | K649A. R650L, R651E 1E, | -, FLAG, His, Xpress, GFP |
| | K652A, R661D. K6621, | |
| | K665E | |
| ΔCoRNR | I439A, 1440S. I443A | -, FLAG |

**Tabelle 2: Verwendete hsGRIM1 Deletionsmutanten**

| **Name** | **aa-Positionen** | **Verwendete Konstrukte** |
|---|---|---|
| GR1M1 | 1-749 | jedes |
| mut A | 3-609 | Gal4-DBD, FLAG, GFP, GST, His |
| mut B | 3-487 | Gal4-DBD. GST, His |
| mut C | 3-423 | Gal4-DBD, GST, His |
| mut D | 3-245 | Gal4-DBD, GST, His |
| mut E | 3-147 | G-l4-DBD, GST, His |
| mut F | 145-749 | Gal4-DBD, GST, His |
| mut G | 246-749 | Gal4-DBD, GST, His |
| mut H | 485-749 | Gal4-DBD, GST, His |
| mut I | 609-749 | Gal4-DBD, FLAG, GFP, GST. His |
| mut J | 145-609 | Gal4-DBD, His |
| mut M | 468-749 | GST |
| mut N | 246-609 | Gal4-DBD |
| mut O | 246-485 | Gal4-DBD |
| mut P | 485-609 | Gal4-DBD |
| mut Q | 246-423 | Gal4-DBD |
| mut R | 423-485 | Gal4-DBD, GST |
| mut S | 145-245 | Gal4-DBD |
| 246-334 | 246-334 | FLAG, GFP |
| Δ 246-334 | 3-245 335-749 | FLAG, GFP |

Viele der verwendeten Mutanten tragen spezifische Tags wie die Gal4-DNA-Bindungsdomäne des Hefe GAL4 Transkriptionsfaktors [Ga14-DBD], Grün-fluorezierendes Protein [GFP], *Herpes Simplex Virus* Transaktivatorprotein 16 [VP16], die antikörperspezifischen Signalsequenzen FLAG-tag [FLAG], Xpress-tag [XPRESS], c-myc-tag [MYC], Nickel-bindendes Epitop His-tag [His], Maltose-bindende Epitope [MBP], Glutathion bindende Anteile der Glutathion-S-Transferase [GST]). Die Verwendung von derart modifizierten Proteinen ist gekennzeichnet durch die Verwendung des Epitop-Names im benutzten GRIM1 Konstrukt. Durch diese Versuche wurde die Funktion der einzelnen Bereiche der erfindungsgemäßen Polypeptide erforscht.

### Beispiel 5

Datenbankanalysen haben gezeigt, daß hsGRIM1 homologe Proteine in einer Vielzahl von Spezies zu finden sind. In *Shizosaccharomyces pombe, Saccharaomyces cerevisiae, Rattus spec., Bos bovis, Mus musculus, Drosophila melanogaster, Caenorhabditis* elegans, *Danio rerio* sowie auch in *Arabidopsis thaliana* sind homologe Sequenzen zu finden. Eine zusammenfassende Übersicht wird aus den Datenbanken UNIGENE (National Center for Biotechnological Information, NCBI) und des PredictProtein-Servers der Columbia-University gewonnen und in der folgenden Tabelle 3 dargestellt:

**Tabelle 3: Darstellung der UNIGENE maximal homologen Proteine aus Maus, Ratte, Arabidopsis, Fadenwurm, Fruchtfliege und Bäckerhefe. Angegeben sind Swissprot Accession-Nummern, UNIGENE-Accession-Nummern, untersuchter Bereich und maximale Homologie (Maus Q9WV70 ist nicht der Gesamtiänge-Klon, vergleiche dazu den beschriebenen und unter Seq.ID Nr. 4 beschriebenen Maus-Klon).**

| | |
|---|---|
| AUSGEWÄHLTE MODELLORGANISMEN IN EINER PROTEINAHNLICHKEITSDARSTELLUNG Organismus, Protein, Prozent-Identität und Länge der verglichenen Region | |
| H.sapiens: | sp:Q9Y3T9 -YU20:HUMAN HYPOTHETICAL 84.9 KDA PROTEIN DKFZP564C186 100% über 753 Aminosäuren (erfindungsgemäß) |
| M.musculus: | sp:Q9WV70-YU20 MOUSE HYPOTHETICAL PROTEIN 73% über 267 As |
| R-norvegicus: | sp:- - [Segment 1. of.2] COLLAGEN ALPHA 1 (1) CHAIN 29 % über 328 AS |
| A.thaliana: | sp:Q9ZPV5-YU20 ARATH HYPOTHETICAL PROTEIN T30D6.27 IN CHROMOSOME II 27% über 722 AS |
| C.elegans: | sp:O17580 - YTB2_CAEEL HYPOTHETICAL 820 KDA PROTEIN C07E3.2 IN-CHROMOSOME-II 27 % über 656 AS |
| YU20 | D.melanogaster. sp:Q9VIF0 -DROME HYPOTHETICAL PRÖTEIN CG9246 37 % über 681i AS |
| S.cerevisiae: | sp:P39744 - YO26_YEAST HYPOTHETICAL 81, 6 KDA PROTEIN IN DED1-RETI INTERGENIC REGION 29% über 6.74 AS |

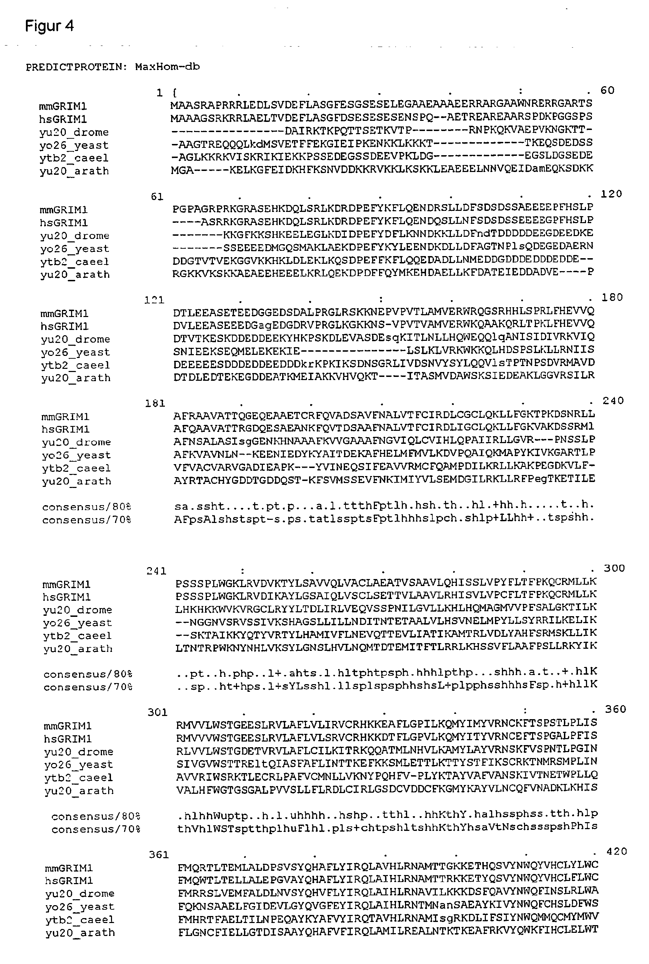

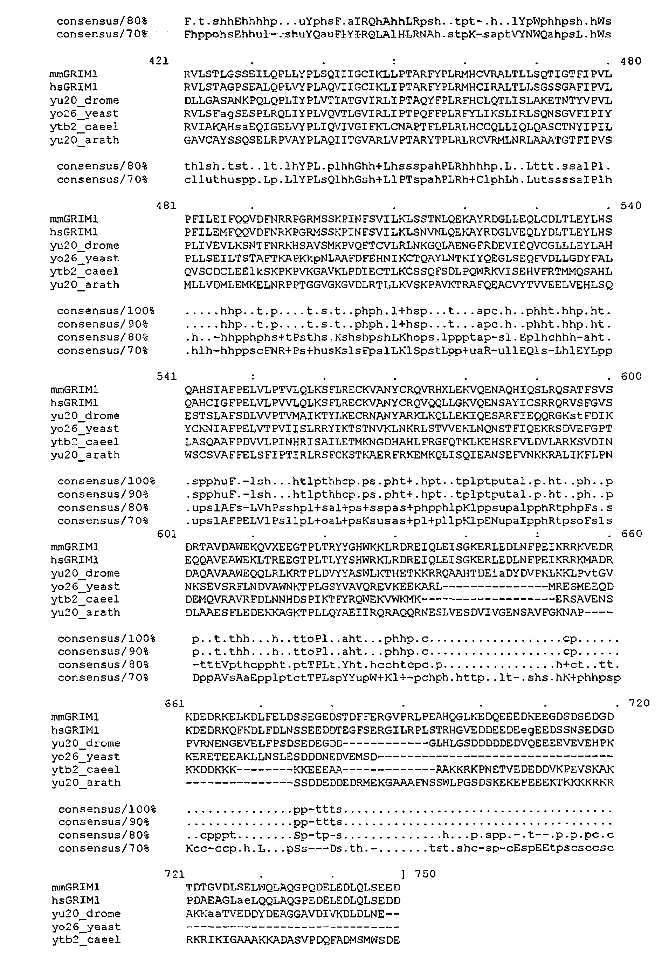

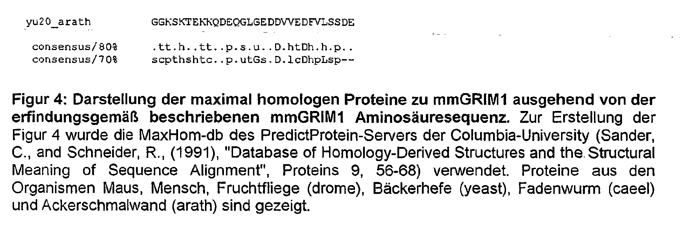

Ein Vergleich der Aminosäuren und die Ermittlung einer Konsenssequenz ist in Figur 4 dargestellt.

### Beispiel 6

### Genomische Organisation

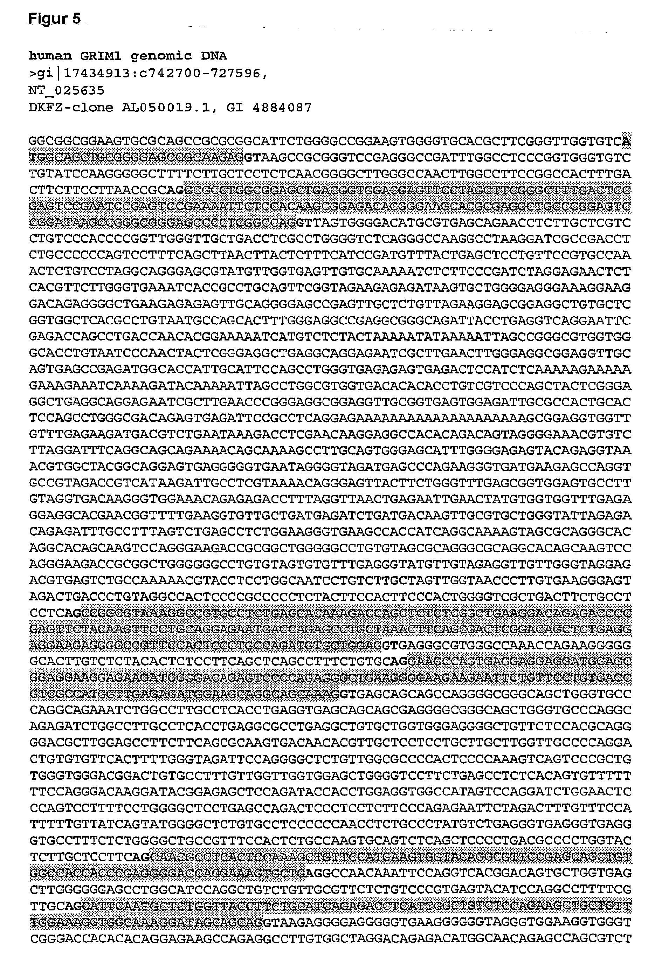

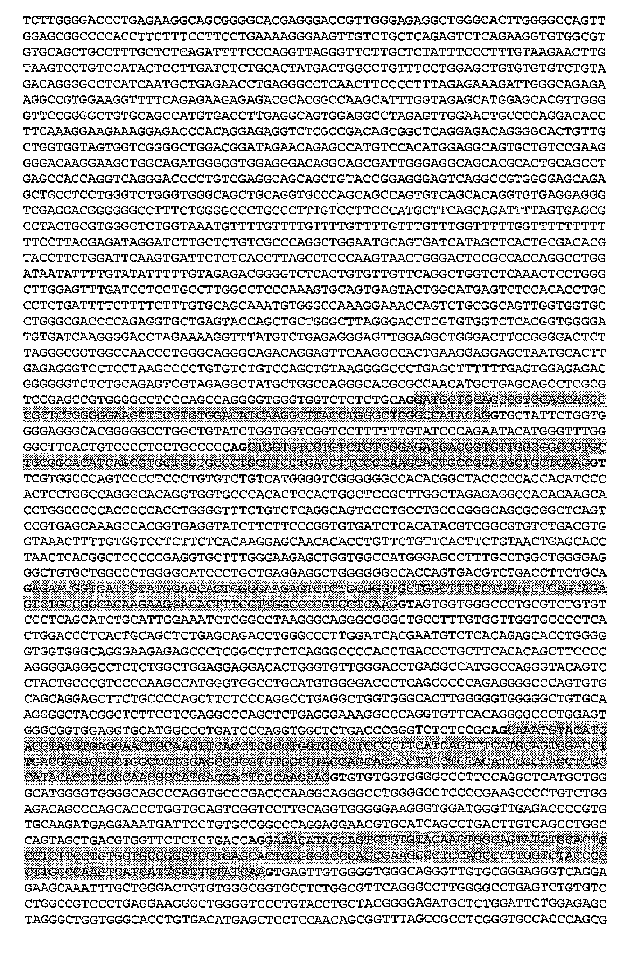

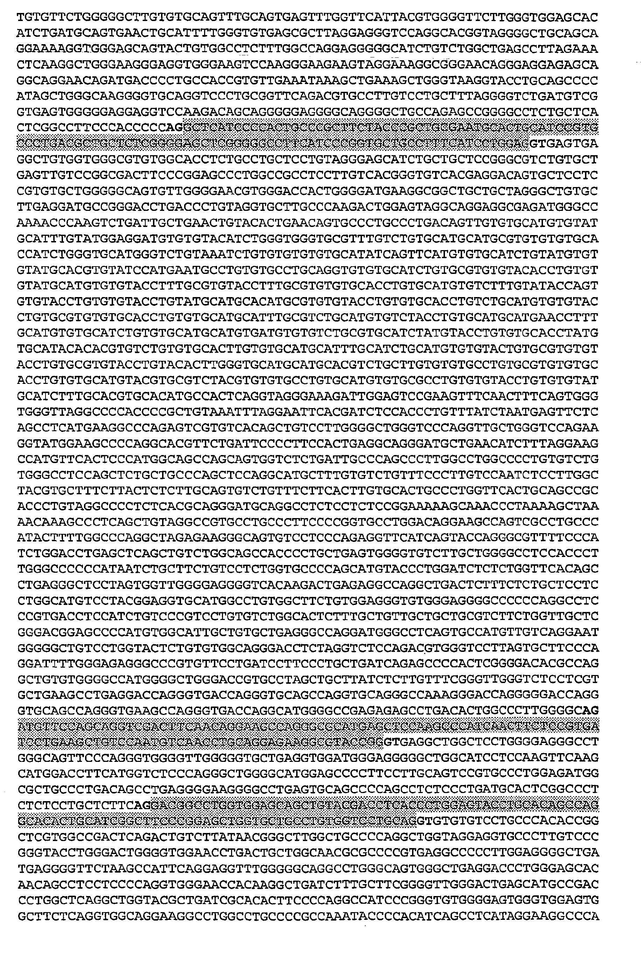

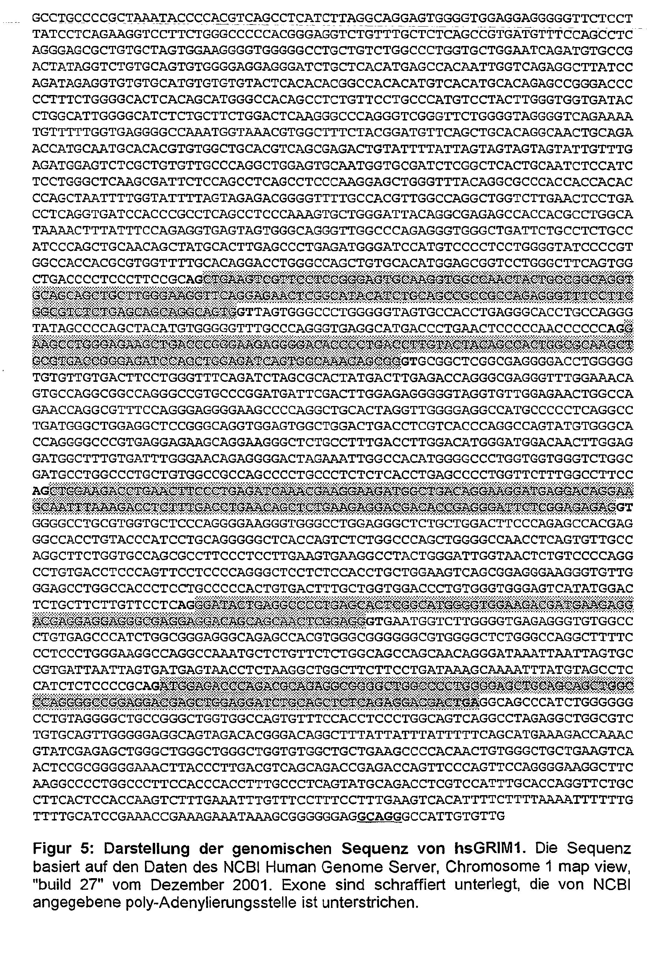

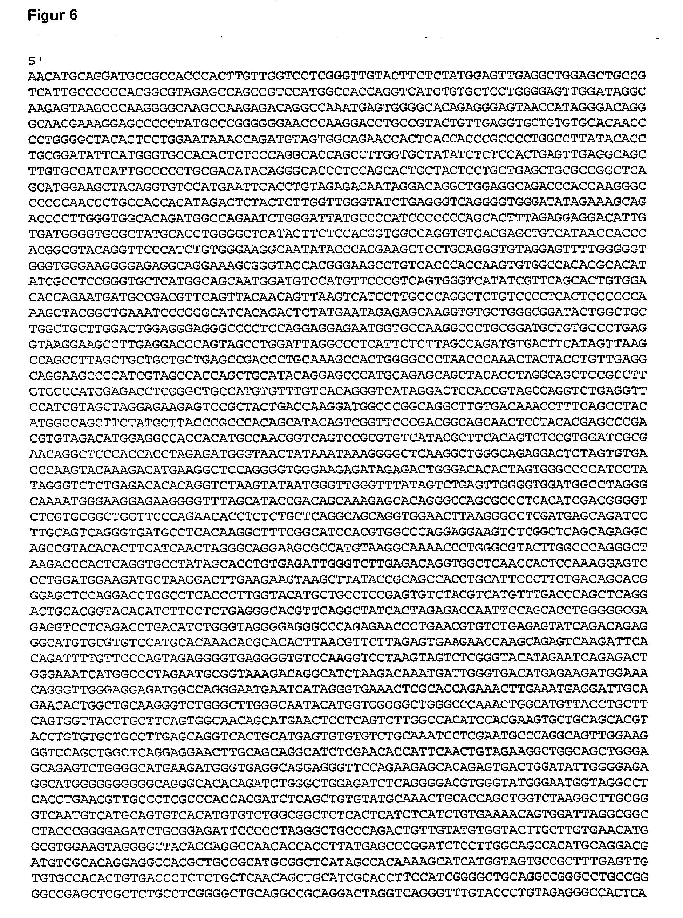

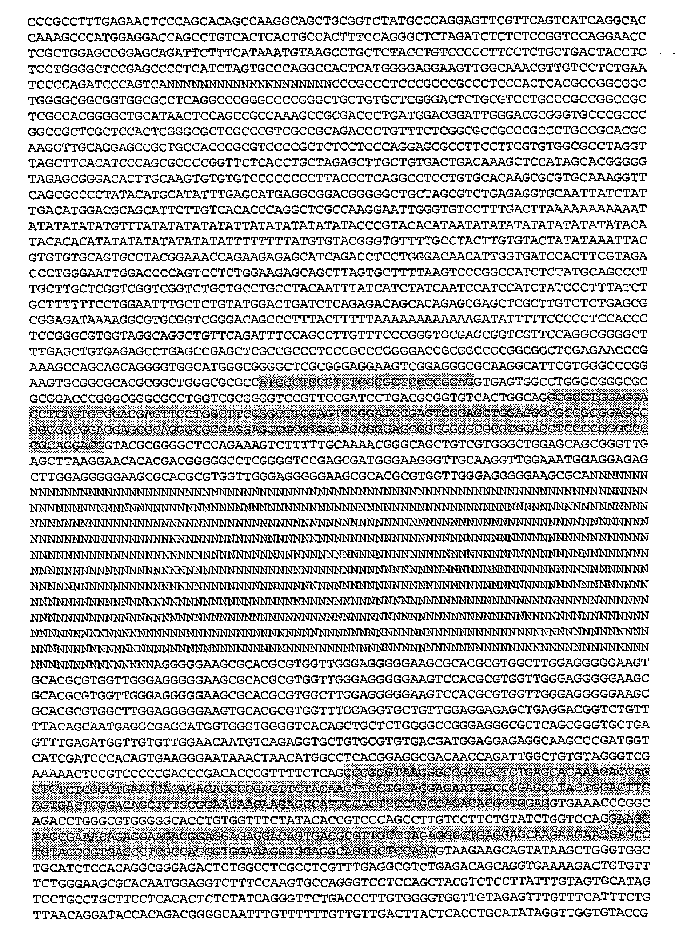

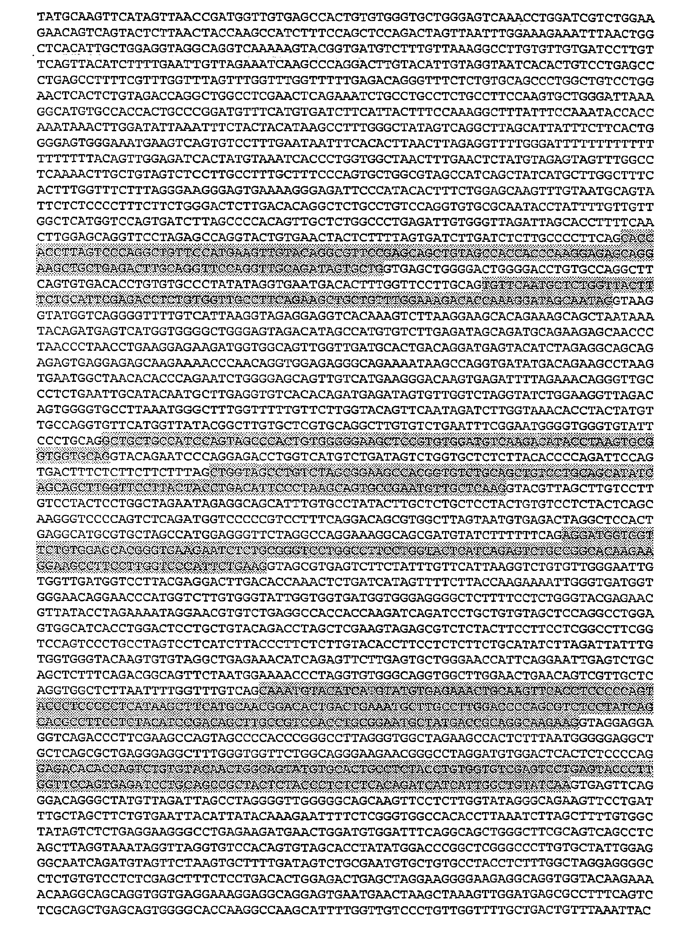

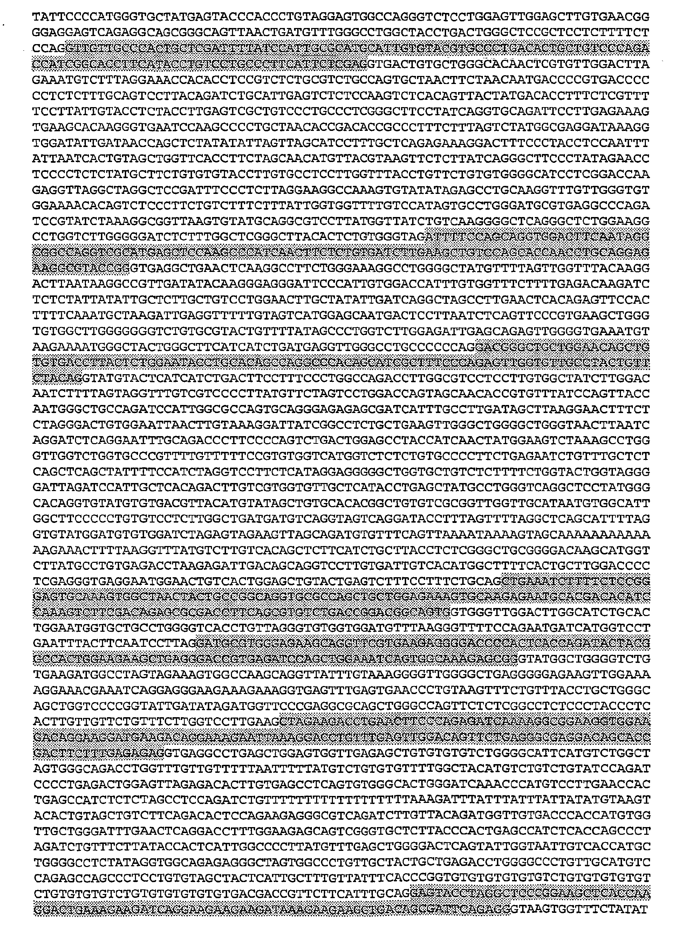

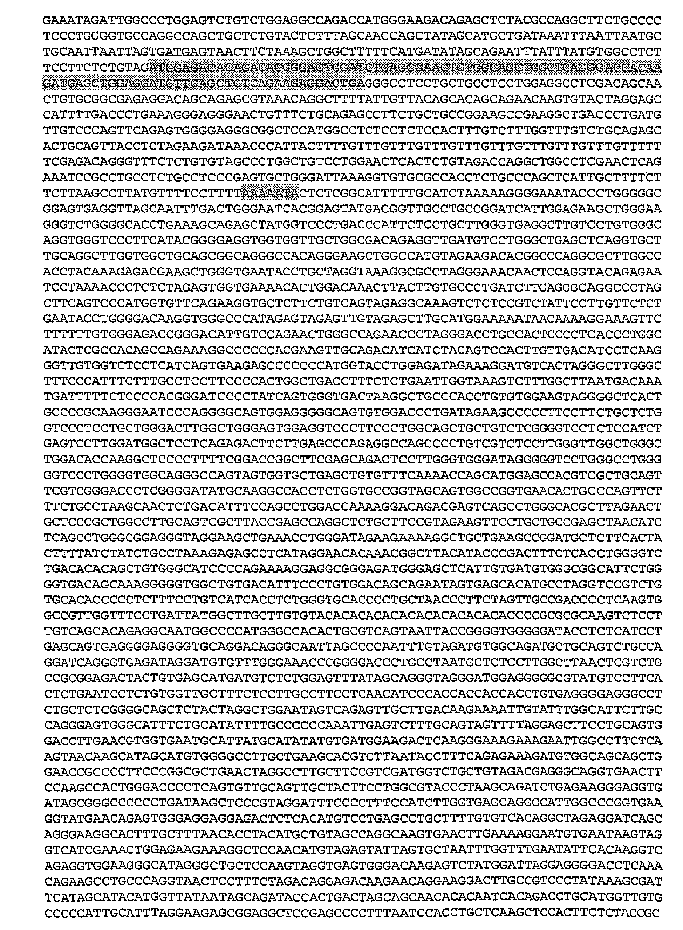

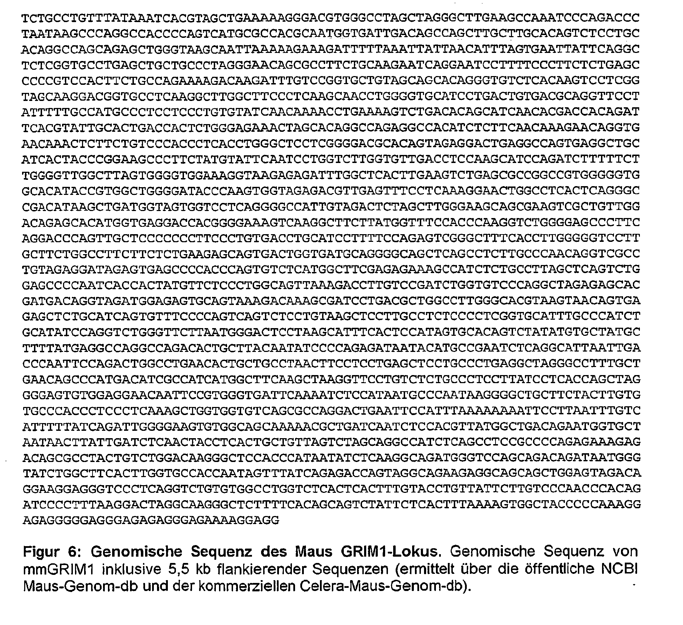

Die Charakteristika der genomischen Sequenzen von mmGRIM1 und hsGRIM1 sind in der folgenden Tabelle 4 gegenübergestellt. Die genomische Sequenz beider Spezies inklusive der detaillierten Exon-Intron Grenzen ist in den Figuren 5 und 6 angegeben.

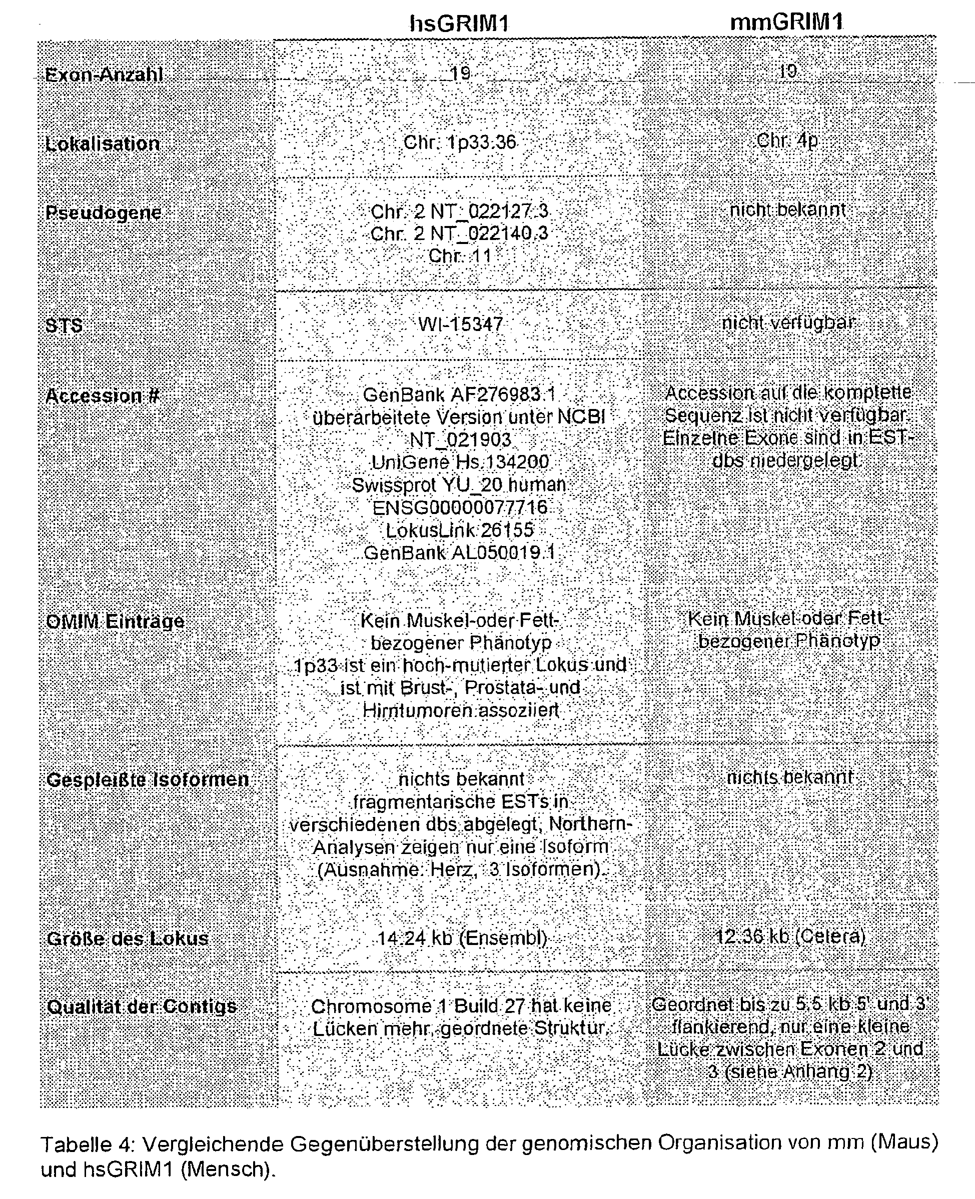

In der Figur 5 ist die genomische Sequenz von hsGRIM1 (NCBI Human Genome Server, Chromosome 1 map view, build 27, Dezember 2001) dargestellt. Exone sind mit Schraffur unterlegt, die putative poly-A site ist unterstrichen. Die Sequenz entspricht Seq. ID Nr. 5.

In der Figur 6 ist die genomische Sequenz von mmGRIM1 inklusive 5,5 kb flankierender Sequenzen (ermittelt über die öffentliche NCBI Maus-Genom-db und der kommerziellen Celera-Maus-Genom-db) dargestellt. Die Exone sind schraffiert unterlegt, intronische Sequenzen sind in heller dargestellt. Exon/Intron-Übergänge sind verstärkt dargestellt. Die putative poly-A site und der putative Transkriptionsstart sind unterstrichen. Noch nicht vollständig assemblierte Sequenzfragmente sind mit "N" dargestellt. Die genomische Maus-Sequenz hat die Seq.ID Nr. 6.

### Beispiel 7

### Expression von rekombinantem GRIM1 Protein

Zur Expression von rekombinantem hsGRIM1 Protein wurden viele verschiedene Strategien und Expressionssysteme getestet, erfolgreich verlief allerdings nur die Expression von kurzen Proteinfragmenten entweder im His₆ₓ- oder im GST-Kontext. Die folgenden Auflistungen fassen die Ergebnisse und die angewandten Methoden kurz zusammen.

### a) Expressionssysteme und kurze Beschreibung der Charakteristika zur Expression von Gesamtlänge hsGRIM1

### Bakterielle Systeme: alle verwendeten Systeme benutzen Escherichia coli Sicherheitsstämme, die von Stratagene käuflich erworben wurden.

*BL21(DE3)lysGOLD* His-fusioniert: Expression im pRSET-System oder in einem modifizierten pRSET-Vektor mit singulärem Hexahistidin-Tag, Aufreinigung über eine Nickelaffinitätsmatrix (TALON, Clontech). Co-expression mit GroESL und hspHJK Chaperonen. *BL21(DE3)lysGOLD* GST-fusioniert: Expression im pGEX4 oder 6 System von Pharmacia, Aufreinigung über GSH-Affinitätssäulen. Expresion über IPTG induzierbar. Co-Expression mit GroESL und hspHJK Chaperonen.
*BL21(DE3)lysGOLD* NusA-fusioniert: Erhöhung der Solubilität des Target-Proteins über den NusA-Anteil, Aufreinigung über GPC (Stratagene, Novagen)
*BL21(DE3)lysGOLD* MBP-fusioniert: periplasmatische Expression, dadurch verringerte Cytotoxizität (Stratagene, New England Biolabs pMAL-System)

*BL21(DE3)lysRIL:* Codon-optimierter BL21-Stamm (Stratagene). Sowohl Hexahistidin- als auch GST-fusionierte Proteinexpression.

*ABLE* C: Sicherheitsstamm, der endogene Plasmid-Kopienzahlen um den Faktor 4x heruntereguliert (Stratagene). Sowohl Hexahistidin- als auch GST-fusionierte Proteinexpression.
*ABLE K:* Sicherheitsstamm, der die endogene Plasmid-Kopienzahlen um den Faktor 10x heruntereguliert (Stratagene). Sowohl Hexahistidin- als auch GST-fusionierte Proteinexpression.

### Pflanzliche Systeme:

Laubmoos *Physcomitrella patens:* stabile Integration eines CaMV-getriebenen Expressionsplasmides für GST-hsGRIM1 (pRT99/35S-GST-hsGRIM1) über nicht-homologe Rekombination, Tripel-Selektion über zwei verschiedene Selektionsantibiotika und mehrfache Aussaaten (in Kooperation mit Prof. Ralf Reski, Institut für Pflanzenbiotechnologie der Uni-Freiburg).

### Zelluläre Systeme:

Expression von GST-Fusionsprotein in eukaryotischen Zellkulturzellen unter der Kontrolle eines konstitutiv aktiven CMV-Promoters.

### b) Expression von Peptidfragmenten von hsGRIM1

Kurze Proteinfragmente von hsGRIM1 wurden sowohl als Hexahistidin- und auch als GST-Fusionsproteine nur in *BL21(DE3)lysGOLD* ohne Ko-Expression von Chaperonen gewonnen. Die folgende Tabelle faßt die erreichten Expressionsresultate zusammen, nichtaufgelistete Fragmente (vergleiche dazu Tabelle der verwendeten hsGRIM1 Konstrukte) konnten nicht exprimiert werden:

**Tabelle 5: Bakteriell exprimierte hsGRIM1 Fragmente**

| | |
|---|---|
| His-E (aa 3-147). | +++ |
| His-I (aa 609-749) | +++ |
| His-H (aa 485-749) | ++ |
| GST-E (aa 3-147) | +++ |
| GST-I (aa 609-749) | +++ |
| GST-H (aa 485-749) | ++ |
| GST-M (aa 468-749) | + |
| GST-R (aa423-485) | ++ |

| | |
|---|---|
| +++ gut löslich, gut induzierbar ++ gut induzierbar, geringe Ausbeute + t.w. schlecht löslich, geringe Ausbeute | |

### Beispiel 8

### Generation und Beschreibung der verwendeten α-GRtM1 Antikörper

Zur Herstellung von hsGRIM1-spezifischen Antikörpern wurden zwei Hexahistidin-fusionierte hsGRIM1-Proteinfragmente in E.coli exprimiert, gereinigt und zur Immunisierung von Kaninchen verwendet. Als Epitope für den Antikörper dienten die Aminosäuren 3-147 und 609-749 von hsGRIM1.

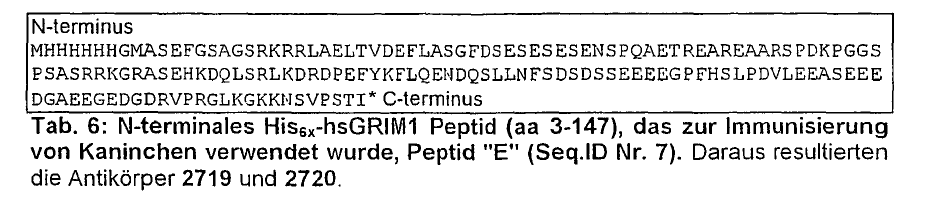

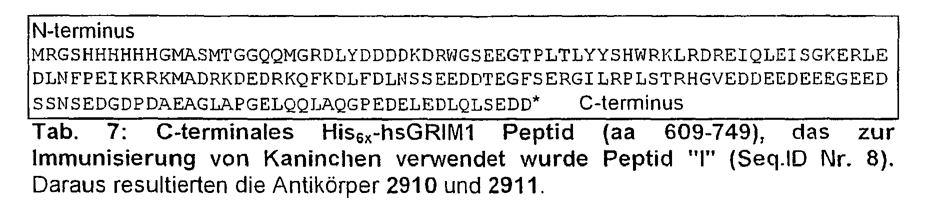

Diese Regionen entsprechen den putativen INHAT-Domänen von hsGRIM1 und zeichnen sich besonders durch saure (polyE/polyD-Cluster) aus. Die entsprechenden Bereiche der hsGRIM1 cDNA-Sequenz wurden durch Restriktionsverdau mit endogenen EcoRl- (aa147) und Xmal- (aa609) Schnittstellen generiert und die Produkte in den Vektor pRSET-B (Invitrogen) (aa 609-749) oder pRSET-N*/H basiert auf dem pRSET-B, mit dem Unterschied, daß weitere Tags entfernt wurden und das Protein nur mit Hexahistidin-Tag exprimiert wird) (aa 3-147) kloniert. Die Hexahistidin-fusionierten hsGRIM1-Proteinfragmente (aa 3-147 "E", aa 609-749 "I") wurden in *E. coli* BL21 (DE3)lysGOLD überexprimiert und nach dem Aufschluß der Bakterien durch TALON-Affinitätschromatographie gereinigt. Nach der Isolierung und Reinigung aus *E. coli* wiesen beide Polypeptide eine geschätzte Reinheit von ca. 80-90 % auf. Die Immunisierung von Kaninchen wurde mit den Polypeptiden durchgeführt und das erzeugte Antiserum gereinigt. Die gereinigten monospezifischen Antikörper gegen aa 3-147 (2719, 2720) und gegen aa 609-749 (2910 und 2911) wurden funktionell charakterisiert.

Die hierbei erhaltenen Antikörper wurden in verschiedenen immunologischen Testverfahren eingesetzt und ermöglichten den Nachweis der Expression von GRIM1. Die beiden gegen den N-Terminus gerichteten Antikörper zeigen GRIM1 Lokalisation exklusiv nukleär in subnukleären Kompartimenten, die gemeinhin als "Speckle" bezeichnet werden. Die Anzahl und Größe der nukleären Speckles variiert von Zellinie zu Zellinie, gemein ist nur die exklusiv nukleäre Lokalisation von GRIM1 in proliferierenden Zellen. Die beiden gegen den C-Terminus gerichteten Antikörper erkennen exklusiv nukleäres GRIM1 in proliferierenden Zellen, allerdings nicht in Speckles, sondern in einer pan-nukleären Lokalisation im gesamten Nukleoplasma.

### Beispiel 9

### Expression von GRIM1-mRNA und -Protein

### cDNA/mRNA-Expression von hsGRIM1 und mmGRIM1

Die cDNA-Expression von hsGRIM1 ist dem Bericht der Unigene Datenbank zufolge (UniGene Cluster Hs.134200, Homo sapiens DKFZP564C186/DKFZP564C186-Protein mit der verwendeten cDNA-Bibliothek 2334BT0407) ubiquitär. Nachgewiesene Expression in:
*Cervix, Pancreas, amnion normal, bone, bone marrow, brain, breast, cervix, colon, colon_est, colon_ins, denis_drash, ear epid_tumor, esophagus, eyes, genitourinary tract, germ cell, head_neck heart, kidney, kidney_tumor, liver lung, lung_normal, lung_tumor, lymph, muscle, nervous_normal, ovary, pancreas, placenta, pool, pooled, pooled brain, lungs, testis, pooled colon, kidney, stomach, pooled lung and spleen, prostate, prostate_tumor, salivary gland, skin, small intestine, stomach, testi, testis-cell line, thymus pooled, tonsil, uterus, uterus_tumor, whole embryo*

Das Expressionsmuster von hsGRIM1 wurde dargestellt über Northern Blot-Analyse (CLONTECH hsMTN1 und hsMTN II, Sonde: sowohl Gesamtlänge hGRIM1 cDNA bzw. N- und C-terminale Fragmente). Die hsGRIM1-Transkriptgröße beträgt ca. 3.3 kb (in Übereinstimmung mit der niedergelegten Gesamtlänge-mRNA unter der Acc. # al 050019):
*Herz (1), Gehirn (2), Plazenta (3), Lunge (4), Leber (5), Skelettmuskel (6) Niere (7), Bauchspeicheldrüse (8), Milz (9), Thymus (10), Prostata (11), Testi (12, Ovarien (13), Dünndarn (14), Dickdarm (15), periphére Lymphozyten (15)*

Die Expression von mmGRIM1 in verschiedenen Geweben der adulten Maus (4-6 Wochen) wurde über RT-PCR bestimmt. Folgende Gewebe und Organe wurden als mmGRIM1 positiv klassifiziert:
*Brust, Brust saugender Tiere, Plazenta, Testis, Ovarien, Fett, Haut, Knochen, Knorpel, Milz, Lunge, Adrenerge Drüse, Niere, Leber, Dünndarm, Magen, Hypophyse, Thymus, Zunge, Skelettmuskel, Herz, Auge, Rückenmart, Kleinhirn Medulla, Hypothalamus, Hirnrinde, Gesamthirn*

Die Expression von mmGRIM1 mRNA in der Embryonalentwicklung wurde über RT-PCR mit Gesamt-Embryo mRNA bestimmt. Zu allen Zeitpunkten wird ein deutliches mmGRIM1 Signal detektiert, eine starke Veränderung der mmGRIM1 mRNA Expression während der Entwicklung ist nicht zu beobachten.

Dargestellt ist in Tabelle 8 eine UNIGENE Datenbank-Analyse zur EST-Expression ausgewählt in Homologie zu mm/hsGRIM1-Protein. Angegeben sind Accession-Nummern der IMAGE-Klone oder der teilweise unbekannten Sequenzen sowie der Gewebetypus, aus dem die cDNAs isoliert wurden. GRIM1 scheint auch auf Proteinebene ubiquitär exprimiert zu werden. Insgesamt wurden 308 ESTs gefunden, davon sind nachfolgend diejenigen aufgelistet, die eine eindeutige Gewebezuordnung zeigen:

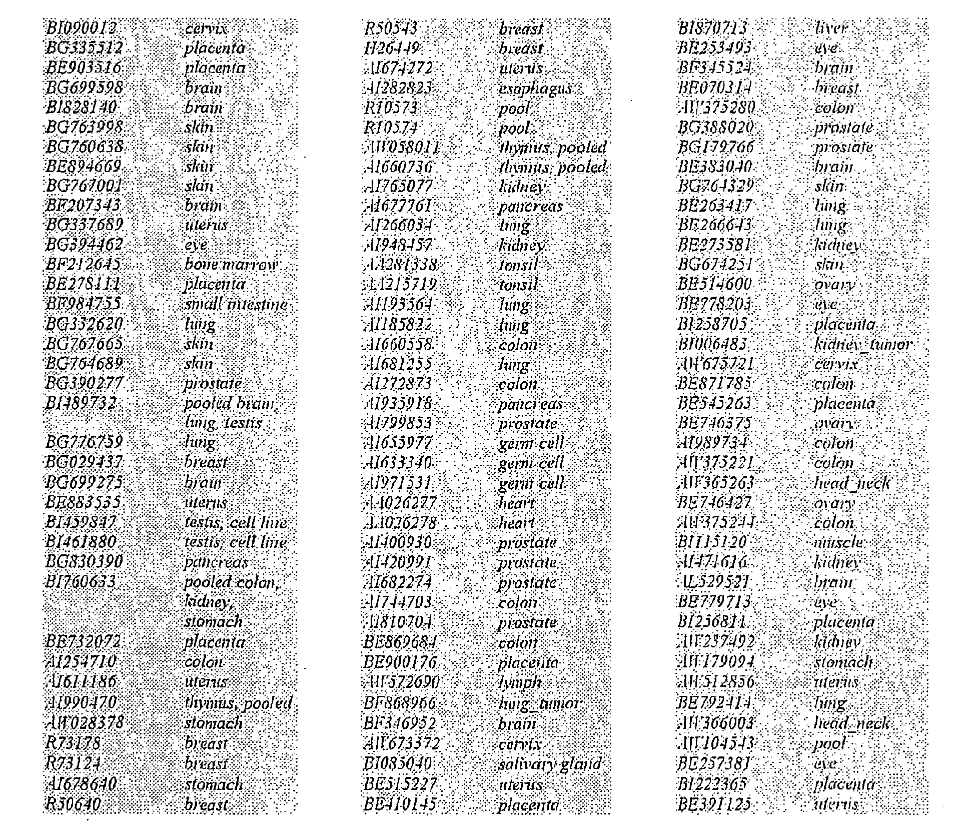

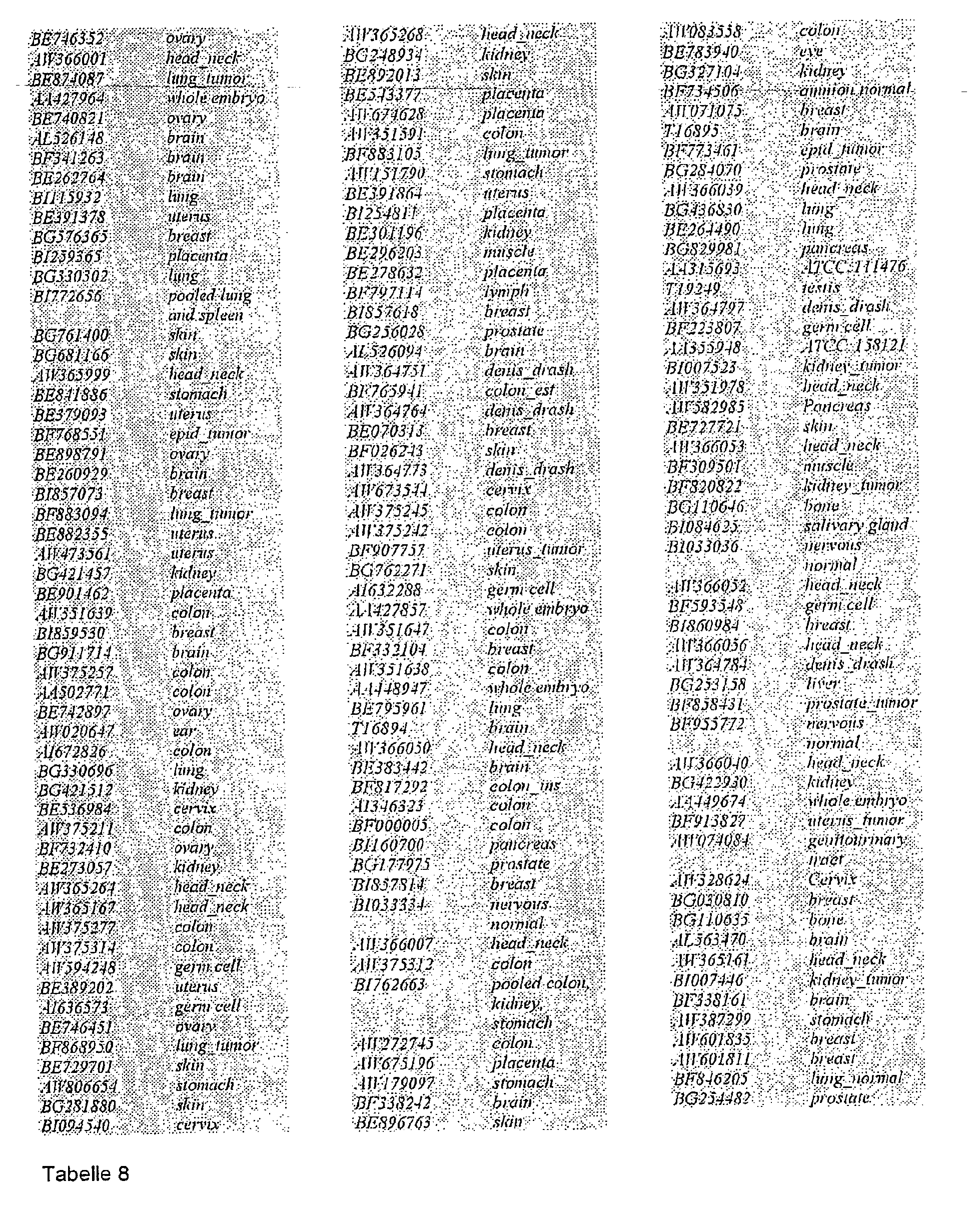

### Beispiel 10

### Protein-Expression

Da verschiedene Experimente nicht ohne weiteres am Menschen durchgeführt werden können, wurden Zellkultur- und Tierversuche durchgeführt, die aber auch für die Situation am Menschen Aussagekraft haben.
a) Die Expression von mmGRIM1-Protein in primären Zellen und Geweben ist nachfolgend angegeben (alle aufgeführten Zellen sind als GRIM1-positiv klassiert in Western-Blot (86kD), bei Zellen wurde auch eine Überprüfung der Expression in IIF vorgenommen). Signale werden mit beiden Antikörpern (N- und C-terminales Epitop) gewonnen:
   *Maus embryonale Fibroblasten Humanes Herzgewebe (DCM-Patienten und Normal-Herz) Maus Glattmuskelzellen (differenziert, undifferenziert und proliferierend) Maus-Orange: Testis, Ovarien, Milz, Thymus und zu geringer Menge auch dargestellt), Gehirn, Lunge, Leber.*
b) Die Expression von mmGRIM1-Protein konnte nachgewiesen werden über *in situ* Immunohistochemie an Sagittalschnitten von E10.5 Maus-Embryonen. mmGRIM1 wird in epithelialen Strukturen, im Dermomyotom der hinteren Extremitäten, in den inner-epithelialen Bereichen des Neuralrohres und in weiteren, noch nicht näher bestimmten Zellpopulationen exprimiert. Nur im Dermomyotom ist eine konzentrierte Expression zu detektieren, in den restlichen Arealen sind nur vereinzelte mmGRIM1 positive Zellen detektierbar. In mRNA- und EST-Analysen wurde eine ubiquitäre Expression von GRIM1 festgestellt. In in situ immunohistochemischen Analysen an E10.5 Embryonen der Maus konnte gezeigt werden, daß mmGRIM1 zwar in jedem Gewebetypus exprimiert wird, bei weitem aber nicht in jeder Zelle, sondern eher in spezialisierten Zellpopulationen. mmGRIM1 ist nicht in jedem Zelltyp exprimiert, jedoch verteilt über den gesamten Organismus in spezialisierten Zelltypen.
c) Die Expression von rnGRIM1 (rn = Ratte) im Gehirn der Ratte wurde nachgewiesen über in situ-Immunohistochemie in Sagittalschnitten durch das Gehirn einer Sprague Dawley Ratte (*Rattus norvegicus ssp*).
   *rnGRIM1 wird in Purkinje-Zellen des Kleinhirns exprimiert (nukleär und t.w. auch cytoplasmatisch; starke Immunoreaktivität in 1-2 sub-nukleären Strukturen), in bipolaren Zellen der Mitralschicht des Lobus olfaktorius (nuklear und t.w. auch cytoplasmatisch; starke Immunoreaktivität in 1-2 und t.w. auch cytoplasmatisch; starke Immunoreaktivität in zumeist 2 sub-nukleären Strukturen), in noch nicht naeher bestimmten Zellen im gesamten Breich des Cortex Immunoreaktivität in 1-2 sub-nukleären Strukturen). rnGRIM1 ist nicht in jedem Zelltyp exprimiert, jedoch verteilt über das gesamte Hirnareal in spezialisierten Zelltypen.*
d) Expression von GRIM1-Protein in Zellkulturzellen. Alle aufgeführten Linien sind als GRIM1-positiv klassiert in Western-Blot Analysen (Spezies-übergreifende Größe von ca. 86 kD) und über indirekte Imtnunfluoreszenz (IIF). Beide Assays wurden mit allen verfügbaren α-GRIM1 Antikörpern durchgeführt. Im proliferativen Zustand ist GRIM1 in jeder Zell-Linie in sogenannten "speckle"-Strukturen zu detektieren (wie in der Literatur auch für viele Ko-Repressoren beschrieben): 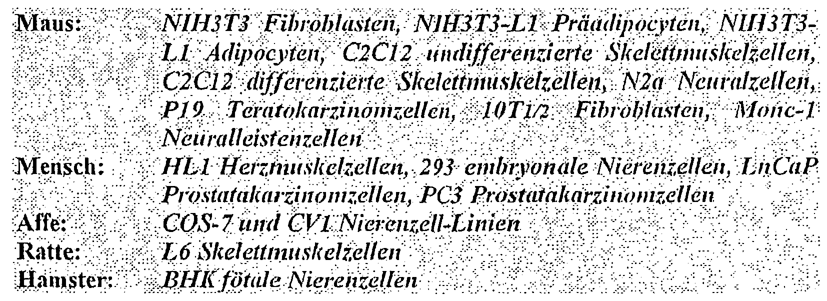
**e) *Drosophila melanogaster* GRIM1**
   Die Expression eines hsGRIM1 ähnlichen Proteins in *Drosophila* melanogaster wurde über Western Blot und whole-fly *in situ* Hybridisierungen hin untersucht.
   Weder in Gesamtlarvenlysat noch in Gesamtzellextrakten von Fliegenköpfen wurde über Western Blotting mit den anti-hsGRIM1 spezifischen Antikörpern ein spezifisches Signal detektiert (Auflösung von 36 bis 210 kD). In *in situ* Hybridisierungen mit verschiedenen Larvalstadien konnte ebenfalls kein spezifisches dmGRIM1 (dm = Drosophila melanogaster) Signal mit den vorhandenen GRIM1-Antikörpern detektiert werden.

### Beispiel 11

### Interaktionspartner von hGRIM1

In den bisher durchgeführten Protein-Protein-Interaktionsstudien wurden folgende potentielle Interaktionspartner von mm/hsGRIM1 auf eine direkte oder indirekte Interaktion mit folgenden Methoden untersucht:

| **METHODE ZUR Detektion der Protein-protein-Interaktion** | **Symbol** |
|---|---|
| Co-IP aus Zellextrakten transfizierter Zellen | **tfIP** |
| Co-IP aus Zellextrakten untransfizierter Zellen | **utfIP** |
| Co-IP mit *in vitro* translatierten Proteinen | **ivIP** |
| Hefe-THS | **yTHS** |
| mammalian THS | **mTHS** |
| Pulldown-Analysen (direkte Interaktion) | **PD** |
| transkriptionelle Studien (transiente Transfektionen) | **tT** |

Unterstrichene Faktoren zeigten eine Interaktion mit hs/mm/maGRIM1 und sind am Ende der Aufzählung gesondert beschrieben (ma= *mesocricetus aureus,* BHK Zellen).

### Nukleäre Hormon-Rezeptoren

hsAR (PD, utfIP, tfIP, yTHS, tT), hsERα, (utfIP, tfIP), hsERβ, rnGR (tT, tfIP), hsMR (tT), hsPR (tT), PPARγ (tT), hsRARα (tfIP), rnTRα (tT), hsTRβ-2 (tT), mmERR1 (tfIP, tT), mmGCNF (PD, ivIP, tfIP, tT), hsRevErbA (tT), mmRVR (tT, tfIP), mmSF1 (tT).

### Transkriptionsfaktoren

Myogenin (ivIP, tT), MEF2A (ivIP), MEF2B (ivIP), MEF2C (ivIP, tT), eHand (ivIP), dHand (ivIP), E47 (ivIP), E12 (ivIP), GATA4 (ivIP), MyoD (ivIP, tT), SEF (ivIP, tT), MRF4 (ivIP, tT), Myf5 (ivIP), Sp1 (tT), CBF (tT).

### Transkriptionelle Ko-Repressoren.

N-CoR (ivIP, tfIP, utfIP, PD), Sin3A (ivIP, tfIP, utfIP), Sin3B (tfIP, utfIP), SuN-CoR (ivIP, tT), SMRT (ivIP, tfIP, utflP), HDAC1 (tfIP, utfIP), HDAC2 (tfIP, utfIP), RIP140 (ivIP).

### Transkriptionelle Ko-Aktivatoren:

p300 (tT), FHL2 (ivIP, PD), FHL3 (PD), TIF2 (ivIP), RIP140 (ivIP) Weitere Faktoren: Histone H3, H4, H2A, H2B (PD)

Eine starke direkte Interaktion zwischen hsGRIM1/mmGRIM1 konnte bislang nur mit dem Androgen-Rezeptor (hsAR) gezeigt werden (nachgewiesen über tfIP in beiden Richtungen, utflP in beiden Richtungen, yTHS, PD und tT). Die Interaktion mit dem Androgen-Rezeptor ist Liganden-unabhängig und ist nicht durch Gegenwart von Antagonisten (Casodex, Hydroxy-Flutamid, Cyproterone-Acetat) beeinflussbar. GRIM1 interagiert sowohl mit dem "normalen" AR mit 17 Glutaminen, als auch mit AR-Isoformen, die mit der Kennedy-Krankheit assoziiert sind und polyglutaminreiche Insertionen (17, 44 und 77 Q) beinhalten, ebenfalls Ligandenunabhängig. Die PD-Analysen wurden mit den GRIM1 Fragmenten GST-I/EIH/M durchgeführt, wobei alle getesteten Fragmente bis auf GRIM1-"E" (aa 3-147) mit dem AR interagierten, in der Reihenfolge M≥H>>I. Die physiologische Relevanz der AR-GRIM1 Interaktion ist in einem separaten Kapitel näher beschrieben.

Schwache Wechselwirkungen wurden zwischen hsGRIM1 und den nukleären Orphan-Rezeptoren Germ Cell Nuclear Factor (mmGCNF) und Estrogen receptor Related Receptor 1 (mmERR1) nachgewiesen (nur ivIP, tfIP und PD) sowie eine schwache Wechselwirkung mit dem Östrogen-Rezeptor α (hsERα; Interaktion wurde nur in tfIP nachgewiesen und zeigte sich Liganden-unabhängig, sowohl in Gegenwart von Agonisten (Estradiol), von Antagonisten (Raloxifen, Tamoxifen, ICI) als auch in Abwesenheit eines Liganden). Die physiologische Relevanz, *in vivo*-Kolokalisation oder *in vivo*-Interaktion ist weitestgehend unklar. In transienten Transfektionen konnte kein direkter Zusammenhang zwischen GRIM1 und mmGCNF, mmERR1 oder hsERα gezeigt werden.

GRIM1 zeigt eine starke Interaktion mit den Core Histonen H4, H2B, H2A und H3 in GST-Pulldown-Analysen. Rekombinant exprimierte GST-GRIM1 Fusionsproteine (GST-aa 3-147 und GST-aa 609-749) zeigen eine deutliche Interaktion mit allen vier Core-Histonen, was die beobachtete INHAT-Aktivität der N- und C-terminalen GRIM1 Domänen weiter untermauert.

Potentielle Interaktionspartner von mm/hsGRIM1 können mit üblichen Methoden identifiziert werden. Diese Methoden umfassen Hefe Two Hybrid-Screen mit diversen cDNA Banken und direkte proteinbiochemische Methoden wie Co-IP von GRIM1 und assoziierten Proteinen, Auftrennung über 2-dimensionale Gelelektrophorese, massenspelctrometrische Analyse und Mikrospray-Sequenzierung der potentiell interagierenden Proteine aus verschiedenen Zellkulturzell-Linien (C2C12 differenziert, C2C12 undifferenziert, 3T3L1 differenziert, 3T3L1 undifferenziert, BHK uvm.).

### Beispiel 12

### Physiologische Funktion von GRIM1: Analyse des Repressionspotentials von hsGRIM1 in transienten Transfektionen

Alle beschriebenen Daten wurden mit dem hsGRIM1 Klon gewonnen. GRIM1 ist ein potenter transkriptioneller Repressor, wenn er als Gal4-DBD-Fusionsprotein in transienten Transfektionen verwendet wird. Gal-GRIM1 reprimiert die Expression von Reportergenen sowohl von Gal4-RE-kontrollierten komplexen und synthetische Minimal-Reporterkonstrukten. Das Repressionspotential von GRIM1 ist zum Beispiel im Vergleich mit dem Ko-Repressor Gal-SuN-CoR deutlich stärker. In diesen Experimenten werden die entsprechenden Ko-Repressoren als heterologe Ga14-DBD Fusionsproteine transient in Zellen exprimiert. Das Repressionsvermögen auf verschiedene synthetische Promoter-Reporterkonstrukte im Vergleich zu gleichen Mengen an transfizierter Wildtyp Gal4-DBD wird über Luciferase-Reportergenexpression bestimmt.

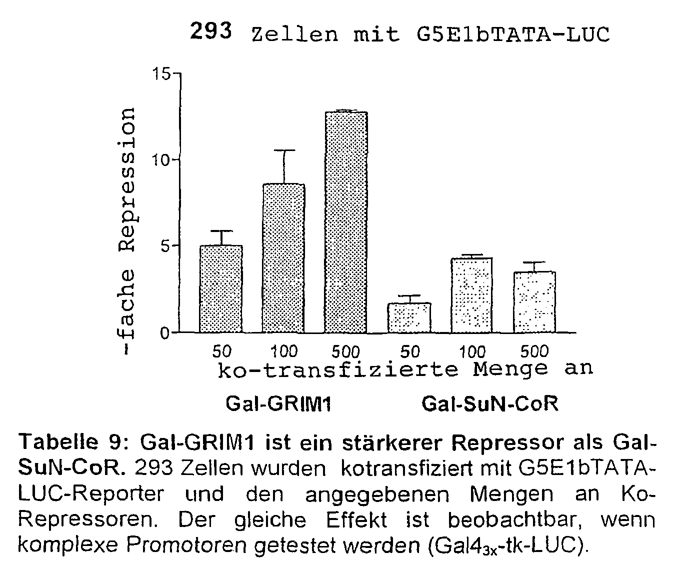

Gal-GRIM1 ist ein gleich starker Repressor wie eine Vielzahl beschriebener Ko-Repressoren wie z.B. Gal-HDAC1-10, Gal-CBF1, Gal-Alien und reprimiert nur unbedeutend schwächer als Gal-N-CoR-RD oder Gal-SMRT-RD. Das Repressionspotential von Gal-GRIM1 wurde in Verbindung mit den komplexen Gal4₃ₓ-tk-LUC und Gal-MMTV-LUC Promotor/Reporterkonstrukten und in Verbindung mit den Minimal-Promotor/Reporterkonstrukten Gal₅ₓ-E1b-TATA-Box und Gal₃ₓ-βglobin-TATA-LUC untersucht. Desweiteren wurde das Repressionsvermögen von Gal-GRIM1 gegenüber einer mittels LexA-DBD an DNA gebundenen autonomen VP16 Transaktivierungsdomäne über einen LexA₈ₓGal₅ₓ-LUC Reporter untersucht (von Dr. S. Khochbhin zur Verfügung gestellt). Die erhaltenen Daten zu Gal-GRIM1 sind in den folgenden Zell-Linien experimentell getestet worden: A7r5, C2C12, CV1, BHK, 293, N2a, COS-7. Gezeigt in der folgenden Abbildung sind repräsentative Ergebnisse für BHK und C2C12 Zellen.

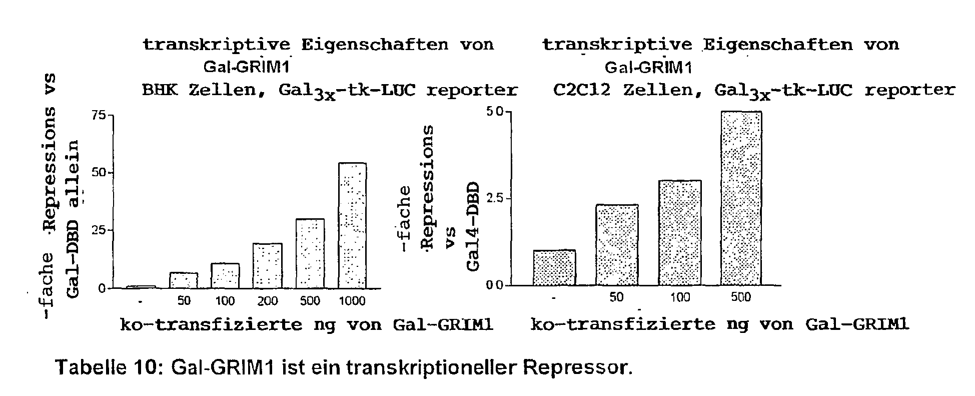

Die Repression von Gal-GRIM1 wird durch Zugabe der spezifischen Histon-Deacetylase (HDAC)-Inhibitoren Trichostatin A (TSA, 300nM) und von Natrium-Butyrat (Na-But, 5 mM) nicht beeinträchtigt, was einen grundlegend anderen Repressionsmechanismus wie für andere Ko-Repressoren beschrieben nahelegt. Vergleichbare Fälle wurden für andere Repressoren beschrieben, allerdings wird keine methodische Erklärung beschrieben. Zudem zeigen die beschriebenen Faktoren keine Beteiligung an Skelettmuskeldifferenzierung oder Adipogenese. Daten zur Unabhängigkeit von HDACs in transienten Transfektionen sind in der folgenden Abbildung exemplarisch für BHK Zellen dargestellt. Die transkriptionelle Aktivität von Gal-GRIM1 wird in keiner bereits getesteten Zell-Linie durch TSA beeinflusst.

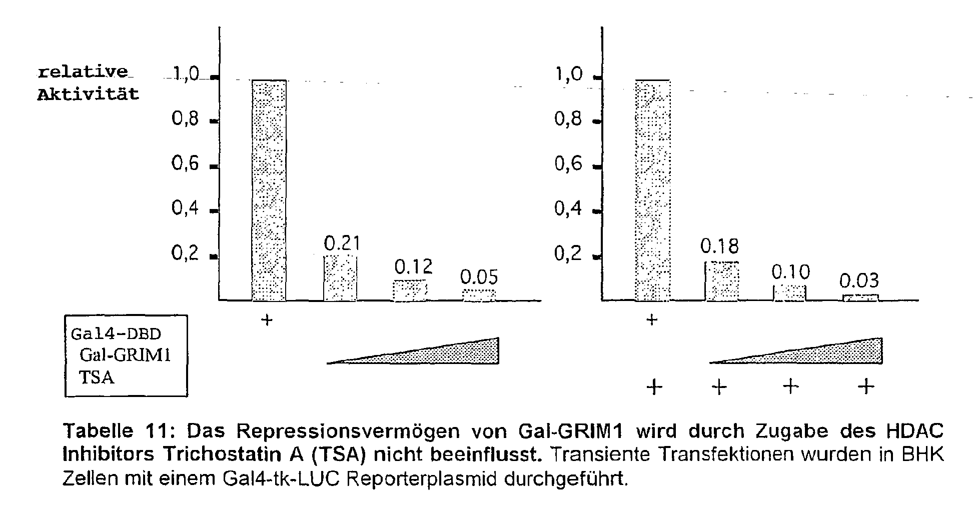

Zur Bestimmung der an der Repression beteiligten Domänen von GRIM1 wurden insgesamt 11 Deletionsmutanten von GRIM1 konstruiert und alle im Ga14-DBD Kontext auf Ihr Repressionspotential in allen bereits beschriebenen Reportersystemen getestet. Alle verwendeten Domänen von GRIM1 zeigen transkriptionelle Repression im Bereich von 2x (Gal-E und Gal-I) bis hin zu 15-20x (Gesamtlänge Gal-GRIM1). Alle getesteten Deletionsmutanten zeigten sich insensitiv gegenüber HDAC-Inhibitoren.

Zusammenfassend konnte gezeigt werden, daß GRIM1 mindestens 5 unabhängige Repressionsdomänen besitzt, wobei die 3 Haupt-Repressionsdomänen im hydrophoben Kernbereich des Proteins liegen (mutQ, mutR, mutP). Allerdings zeigt jede analysierte Deletionsmutante im Gal4-DBD Kontext ein Repressionsvermögen, welches signifikant über dem Hintergrund liegt.

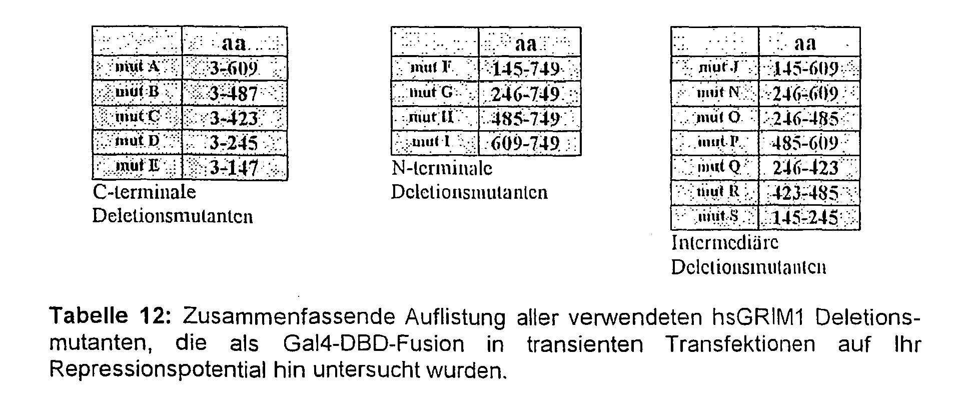

Die innerhalb der Sektion "INHAT-Aktivität" beschriebenen Subdomänen "E" und "I" von GRIM1 zeigen in den transkriptionellen Assays als Gal4-DBD Fusionen den geringsten repressiven Effekt, sind aber beteiligt an der Verhinderung der Histon-Acetylierung durch p300. Weitere Informationen zu den Domänen Q, P und R liegen nicht vor, eine direkte Beteiligung bei der Rekrutierung von zusätzlichen Ko-Repressoren konnte durch die oben angeführten Interaktionsstudien nicht belegt werden. Momentan gibt es nur Spekulationen über den von GRIM1 angewandten Repressionsmechanismus, allerdings kann eine Beteiligung von HDACs ausgeschlossen werden, und es scheinen mindestens zwei verschiedenen Mechanismen verwendet zu werden (E und I im Gegensatz zu Q, R und P).

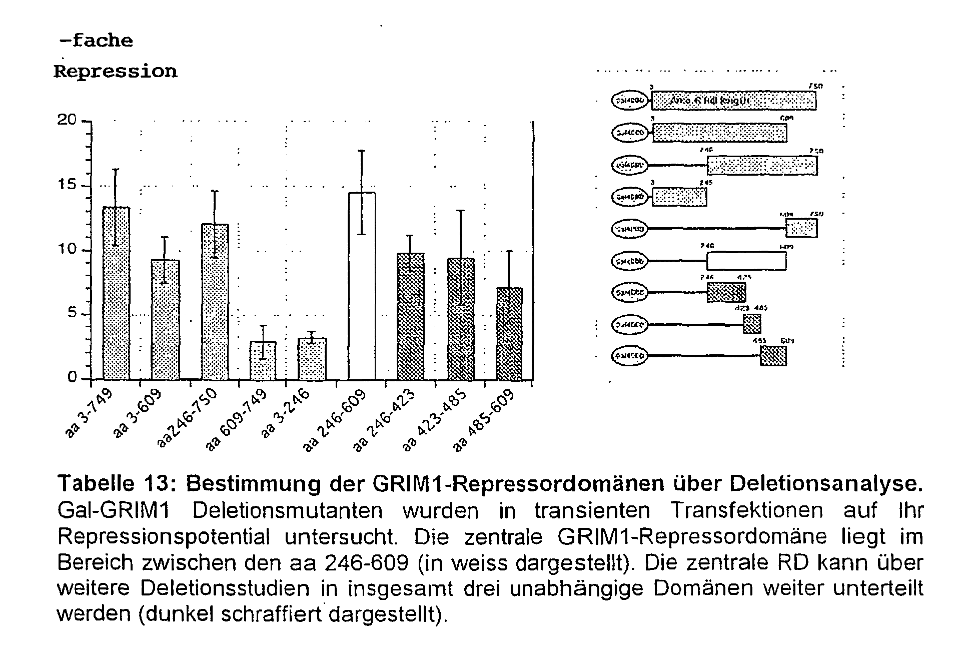

Ergebnisse mit dem LexA₈ₓGal₅ₓ-System bestätigen diese Ergebnisse, zeigen aber eine andere relative Repression der einzelnen untersuchten Fragmente (Daten nicht gezeigt).

Gesamtlänge GRIM1 ist in der Lage, sowohl komplexe natürlich vorkommende als auch synthetische Minimalpromotoren zu reprimieren. In transienten Transfektionen wurden folgende Systeme getestet: GRIM1 und komplexe Promotoren (p21 (-4542)LUC, SKA-LUC, Calponin-LUC, Myogenin-LUC, Myogenin proximaler Promotor-LUC, Thymidin-Kinase-LUC, Probasin-LUC, MMTV-LUC uvm.).

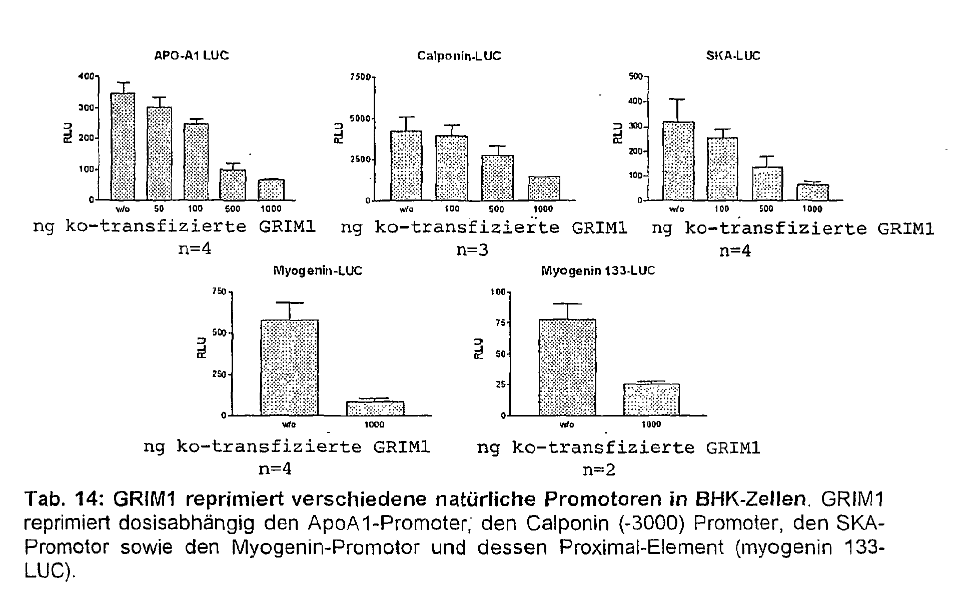

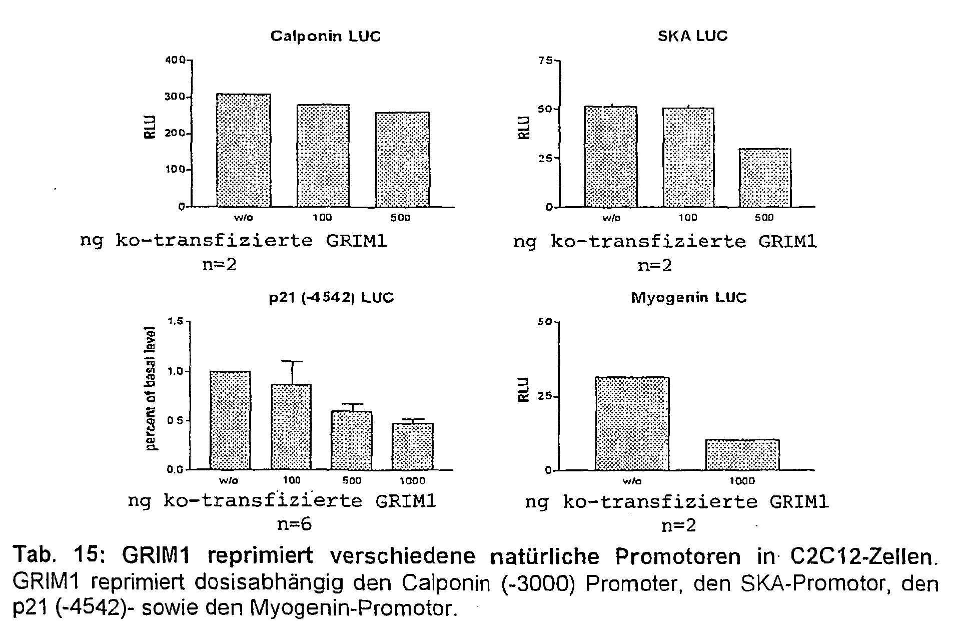

Kotransfektionen von-GRIM1 und E1b TATA Box beinhattende Minimalpromatoren mit synthetischen TF -Bindestellen (AR-, Gal4- und SEF-Bindestellen) oder mit dem komplexen MMTV-Promoter führt zu einer Repression der basalen transkriptionellen Aktivität (alle gezeigten Transfektionen wurden in BHK Zellen durchgeführt).

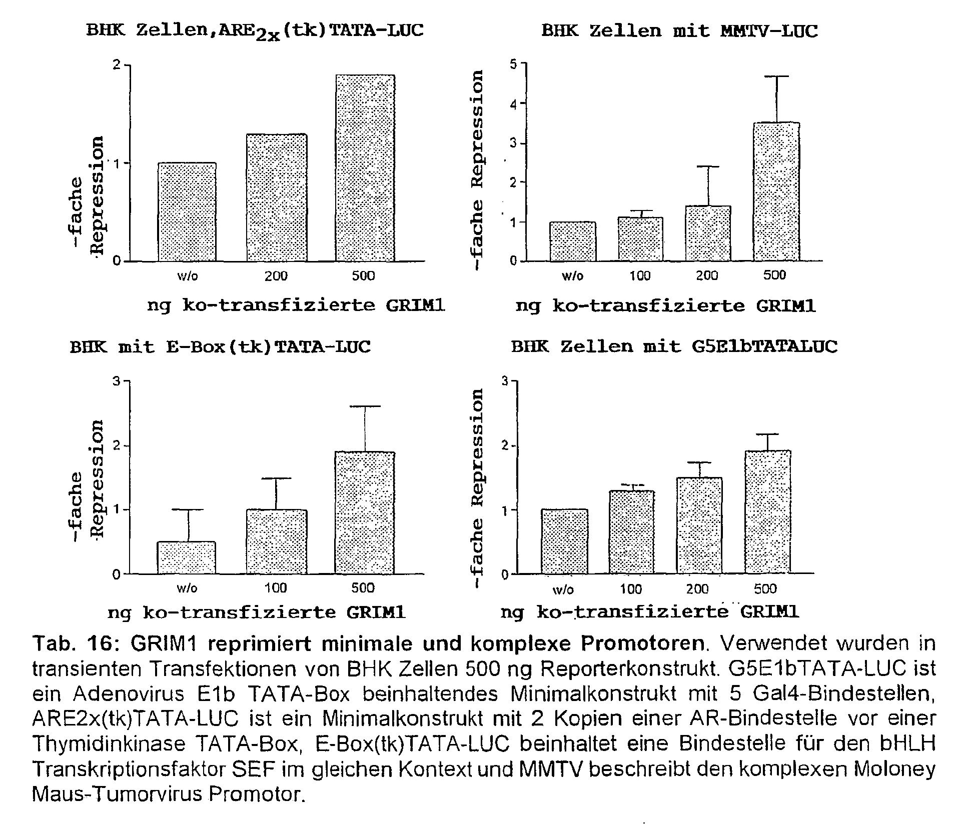

Die Repression von GRIM1 bei Minimalpromotoren ohne synthetische TF Bindestellen ist durch den niedrigen Basalwert der verwendeten Konstrukte nicht genauer darstellbar.

Das Repressionspotential von GRIM1 auf Promoter-Luciferase-Reporter-Konstrukte, die stabil ins Genom der jeweiligen Zell-Linie integriert wurden (HRL+N mit RARE₃ₓ-LUC, NIH3T3 mit cycA-LUC, 293 mit NFκB-RE₄ₓ-LUC) ist in der nächsten Abbildung zusammengefasst. Transfektion der Zellen mit GRIM1 zeigt in induziertem und in basalem Zustand keinen Einfluss auf die Reportergen-Transkription.

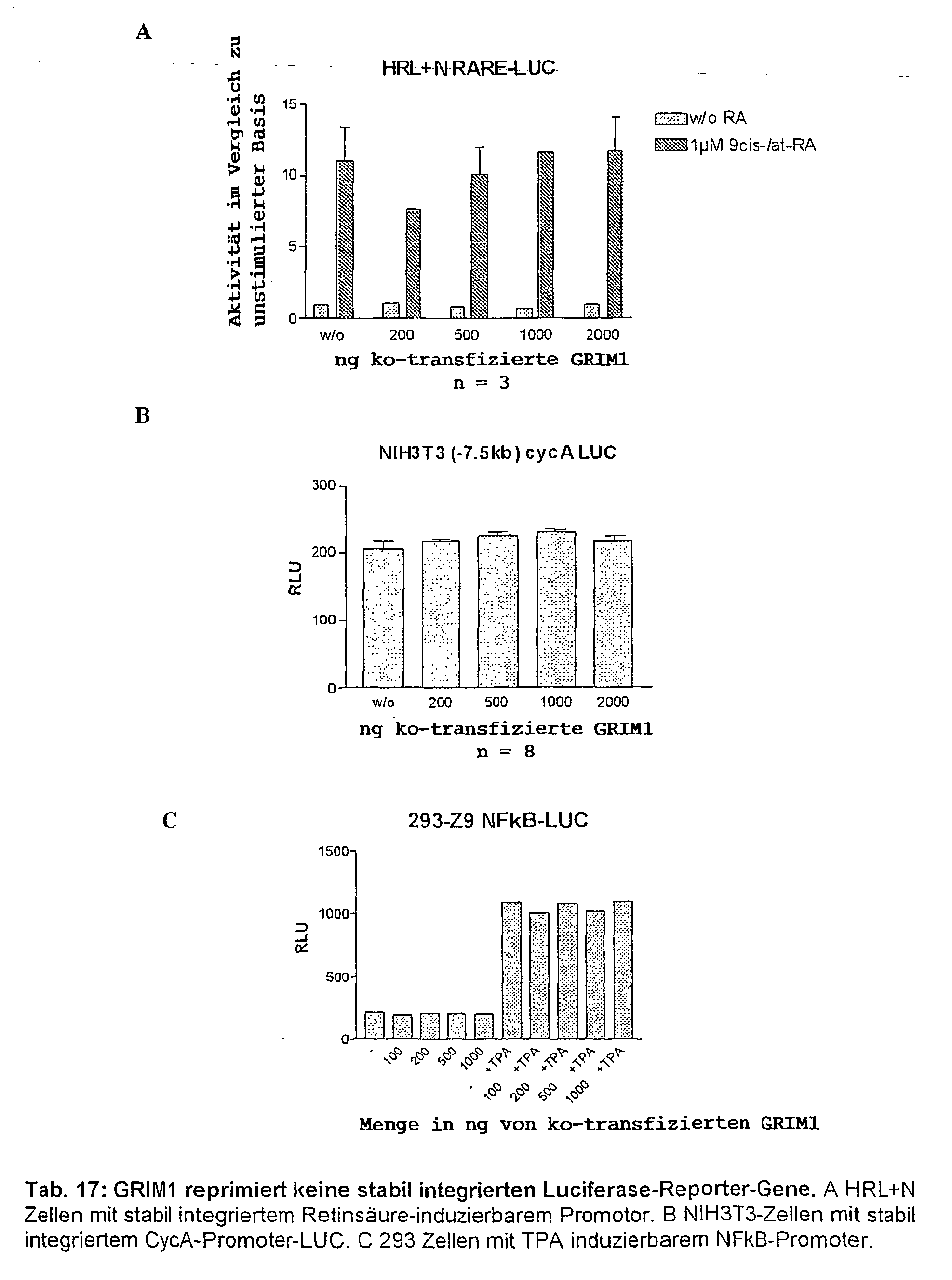

Desweiteren wurden in transienten Transfektionen direkte Effekte von GRIM1 auf diverse Transkriptionsfaktoren untersucht (vergleiche auch Beispiel 11). Neben dem AR wurden insbesondere mmGCNF, mmERR1 und mmRVR als Gesamtlängeproteine mit den korrespondierenden Reporterkonstrukten experimentell auf eine funktionelle Interaktion mit GRIM1 analysiert. In keiner der Transfektionen konnte ein signifikanter Effekt beobachtet werden.

Interaktionen von GRIM1 mit weiteren Transkriptionsfaktoren oder Ko-Faktoren wurde im Gal4-DBD basierenden Kontext untersucht, wobei der jeweilige Gal4-DBD fusionierte Faktor in konstanter Menge mit ansteigender Menge an Wildtyp GRIM1 kotransfiziert wurde. Untersuchte Faktoren innerhalb dieses Systems sind: Gal4-p300, Gal4-DBD, Gal-Sp1-4, Gal4-CBF). Auch hier wurde in keiner der Transfektionen ein signifikanter Effekt beobachtet.

### Beispiel 13

### Repressionsmechanismus von GRIM1

### a) HDAC Aktivität

Der bisher am weitesten verbreitete Repressionsmechanismus über Rekrutierung von Histon-Deacetylasen (HDACs) scheint bei GRIM1 nicht involviert zu sein. Das Repressionspotential von Gal4-DBD-GRIM1 in transienten Transfektionen wird über Applikation spezifischer HDAC Inhibitoren in einer Konzentration bis zu 5 fach über dem K_{D} (finale Konzentration von Trichostatin A 300nM, von Natrium-Butyrat 5 mM) nicht beeiträchtigt. Immun-affinitätsgereinigtes und hochstringent gewaschenes GRIM1 zeigt keine endogene HDAC-Aktivität. Auch assoziierte Komplexe, die über milde Waschbedingungen mit GRIM1 co-immunpräzipitiert wurden zeigen keinerlei HDAC-Aktivität. Desweiteren wurde eine direkte Beteiligung in HDAC/N-CoR/SMRT-Komplexen über Co-IP Studien ausgeschlossen.

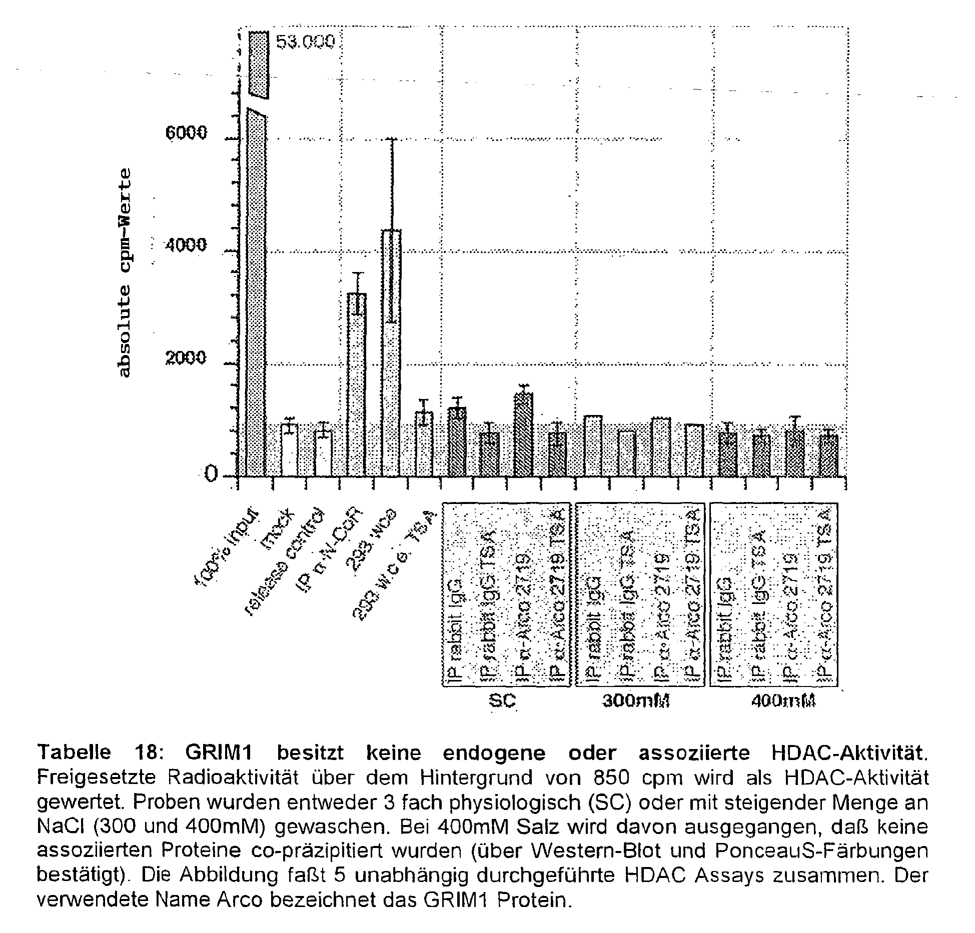

### b) INHAT-Aktivität

Eine weitere Möglichkeit zur transkriptionellen Repression besteht in der direkten Maskierung der Histon N-Termini, so daß eine Acetylierung und damit eine Auflockerung des Chromatins der betroffenen Region verhindert wird. Über Datenbankanalyse wurde herausgefunden, daß der N-Terminus (aa 25-135) und der absolute C-Terminus (aa 638-749) von hsGRIM1 über saure Domänen verfügt, die eine Homologie zu einer beschriebenen INHAT Domäne des Set Proteins aufweisen. INHAT ist ein Akronym für Proteindomänen / Proteine, die in der Lage sind, Lysine zu binden und dadurch eine Histon-Acetylierung zu verhindern.

In GST-Pulldown-Analysen konnte gezeigt werden, daß die N- und C-terminalen Fragmente von hsGRIM1 in vitro direkt mit Histonen assoziieren können. Darüber hinaus kann in in in vitro-Acetylierungs-Assays gezeigt werden, daß rekombinant exprimiertes GRIM1 aa 3-147 und aa 609-749 effizient die Acetylierung aller vier Core-Histone (H3, H2A, H2B und H4) durch p300 blockiert. Dadurch konnte gezeigt werden, daß ein möglicher Mechanismus der GRIM1 vermittelten Repression durch Histon-Maskierung gewährleistet werden könnte.

Die Hemmung der Acetylierung einzelner Histone durch GRIM1 erfolgt mit unterschiedlicher Effizienz. Bis jetzt konnte nur der Block der Acetylierung von H2A und H2B eindeutig bewiesen werden.

### c) Weitere Repressionsmechanismen

### • Lysin-Methylierung von Histonen

Methylierung spezifischer Lysine in den freien Histon-N-Termini (H3K9, H3K4) sorgen für eine Kompaktierung der Chromatin-Struktur, indem eine Acetylierung der regulatorisch wichtigen Lysine verhindert wird und dadurch eine Auflockerung des Chromatins verhindert wird. Desweiteren stellen methylierte Lysine eine hochaffine Bindestelle für das HP1 Protein (Heterochromatic Protein 1) dar. HP1 kann in Folge DNA-Methylasen, MeCPs und auch HDACs rekrutieren und zu einer gezielten Formierung von transientem oder von konstitutivem Heterochromatin führen und somit transkriptionelle Vorgänge reprimieren. Bisher beschriebene HMTasen sind zum Beispiel Suv39h1, Suv39h2 und PRMT1.

Homologieanalysen zeigen, daß in der GRIM1 Sequenz keine konservierte SET-Domäne vorhanden ist, welche für die Histon-Methyltransferase (HMTase)-Aktivität verantwortlich ist. Es wird daher angenommen, daß GRIM1 keine endogene HMTase Aktivität besitzt.

### • Rekrutierung von Chromatin-Reorganisierenden Komplexen

Eine weitere Möglichkleit der gezielten Repression wäre die zielgerichtete Rekrutierung von Chromatin reorganisierenden Komplexen (z.B. BRG, SWI/SNF, CHRAC, ARC, RSC etc.). Durch aktive Chromatin-Reorganisation können Promoterbereiche der zu reprimierenden Gene derart in kompaktes Chromatin eingewickelt werden, daß transkriptionelle Initiation deutlich erschwert wird.

Es kann nicht ausgeschlossen werden, daß GRIM1 mit einem der Komponenten einer der Remodelling-Komplexe eine funktionelle Interaktion eingeht.

### • Blockierung der PIC-Bildung / Behinderung der Initiationsreaktion / Behinderung der Elongation

Eine weitere Form der Repression ist die Be- oder Verhinderung der Formation des Präinitiationskomplexes (PIC) am Transkriptionsstart. Eine erfolgreiche Initiation kann nur erfolgen, wenn der TATA-Box-bindende Anteil von TFIID den Polymerase-Holokomplex an den Transkriptionsstartpunkt rekrutieren kann und wenn alle beteiligten basalen Transkriptionsfaktoren in ausreichender Quantität zur Verfügung stehen. Ein weiteres Prärequisit zu einer erfolgreichen Transkription ist, daß der C-Terminus der RNAPII phosphoryliert werden muß, damit die Initiationsstelle verlassen werden kann.

In einem dem Fachmann geläufigen Experiment kann geklärt werden, ob GRIM1 direkt mit einem der basalen Transkriptionsfaktoren oder Elongationsfaktoren in Wechselwirkung tritt und dadurch eine effiziente Transkription blockiert (Hefe Zwei-Hybrid System, Pulldown-Analysen). Eine Phosphatase-Domäne innerhalb der GRIM1 Struktur ist über Homologieanalysen nicht zu finden, der experimentelle Beweis, daß GRIM1 RNAPII dephosphorylieren könnte, steht noch aus.

### • Austitrierung von Ko-Aktivatoren durch Bindung an GRIM1

Das Potential von GRIM1, basale oder generelle Ko-Aktivatoren durch Bindung zu inaktivieren und dadurch den Initiations- oder Elongationsprozess zu reprimieren wird zur Zeit über Suche nach potentiellen Interaktionspartnern von hsGRIM1 untersucht.

Innerhalb des GRIM1-Proteins wurden insgesamt mindestens 5 unabhängige Repressordomänen (RDs) in transienten Transfektionen ermittelt. Die Fragmente E und I von GRIM1 zeigten in transienten Transfektionen das geringste Repressionspotential, doch Ihre Funktion bei der Repression durch GRIM1 wurde in den INHAT Analysen belegt.

Allerdings zeigen die Deletionsmutanten P, Q, R und auch S eine weitaus stärkere Repressionsfunktion in Gal-basierenden Transfektionen, was jenseits der bekannten Tatsachen noch weitere Mechanismen nahelegt. Durch übliche Experimente kann herausgefunden werden, welchen Beitrag zur Repression die übrigen Bereiche von GRIM1 leisten. Hierbei sollten interagierende Partnerproteine von GRIM1 identifiziert werden, um herauszufinden, ob eine Assoziation mit nicht-HDAC Komponenten transkriptioneller Ko-Repressor-Komplexe vorliegt.

### Beispiel 14

### Analyse funktioneller Motive in hsGRIM1

Funktionelle Motive innerhalb der hsGRIM1 Struktur wurden über Datenbank-Homologieanalysen herausgefunden. Eine Zusammenfassung der ermittelten Motive, deren Lokalisation innerhalb des Gesamtproteins und die verwendete experimentellen Strategie ist in der folgenden Tabelle dargestellt.

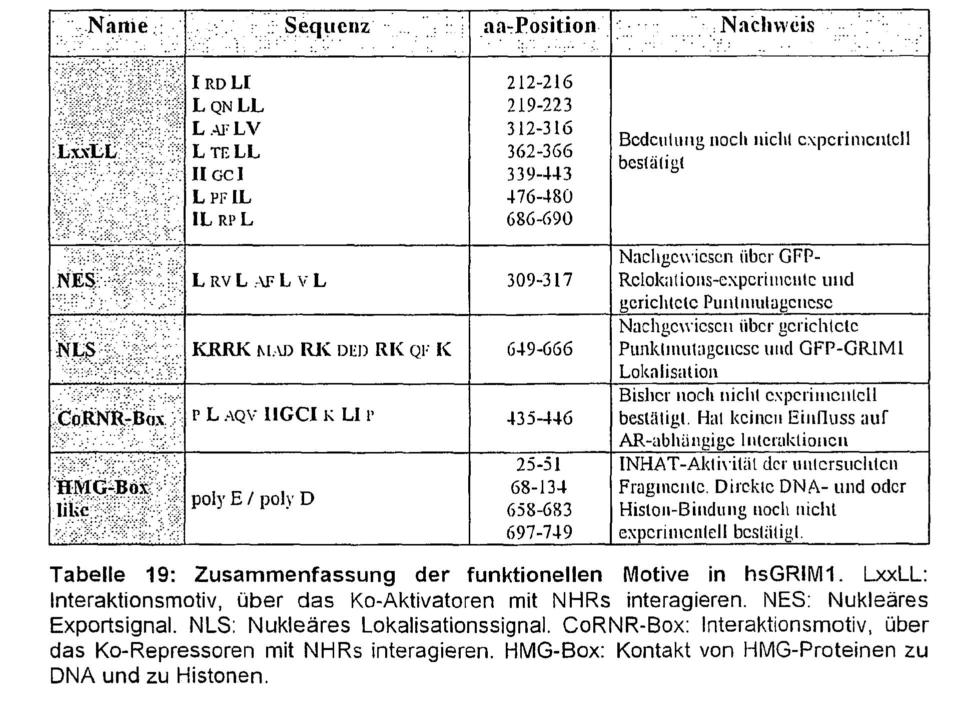

### Nachweis des NES innerhalb von hsGRIM1

Zur direkten Analyse des Motivs wurden alle vier relevanten Lysine durch gerichtete Punktmutagenese inaktiviert, so daß die α-helicale Struktur des NES zerstört wurde. Die inaktivierenden Mutationen sind in der folgenden Abbildung dargestellt. Eine gerichtete Inaktivierung der beiden 3' gelegenen Lysine alleine erwies sich als nicht ausreichend, die Funktionalität des NES zu zerstören.

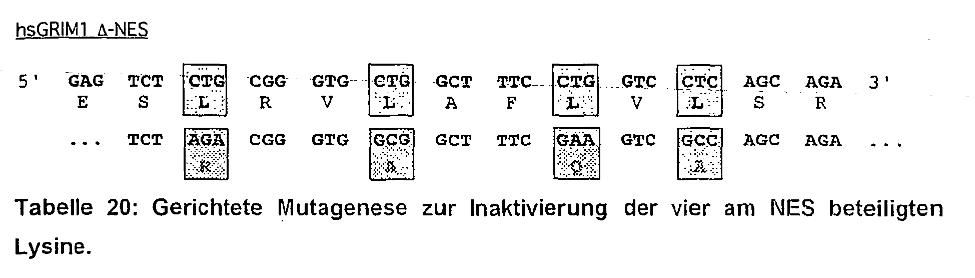

Zum Nachweis der Funktionalität des Exportsignales wurden hsGRIM1 Proteinfragmente mit GFP (Green Fluorescent Protein) fusioniert (dargestellt in der folgenden Abbildung) und über transiente Transfektion in Zellen exprimiert. Die Lokalisation der jeweiligen Fragmente wurde über Fluoreszenzmikroskopie nachgewiesen.

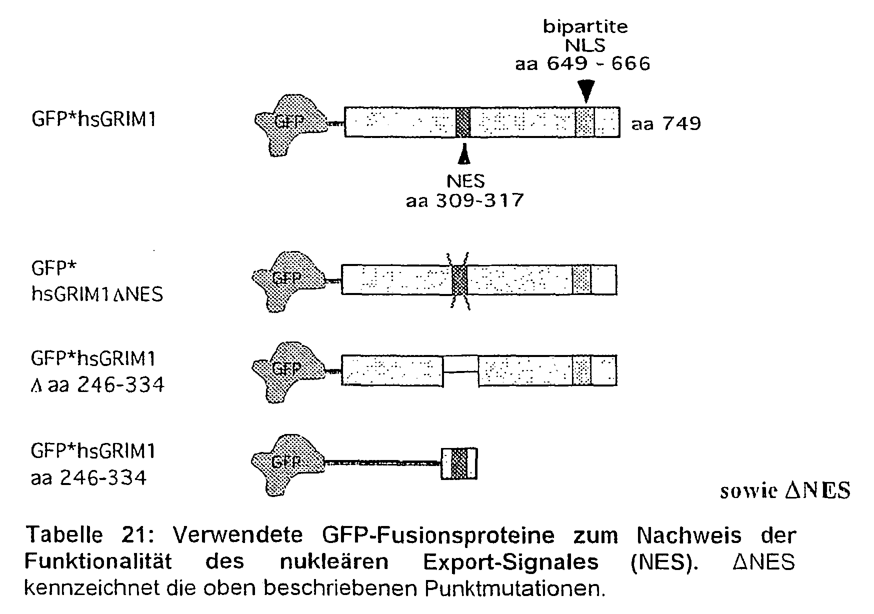

GFP allein zeigte eine pan-zelluläre Lokalisation, Gesamtlänge GFP-hsGRIM1 war exklusiv nukleär lokalisiert, durch die Dominanz des NLS ebenso die NES-Deletionsmutante und das Fragment, dem ein das NES flankierender Bereich deletiert wurde (hsGRIM1 Δa246-334). Wurde das hsGRIM1-Fragment, welches das NES beinhaltet, an GFP fusioniert (hsGRIM1 aa246-334), zeigte das resultiuerende Protein exklusiv cytoplasmatische Lokalisation. Wurde das punktmutierte Fragment von aa 246-334 an GFP gekoppelt, wurde die pan-zelluläre Lokalisation wiederhergestellt, so daß die Funktionalität des Motives belegt werden konnte.

### Nachweis des NLS innerhalb von hsGRIM1

Zur Analyse des Motives wurden neben den weiter unten angeführten Deletionsmutanten alle sieben relevanten basischen Reste durch gerichtete Punktmutagenese inaktiviert. Eine Inaktivierung der 3' gelegenen 3 basischen Reste allein erwies sich als nicht ausreichend, das NLS zu inaktivieren.

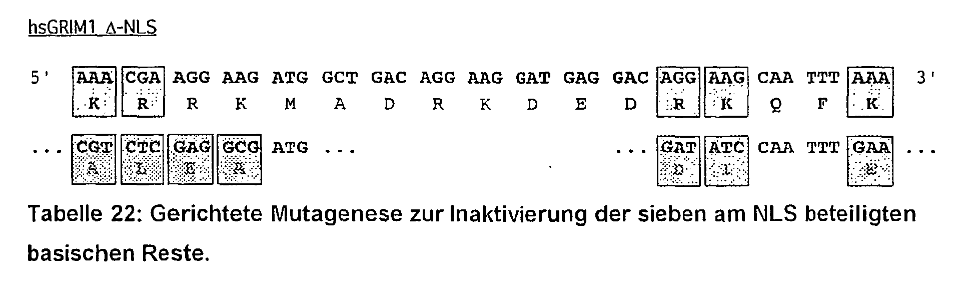

Zum Nachweis der Funktionalität des Importsignales wurden hsGRIM1 Proteinfragmente mit GFP fusioniert (dargestellt in der folgenden Abbildung) und über transiente Transfektion in Zellen exprimiert. Die Lokalisation der jeweiligen Fragmente wurde über Fluoreszenzmikroskopie nachgewiesen.

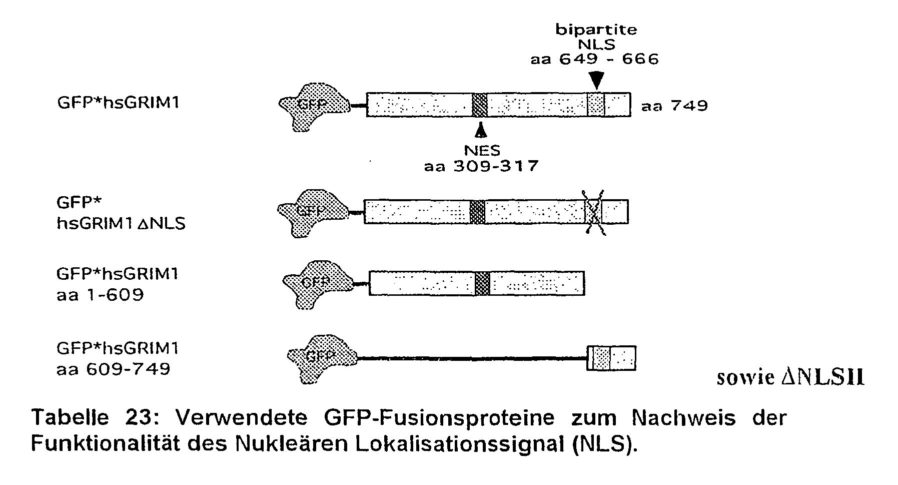

GFP allein zeigte eine pan-zelluläre Lokalisation, Gesamtlänge GFP-hsGRIM1 war exklusiv nukleär lokalisiert. Wurden die letzten 140 Aminosäuren deletiert (hsGRIM1 aal-609), zeigte das GFP-Fusionsprotein sowohl cytoplasmatische als auch eine schwache nukleäre Lokalisation, das korrespondierende Gegenstück an GFP fusioniert (GFP-hsGRIM1 aa609-749) zeigt exklusiv nukleäre Lokalisation. Das Gesamtlänge hsGRIM1 mit der siebenfachen Punktmutation innerhalb des NLS zeigte das gleiche Verteilungsmuster wie die hsGRIM1 aa1-609 Deletionsmutante und bestätigte die Funktionalität des NLS. Wurde das punktmutierte Fragment von aa609-749 an GFP gekoppelt, war das Fusionsprotein ebenfalls cytoplasmatisch lokalisiert.

### Nachweis der CoRNR-Box innerhalb von hsGRIM1

Zur Analyse der CoRNR-Box wurden alle drei hochkonservierten und notwendigen Isoleucine im hydrophoben core der Helix durch Alanine bzw. durch ein Serin ersetzt: I436A, l437S I444A. Eine weitergehende funktionelle Charakterisierung der gesetzten Punktmutationen hat bisher nur ergeben, daß das Repressionspotential von hsGRIM1 nicht beeinträchtigt wird und daß die Interaktion mit dem AR durch Zerstörung der CoRNR Box nicht beeinträchtigt wird.

### Intrazelluläre Relokalisation von mmGRIM1 in Zellkultur-basierenden Differenzierungsmodellen

GRIM1 zeigt eine Änderung seiner subzellulären Lokalisation innerhalb spezifischer Differenzierungsprozesse. In Zellkultursystemen wird GRIM1 während der Shelettmuskel-Differenzierung von Maus-C2C12 Zellen aus dem Kern in das umgebende Cytoplasma transportiert. Der Zeitrahmen der beobachteten Translokation von GRIM1 liegt im Bereich der Expression des myogenen bHLH-Transkriptionsfaktors Myogenin (Myf4) und des Strukturproteins Skelettmuskel Myosin-Heavy-chain (MHC), also zu einem weiter fortgeschrittenen Zeitpunkt der Muskeldifferenzierung als bisher beschriebene Faktoren wie HDAC4, HDAC5 und HDAC7. C2C12 Zellen differenzieren unter Niedrig-Serum-Bedingungen innerhalb von 6-8 Tagen aus proliferierenden Myoblasten zu fusionierten, multinukleären Myotuben aus. Dabei unternehmen die Zellen einen G₀-Arrest, der CDK-Inhibitor p21 wird hochreguliert und eine geordnete Kaskade von myogenen Transkriptionsfaktoren wie Myf5, MyoD und Myogenin bewirken die transkriptionelle Voraussetzung der Myogenese. GRIM1 verläßt in differenzierenden C2C12 Skelettmuskelzellen nach ca. 6 bis 8 Tagen die Kerne fusionierter Myotuben, wohingegen GRIM1 in mononukleären Myoblasten immer nukleär lokalisiert ist. Die Expression des Muskelstrukturproteins MHC erfolgt zu dem Zeitpunkt, an dem erste GRIM1-freie Nuklei zu beobachten sind und wurde in IIFs als temporaler Marker für die GRIM-Relokalisation verwendet.

Die für C2C12 bestimmten Daten gelten auch für weitere zelluläre Skelettmuskeldifferenzierungs-Systeme. Der gleichen Effekt konnte auch in Ratten L6 Skelettmuskelzellen nachgewiesen werden. Die Zellen haben eine andere Differenzierungskinetik und fusionieren erst ab Tag 10-12 in Differenzierungsmedium (DM). GRIM1 relokalisiert auch in L6 Zellen, GRIM1 freie Kerne sind erst ab Tag 16 in DM zu detektieren. In einem weiteren Differenzierungssystem wurden Maus 10T1/2 Fibroblasten stabil mit einer Östrogen-induzierbaren MyoD-Expressionskassette transfiziert (Zellen freundlicherweise zur Verfügung gestellt von Dr. D. Bergstrom). Nach Zugabe von 1µM Estradiol wird über MyoD Expression die Skelettmuskeldifferenzierung gewährt. Nach ca. 3-4 Tagen in DM sind bereits viele Zellen als multinukleäre Myotuben zu detektieren.

Während der Skelettmuskeldifferenzierung wird das GRIM1 Protein nicht degradiert und / oder abgebaut, sondern ist in vollem Umfang auch im Cytoplasma vorhanden. In Western-Blot-Analysen mit Gesamtzellextrakten von C2C12 Zellen, die über 8 Tage in DM zu Myotuben differenziert wurden (am Tag 8 nach Zugabe des DM waren ≥ 60% aller vorhandenen Zellen Myotuben) ist GRIM1 lmmunoreaktivität unverändert nachzuweisen. Die Ergebnisse können auch über Zellfraktionierungen weitergehend bestätigt werden. Desweiteren haben RT-PCR Analysen bestätigt, daß die mmGRIM1-mRNA Menge während der C2C12 Differenzierung nicht verändert ist.

Als weitergehende Kontrolle wurde untersucht, ob GRIM1 auch während der GlattmuskelDifferenzierung eine subzelluläre Relokalisation erfährt. Zu diesem Zweck wurden ex vivo Glattmuskelzellen aus der Maus gewonnen und über Wachstumsfaktor-Zugabe dedifferenziert. Zusätzlich wurden Zellen über Differenzierungsmedium in vitro wieder weiter zu Glattmuskelzellen differenziert. Die Ergebnisse zeigen, daß GRIM1 während der Glattmuskeldifferenzierung nicht den Kern verlässt. Desweiteren wurde ein Zellkultur Glattmuskel-Differenzierungssystem (Monc-1 Zellen) verwendet, um in vitro Daten über eine eventuelle GRIM1 Relokalisation zu erhalten. Wie auch für die ex vivo Zellen bereits gezeigt, konnte innerhalb der untersuchten Differenzierungszeiten keine GRIM1 Relokalisation in Glattmuskelzellen beobachtet werden.

Ein weiteres *in vitro* Differenzierungsmodell, das für GRIM1-Relokalisationsanalysen verwendet wurde, ist die Differenzierung von 3T3-L1 Präadipocyten zu reifen Fettzellen. Bei insgesamt drei durchgeführten Differenzierungsprotokollen zeigte GRIM1 zweimal eine detektierbare Re-Lokalisation aus dem Kern in das Cytoplasma, in einem Fall war keine Veränderung der Lokalisation der GRIM1 Immunoreaktivität zu detektieren. Western-Blot-Analysen haben gezeigt, daß in keinem der drei Protokolle eine Abnahme von GRIM1 Immunoreaktivität in Gesamtzellextrakten zu detektieren war.

### Beispiel 15

### Regulation der GRIM1 Funktion

Vollständige experimentelle Ergebnisse zur Regulation der GRIM1 Funktion und zu Signalwegen, die an der subzellulären Relokalisation von GRIM1 beteiligt sein könnten, liegen noch nicht vor. Bislang konnte kein Signalweg eindeutig identifiziert werden, der die transkriptionelle Aktivität von GRIM1 moduliert. Es gibt auch noch keine experimentellen Beweise für die Beteiligung einer Signalkaskade, die in den Relokalisationsprozess von GRIM1 eingreift. Im folgenden sind die vorhandenen Daten zu diesem Themenfeld kurz zusammengefasst.

### Transkriptionelle Aktivität:

- In unmittelbarer Nähe des NES innerhalb der GRIM1 Sequenz befindet sich eine Konsensus PKC-Phosphorylierungsstelle (Serin 308). Die transkriptionelle Aktivität von Gal-GRIM1 wurde in transienten Transfektionen in verschiedenen Zell-Linien untersucht. Weder eine Behandlung der Zellen mit dem spezifischen PKC-Inhibitor Gö6850 (Parke-Davis, vormals Gödecke AG) noch eine Stimulation mit dem PKC aktivierenden Agens TPA brachte eine Veränderung der transkriptionellen Aktivität.
- Die einzige experimentelle Evidenz für eine Modifikation von GRIM1 ist der Nachweis einer direkten Acetylierung von GRIM1 (aa3-147 sowie von Gesamtlänge GRIM1) durch die HAT-Domäne von p300.
- Eine Ko-Transfektion von Gesamtlänge-p300 und Gal-GRIM1 zeigt kein verändertes Repressionsmuster, Zugabe von Trichostatin A (TSA) verstärkt die generelle Transkription, doch die relative Repression durch Gal-GRIM1 bleibt unverändert.
- Ko-Transfektion von Gal-GRIM1 in Kombination mit einer konstitutiv aktiven Version der Rho-GTPase (RhoAV14) zeigt ebenfalls keine Veränderung der Aktivität.

### Relokalisation:

- Der nukleäre Export von GFP-GRIM1 bzw. der Mutante GFP-GRIM1 aa234-336 wird durch Behandlung der Zellen mit dem bakteriellen Toxin Leptomycin B (LMB, 20nM für 6 h vor der Zellernte und Analyse durch IIF) nicht beeinflusst. LMB ist ein spezifischer Inhibitor des Exportfaktors Exportin1/Crm1, so daß aus dem Experiment geschlossen werden kann, daß ein Exportin-unabhängiger Transportmechanismus vorliegen muss.
- 14-3-3 Proteine sind dafür verantwortlich, daß während der Muskeldifferenzierung die HDACs 4, 5 und 7 aktiv aus dem Nukleus entfernt und im Cytoplasma zurückgehalten werden. Über Co-IP Studien konnte eine Interaktion zwischen GRIM1 und 14-3-3 Proteinen (Familienmitglieder zeta, beta, tau) ausgeschlossen werden.
- Behandlung von differenzierenden C2C12 Skelettmuskelzellen mit diversen Inhibitoren zentraler Signaltransduktionswege haben keinen Effekt auf die Relokalisation oder die Anzahl der nukleären Speckles von GRIM1 innerhalb der untersuchten Differenzierung von Tag 0 bis Tag 6 nach Zugabe des Differenzierungsmediums. Getestete Inhibitoren: pan-HDAC-Inhibitoren TSA und Natrium-Butyrat, Proteinkinase-Inhibitoren Gö6850 (spezifischer PKC Inhibitor, 1µM), LY294002 (spezifischer Pl3Kinase-Inhibitor, 10µM), Ro31-8220 (PKC Breitband-Inhibitor, 0.5µM), PD98059 (MEK1-Inhibitor, 10µM), Fasudil (Rock-Kinase Inhibitor, 10µM), SB203580 (p38α/β Inhibitor). Überacetylierung und Block zentraler Signalkaskaden hatte für die Zellen teils drastische morphologisch sichtbare Konsequenzen, doch die Relokalisation oder die Anzahl der detektierbaren GRIM1-Speckles war unbeeinflußt.

### Beispiel 16

### Interaktion von hsGRIM1 und dem Androgen Rezeptor (AR) und daraus resultierende funktionale Konsequenzen

In einem Hefe-Zweihydrid Interaktionstest konnte eine Interaktion von GRIM1 mit dem nukleären Hormon-Rezeptor Androgen Rezeptor (AR) nachgewiesen werden. Die Interaktionsdomäne von GRIM1 in dem Hefe-basierenden Assay liegt im C-Terminus zwischen aa 606 bis 749. Eine weitergehende funktionale Charakterisierung der Interaktionsdomänen innerhalb von GRIM1 wurde über Pulldown-Analysen mit den exprimierbaren Fragmenten von GRIM1 durchgeführt. Die Interaktion von GRIM1 mit dem AR ist unabhängig von der Art des verwendeten Liganden (Agonisten, Antagonisten oder keine Liganden). Die vorhandenen Daten sind in der folgenden Graphik zusammengefasst.

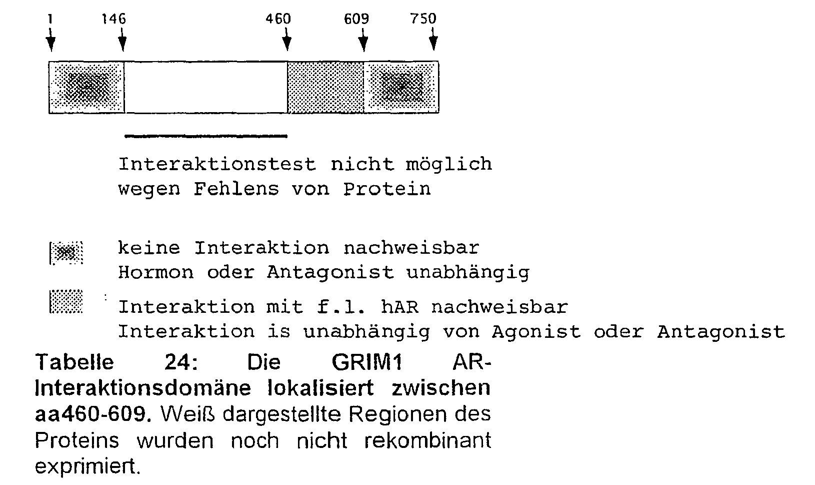

Der C-Terminus von GRIM1 zeigt im Gegensatz zu den in Hefe gewonnenen Daten keine *in vitro*-Interaktion mit dem AR, das Fragment von aa 460 bis aa 609 jedoch zeigt eine starke Interaktion unabhängig vom verwendeten Liganden.

Die Interaktion zwischen dem AR und GRIM1 konnte in *in vivo* Interaktionsassays ebenfalls nachgewiesen werden. In Co-Immunopräzipitationen war die Interaktion zwischen GRIM1 und dem AR ebenfalls Liganden-unabhängig und konnte selbst in Waschpuffer mit 400 mM NaCl nicht signifikant geblockt werden. Die Interaktion konnte mit IP-Studien mit Antikörpern sowohl gegen den AR als auch gegen GRIM1 gezeigt werden.

Der AR ist ein Mitglied der Superfamilie der Ligand-aktivierbaren nukleären Hormon-Rezeptoren. Nach Bindung des natürlichen AR-Liganden 5α-Dihydrotestosteron wird der AR vom Cytoplasma in den Kern transportiert und bewirkt eine transkriptionelle Aktivierung seiner Zielgene. Neben den aktivierenden Liganden gibt es für jeden Rezeptor spezifische Antagonisten, die ebenfalls eine Translokation in den Kern bewirken, allerdings eine repressive Konformation der Rezeptor-Ligandenbindungsdomäne bewirken. Antagonistgebundener Rezeptor kann Ko-Repressorkomplexe rekrutieren und aktiv Transkription reprimieren. In manchen Zell-Linien oder unter gewissen pathologischen Situationen können Antagonisten aber zu Partialagonisten werden und unnatürlicherweise eine Transaktivierung durch den Rezeptor bewirken. Im Fall des AR ist bekannt, daß bei Prostata-Karzinomen therapeutisch eingesetzte Substanzen wie Cyproterone-Acetat, Casodex oder Hydroxy-Flutamid in hormonrefraktären Tumoren partiell agonistisch wirken können.

Im Zellkultursystem konnte gezeigt werden, daß GRIM1 spezifisch eine CPA-, Casodex- oder OH-Flutamid-bewirkte Transaktivierung des AR reprimieren kann. Nicht-ligandierter oder mit DHT beladener AR wird durch GRIM1 transkriptionell nicht beeinflusst. Der beobachtete Effekt schwankt im Bereich zwischen 2-3 facher Repression der CPAhervorgerufenen AR-Transaktivierung.

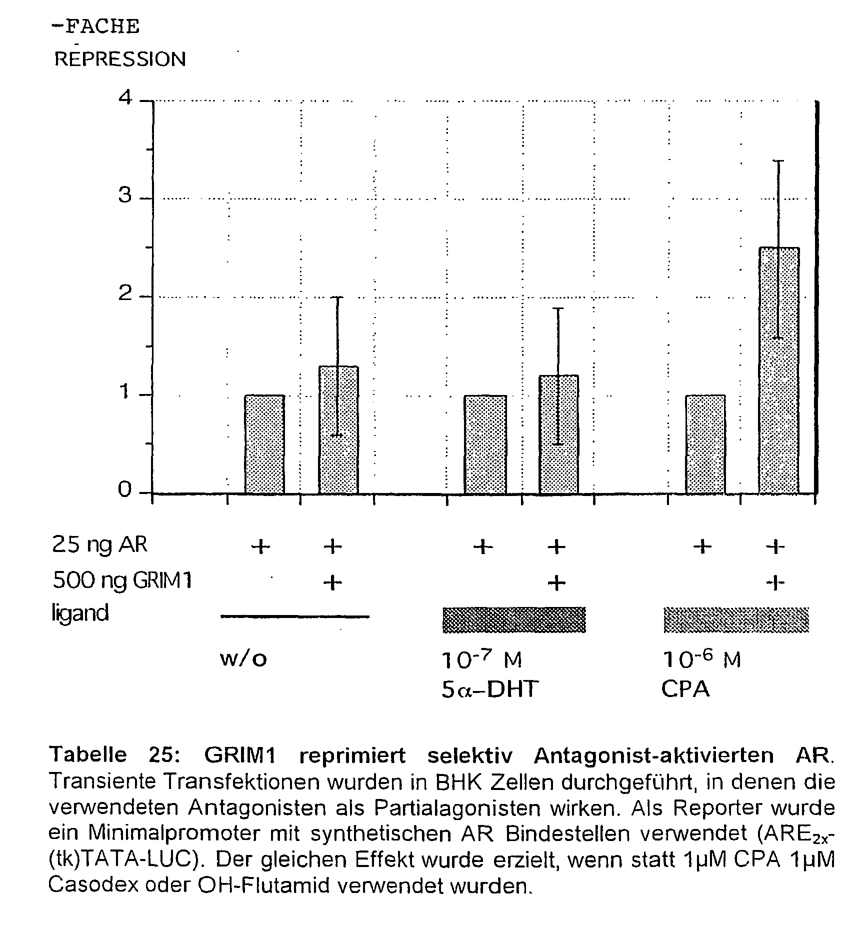

Ein weiterer Hinweis auf die Funktionalität des Effektes ist, daß die Superaktivierung des CPA-beladenen AR durch den Co-Aktivator TIF2 in BHK-Zellen durch Cotransfektion von GRIM1 reprimiert oder zumindest deutlich eingeschränkt werden kann.

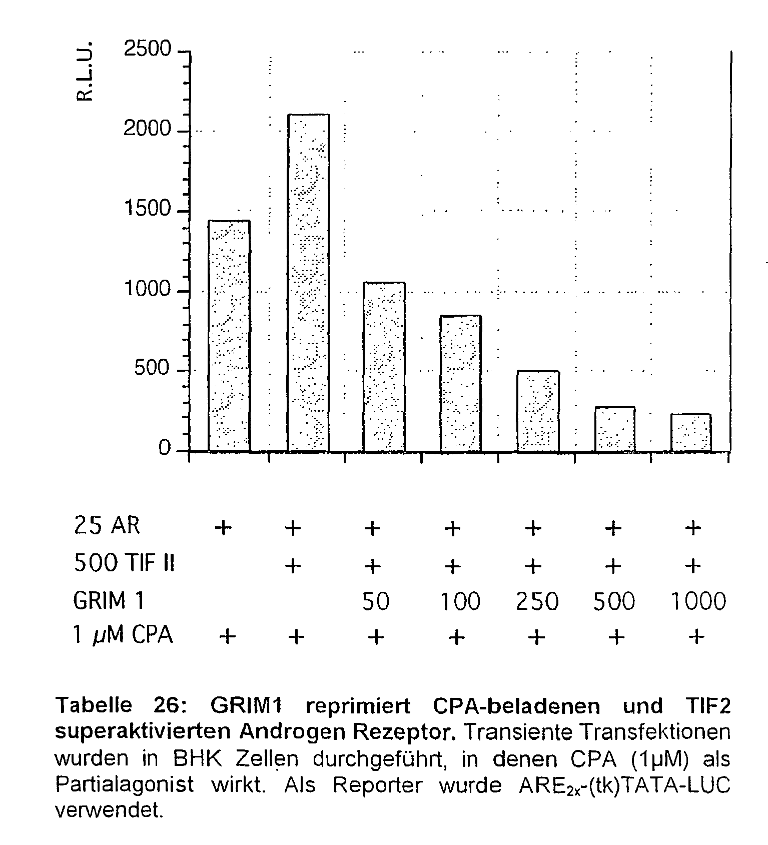

## Patentansprüche

1. In vitro Verwendung eines Antikörpers, der spezifisch an ein Epitop eines Polypeptids mit der Aminosäuresequenz SEQ ID NO:3 bindet, zum Nachweis von GRIM1 in Zellen, **dadurch gekennzeichnet, dass** die Zellen ausgewählt sind aus Skelettmuskelzellen und Präadipocyten.

2. Verwendung nach Anspruch 1, **dadurch gekennzeichnet, dass** der Antikörper ein monoklonaler Antikörper ist.

3. Verwendung nach Anspruch 1, **dadurch gekennzeichnet, dass** der Antikörper ein polyklonaler Antikörper ist.

4. Verwendung nach Anspruch 3, **dadurch gekennzeichnet, dass** der polyklonale Antikörper gegen die Aminosäuren 3-147 von SEQ ID NO:3 gerichtet ist.

5. Verwendung nach Anspruch 3, **dadurch gekennzeichnet, dass** der polyklonale Antikörper gegen die Aminosäuren 609-749 von SEQ ID NO:3 gerichtet ist.

6. Verwendung nach einem der vorhergehenden Ansprüche, **dadurch gekennzeichnet, dass** die subzelluläre Lokalisation von GRIM1 nachgewiesen wird.

7. Verwendung nach einem der Ansprüche 1 bis 6, **dadurch gekennzeichnet, dass** die intrazelluläre Lokalisation von GRIM1 durch indirekte Immunfluoreszenz nachgewiesen wird.

8. Verwendung nach einem der vorhergehenden Ansprüche, **dadurch gekennzeichnet, dass** der Nachweis von GRIM1 oder der GRIM1-Lokalisation immunhistologisch erfolgt.

9. Verwendung nach einem der vorhergehenden Ansprüche, **dadurch gekennzeichnet, dass** Fluoreszenz-gekoppelte oder Enzym-gekoppelte Antikörper verwendet werden.

10. Verwendung nach einem der Ansprüche 1 bis 9, **dadurch gekennzeichnet, dass** die Zellen Skelettmuskelzellen sind.

11. Verwendung nach Anspruch 10, **dadurch gekennzeichnet, dass** die Skelettmuskelzellen C2C12-Zellen sind.

12. Verwendung nach einem der Ansprüche 1 bis 9, **dadurch gekennzeichnet, dass** die Zellen Präadipocyten sind.

13. Verwendung nach Anspruch 12, **dadurch gekennzeichnet, dass** die Präadipocyten N1H3T3-L1 Präadipocyten sind.

## Claims

1. In vitro use of an antibody which binds specifically to an epitope of a polypeptide having the amino acid sequence SEQ ID NO:3 for detecting GRIM1 in cells, **characterized in that** the cells are selected from skeletal muscle cells and preadipocytes.

2. The use according to claim 1, **characterized in that** the antibody is a monoclonal antibody.

3. The use according to claim 1, **characterized in that** the antibody is a polyclonal antibody.

4. The use according to claim 3, **characterized in that** the polyclonal antibody is directed against amino acids 3-147 of SEQ ID NO:3.

5. The use according to claim 3, **characterized in that** the polyclonal antibody is directed against amino acids 609-749 of SEQ ID NO:3.

6. The use according to any one of the preceding claims, **characterized in that** the subcellular localization of GRIM1 is detected.

7. The use according to any one of claims 1 to 6, **characterized in that** the intracellular localization of GRIM1 is detected via indirect immunofluorescence.

8. The use according to any one of the preceding claims, **characterized in that** the detection of GRIM1 or of the GRIM1 localization is carried out immunohistologically.

9. The use according to any one of the preceding claims, **characterized in that** fluorescence-linked or enzyme-linked antibodies are used.

10. The use according to any one of claims 1 to 9, **characterized in that** the cells are skeletal muscle cells.

11. The use according to claim 10, **characterized in that** the skeletal muscle cells are C2C12 cells.

12. The use according to any one of claims 1 to 9, **characterized in that** the cells are preadipocytes.

13. The use according to claim 12, **characterized in that** the preadipocytes are NIH3T3-L1 preadipocytes.

## Revendications

1. Utilisation in vitro d'un anticorps qui se lie de manière spécifique à un épitope d'un polypeptide ayant la séquence d'acides aminés représentée par la SEQ ID n° 3, dans le but de détecter la protéine GRIM1 dans des cellules, **caractérisée en ce que** les cellules sont sélectionnées parmi les cellules musculaires du squelette et les préadipocytes.

2. Utilisation selon la revendication 1, **caractérisée en ce que** l'anticorps est un anticorps monoclonal.

3. Utilisation selon la revendication 1, **caractérisée en ce que** l'anticorps est un anticorps polyclonal.

4. Utilisation selon la revendication 3, **caractérisée en ce que** l'anticorps polyclonal est dirigé contre les acides aminés 3 à 147 de la séquence représentée par la SEQ ID n° 3.

5. Utilisation selon la revendication 3, **caractérisée en ce que** l'anticorps polyclonal est dirigé contre les acides aminés 609 à 749 de la séquence représentée par la SEQ ID n° 3.

6. Utilisation selon l'une quelconque des revendications précédentes, **caractérisée en ce que** l'on procède à la localisation subcellulaire de la GRIM1.

7. Utilisation selon l'une quelconque des revendications 1 à 6, **caractérisée en ce que** la localisation intracellulaire de la GRIM1 est effectuée par le biais d'une détection par immunofluorescence indirecte.

8. Utilisation selon l'une quelconque des revendications précédentes, **caractérisée en ce que** l'on procède à la détection de la GRIM1 ou à la localisation de la GRIM1 par immunohistologie.

9. Utilisation selon l'une quelconque des revendications précédentes, **caractérisée en ce que** l'on utilise des anticorps couplés à une molécule fluorescente ou couplés à une enzyme.

10. Utilisation selon l'une quelconque des revendications 1 à 9, **caractérisée en ce que** les cellules sont des cellules musculaires du squelette.

11. Utilisation selon la revendication 10, **caractérisée en ce que** les cellules musculaires du squelette sont représentées par des cellules de la lignée C2C12.

12. Utilisation selon l'une quelconque des revendications 1 à 9, **caractérisée en ce que** les cellules sont des préadipocytes.

13. Utilisation selon la revendication 12, **caractérisée en ce que** les préadipocytes sont des préadipocytes de la lignée NIH3T3-L1.
